(19) Europäisches Patentamt
European Patent Office
Office européen des brevets

(11) EP 2 646 045 B1

(12) # EUROPEAN PATENT SPECIFICATION

(45) Date of publication and mention of the grant of the patent:
**05.07.2017 Bulletin 2017/27**

(21) Application number: **11845326.5**

(22) Date of filing: **02.12.2011**

(51) Int Cl.:
*A61K 38/21* (2006.01) *A61K 31/194* (2006.01)
*A61K 31/00* (2006.01) *A61K 31/19* (2006.01)
*A61K 31/225* (2006.01) *A61K 31/4745* (2006.01)
*C12N 5/00* (2006.01) *C12N 15/113* (2010.01)
*C12N 15/115* (2010.01) *G01N 33/50* (2006.01)

(86) International application number:
**PCT/US2011/063087**

(87) International publication number:
**WO 2012/075408 (07.06.2012 Gazette 2012/23)**

(54) **SUPPRESSORS OF NEUROTOXICITY IN HUNTINGTON'S DISEASE**

UNTERDRÜCKER DER NEUROTOXIZITÄT BEI DER HUNTINGTON-KRANKHEIT

SUPPRESSEURS DE LA NEUROTOXICITÉ DANS LA MALADIE DE HUNTINGTON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2010 US 419157 P**
**15.04.2011 US 201161476040 P**

(43) Date of publication of application:
**09.10.2013 Bulletin 2013/41**

(73) Proprietor: **Massachusetts Institute of Technology**
**Cambridge, MA 02139 (US)**

(72) Inventors:
- **SCHULTE, Joost**
  **Ontario**
  **M1S 2L4 (CA)**
- **SEPP, Katharine, Julia**
  **Agincourt**
  **ONM1S 2L4 (CA)**
- **LITTLETON, J., Troy**
  **Littleton**
  **MA 01460 (US)**

(74) Representative: **Hoffmann Eitle**
**Patent- und Rechtsanwälte PartmbB**
**Arabellastraße 30**
**81925 München (DE)**

(56) References cited:
**WO-A1-2008/000070** **WO-A1-2010/007788**
**US-A1- 2005 137 209** **US-A1- 2006 121 488**
**US-A1- 2009 023 736** **US-A1- 2009 023 798**
**US-A1- 2009 143 279** **US-A1- 2009 208 455**
**US-A1- 2009 252 717** **US-A1- 2010 099 760**

- **SCHULTE ET AL.: 'High-Content Chemical and RNAi Screens for Suppressors of Neurotoxicity in a Huntington's Disease Model. e23841' PLOS ONE vol. 6, no. 8, 31 August 2011, XP055109591**
- **CHEN ET AL.: 'Identification of common genetic modifiers of neurodegenerative diseases from an integrative analysis of diverse genetic screens in model organisms' BMC GENOMICS vol. 13, no. 71, 25 February 2012, XP021117860**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 646 045 B1

## Description

RELATED APPLICATIONS

This application claims priority under 35 U.S.C. § 119(e) to United States provisional patent applications, U.S.S.N. 61/419,157, filed December 2, 2010; and U.S.S.N. 61/476,040, filed April 15, 2011, both entitled "Chemical and RNAi suppressors of Neurotoxicity in Huntington's Disease."

GOVERNMENT SUPPORT

This invention was made with Government support under Grant No. NS052203 awarded by the National Institute of Health. The U.S. Government has certain rights in the invention.

FIELD OF THE INVENTION

This invention relates to the biological and medical fields. In some aspects, the invention relates to the field of polyQ tract expansion diseases and disorders, for example, the field of Huntington's disease.

BACKGROUND OF THE INVENTION

Huntington's Disease (HD) is a fatal polyQ tract expansion disorder for which there are no effective therapeutics. The disease results from expansion of a poly-glutamine (poly-Q) tract in the Huntingtin (Htt) protein that alters its conformation and function. Neuropathological hallmarks of the disease include Htt aggregation and striatal neuron degeneration. Mammalian models of HD indicate that neuron-specific dysregulation of cellular physiology contributes to the underlying neuropathology (Roze et al., 2008).

Mutant Htt has been suggested to disrupt transcription, proteosome activity, axonal transport, synaptic function, signaling cascades (including the mTOR/Insulin pathway), and other physiological processes in a variety of neuronal subtypes. The relative contribution of these potential pathologies to overall HD pathogenesis is unknown.

SUMMARY OF THE INVENTION

The invention is as defined in the claims. Some aspects of this disclosure relate to methods, compositions, disease models, and cells for high-content screening strategies to identify suppressors of neurotoxicity in polyQ tract expansion disease, for example, in Huntington's Disease.

Some aspects of this invention as described herein provide a morphometric analysis with high-content RNAi and compound screening to identify suppressors of HD toxicity using a *Drosophila* primary neuronal culture system.

Some aspects of the invention relate to screening methods for the identification of compounds or compositions that modulate polyQ tract expansion disease-associated phenotypes. Some embodiments of this invention provide *in vitro* screening methods employing a polyQ tract expanded protein, for example, a polyQ tract expanded Htt protein (e.g. HttQ138).

Some aspects of this invention relate to compounds and compositions useful in the treatment of polyQ tract expansion disease, for example, HD. Some embodiments of this invention provide compounds and compositions that ameliorate a phenotype associated with a polyQ tract expansion disease, for example, increased polyQ tract protein aggregation, or pathologic changes in cell morphology. Some embodiments of this invention provide compounds and compositions that ameliorate a phenotype associated with polyQ tract expansion disease without displaying significant cytotoxic or cytostatic characteristics, and without affecting tissue homeostasis or cell differentiation patterns. Some embodiments of this invention provide compounds and compositions for the treatment of a polyQ tract expansion disease.

Some aspects of this invention relate to methods of treatment of a polyQ tract expansion disease. For example, some embodiments provide a method for treating a polyQ tract expansion disease or disorder, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of carbenoxolone, or an analog, salt, or solvate thereof. In some embodiments, the polyQ tract expansion disease or disorder is Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12). In some embodiments, the polyQ tract expansion disease or disorder is a polyQ tract expansion mutation in the ATN1, DRPLA, HTT, Androgen receptor on

the X chromosome, ATXN1, ATXN2, ATXN3, ATXN12, CACNA1A, ATXN7, TBP, PPP2R2B, or SCA12 gene. In some embodiments, the subject expresses an ATN1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the subject expresses an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the polyQ tract expansion disease or disorder is HD. In some embodiments, the subject expresses a HTT (Huntingtin) protein comprising a polyQ tract of 35-140 Q residues. In some embodiments, the subject is a human subject. In some embodiments, the carbenoxolone is administered orally. In some embodiments, the carbenoxolone is administered at a dose of about 10 mg/day to about 10000 mg/day. In some embodiments, the carbenoxolone is administered at a dose of about 150mg/day to about 600 mg/day. In some embodiments, the method further comprises assessing the subject for symptoms of the polyQ tract expansion disease or disorder after administration of carbenoxolone and adjusting the dosage of carbenoxolone based on the assessment. In some embodiments, the subject exhibits a symptom associated with the polyQ tract disease or disorder. In some embodiments, the method comprises maintaining or decreasing the dosage of carbenoxolone , if the subject exhibits a desired change in a symptom associated with the polyQ tract disease or disorder. In some embodiments, the method comprises increasing the dosage of carbenoxolone, if the subject exhibits no desired change in a symptom associated with the polyQ tract disease or disorder. In some embodiments, the subject does not exhibit a clinically manifest symptom of the polyQ tract expansion disease or disorder. In some embodiments, the clinically manifest symptom is an impairment in motor function, an impairment in cognitive function, an behavioral impairment, a functional impairment, or an impairment in Total Functional Capacity (TFC), either alone or in any combination thereof. In some embodiments, the subject exhibits an elevated glucocorticoid level. In some embodiments, the elevated glucocorticoid level is an elevated cortisol level. In some embodiments, the elevated cortisol level is a blood plasma level of more than 350mol/l. In some embodiments, the elevated cortisol level is a blood plasma level of more than 700mol/l. In some embodiments, the carbenoxolone, or analog, salt, or solvate thereof, is administered in an amount effective to reduce the elevated glucocorticoid level. In some embodiments, the carbenoxolone, or analog, salt, or solvate thereof, is administered in an amount effective to reduce the elevated glucocorticoid level to a level observed or expected in a healthy subject. In some embodiments, the carbenoxolone, or an analog, salt, or solvate thereof is administered to the subject based on the subject exhibiting an elevated glucocorticoid level. In some embodiments, the carbenoxolone, or an analog, salt, or solvate thereof is administered to the subject based on the subject exhibiting an elevated cortisol level.

In some embodiments of the disclosure a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a compound chosen from the group of camptothecin, 10-hydroxycamptothecin, topotecan, irinotecan, 18β-Glycyrrhetinic acid, carbenoxolone, Etoposide, Ouabain, Proscillaridin A, Ethacrynic acid, or an analog, salt, or solvate of any of these compounds, and/or an Ikb1 inhibitor, Topoisomerase I inhibitor, Topoisomerase II inhibitor, Topoisomerase III inhibitor, Topoisomerase IIIα inhibitor, Topoisomerase IIIβ inhibitor, Na+/K+ ATPase inhibitor, or GST inhibitor, either alone, or in any combination. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of camptothecin, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of 10-hydroxycamptothecin, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of topotecan,

or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of irinotecan, or an analog, salt, or solvate thereof. In some embodiments, a method for treating a polyQ tract expansion disease or disorder is provided, comprising administering to a subject having or suspected of having a polyQ tract expansion disease or disorder or carrying a polyQ tract expansion mutation of a gene implicated in a polyQ tract expansion disease or disorder, or expressing a polyQ tract-expanded polypeptide implicated in a polyQ tract expansion disease or disorder, an effective amount of 18β-Glycyrrhetinic acid, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of an lkb1 inhibitor, or an analog, salt, or solvate thereof. LKB 1 is also known to those of skill in the art as liver kinase B 1, STK11, serine/threonine kinase 11, renal carcinoma antigen, or NY-REN-19. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase inhibitor, or an analog, salt, or solvate thereof. Topoisomerases, also referred to as topos or tops herein, are enzymes that manage the topological state of DNA in a cell, for example, by altering DNA molecule coiling, DNA catenation, and inter-molecular DNA entanglement. The structure and activity of topoisomerases of higher eukaryotes, including Drosophila and human, are well known to those of skill in the art (for an overview, see, e.g., James Champoux, DNA Topoisomerases: Structure, Function, and Mechanism. Annu. Rev. Biochem. 2001. 70:369-413; the entire contents of which are incorporated herein by reference). In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase I inhibitor, or an analog, salt, or solvate thereof. Topoisomerase I is also known to those of skill in the art as topoisomerase 1, topo I, topo 1, top I, or top 1. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase II inhibitor, or an analog, salt, or solvate thereof. Topoisomerase II is also known to those of skill in the art as topoisomerase 2, topo II, topo 2, top II, or top 2. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase III inhibitor, or an analog, salt, or solvate thereof. Topoisomerase III is also known to those of skill in the art as topoisomerase 3, topo III, topo 3, top III, or top 3. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase IIIα inhibitor, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a topoisomerase IIIβ inhibitor, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a Na+/K+ ATPase inhibitor, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of a GST inhibitor, or an analog, salt, or solvate thereof. In some embodiments,

a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of Etoposide, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of Ouabain, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of Proscillaridin, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of Ethacrynic acid, or an analog, salt, or solvate thereof. In some embodiments, a method for treating a polyQ tract expansion disease or disorder is provided, comprising administering to a subject having or suspected of having a polyQ tract expansion disease or disorder, or carrying a polyQ tract expansion mutation of a gene implicated in a polyQ tract expansion disease or disorder, or expressing a polyQ tract-expanded polypeptide implicated in a polyQ tract expansion disease or disorder, an effective amount of carbenoxolone, or an analog, salt, or solvate thereof. In some embodiments; the polyQ tract expansion disease or disorder is Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12). In some embodiments, the polyQ tract expansion disease or disorder is a polyQ tract expansion mutation in the ATN1, DRPLA, HTT, Androgen receptor on the X chromosome, ATXN1, ATXN2, ATXN3, ATXN12, CACNA1A, ATXN7, TBP, PPP2R2B, or SCA12 gene. In some embodiments, the subject expresses an ATN1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the subject expresses an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the lkb1 inhibitor is an antibody, or fragment thereof, an aptamer, or an adnectin, specifically binding lkb1. In some embodiments, the lkb1 inhibitor comprises an antisense nucleic acid or a nucleic acid encoding an antisense nucleic acid corresponding to a transcript of the lkb1 gene.

In some embodiments, the subject is a non-human mammal. In some embodiments, the subject is a human.

Some aspects of this invention provide a method for identifying an agent for the treatment of a polyQ tract expansion disease, comprising (a) contacting a cell expressing a polyQ tract expanded polypeptide fused to a detectable agent with a candidate agent; (b) determining expression of the polyQ tract expanded polypeptide and/or cellular morphology of the cell contacted with the candidate agent; (c) determining expression of the polyQ tract expanded polypeptide and/or cellular morphology representative of a cell expressing the polyQ tract expanded polypeptide, but not contacted with the candidate agent; and (d) comparing the expression and/or the cellular morphology determined in (b) and (c) to a reference or control expression and morphology representative of a cell not expressing the polyQ tract expanded polypeptide, wherein if the expression and the cellular morphology determined in (b) is more similar to the reference or control expression and morphology than the expression and the cellular morphology determined in (c), then the candidate agent is identified to be an agent for the treatment of a polyQ tract expansion disease, or if the expression and the cellular morphology determined in (b) is not more similar to the reference or control expression and morphology than the expression and the cellular morphology determined in (c), then the candidate agent is identified to not be an agent for the treatment of a polyQ tract expansion disease. In some embodiments, the polyQ tract expanded polypeptide is a polyQ

tract expanded polypeptide implicated in Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12), or a fragment of such a peptide. In some embodiments, the polyQ tract expanded polypeptide is a gene product of the ATN1, DRPLA, HTT, Androgen receptor on the X chromosome, ATXN1, ATXN2, ATXN3, CACNA1A, ATXN7, ATXN12, TBP, PPP2R2B, or SCA12 gene, or a fragment of such a gene product. In some embodiments, the cell is a neuronal or glial cell. In some embodiments, determining the expression of a polyQ tract expanded polypeptide is determining the level of aggregation of the polyQ tract expanded polypeptide. In some embodiments, a cell not expressing the polyQ tract expanded polypeptide is a cell expressing a non-pathogenic version of the polyQ tract expanded polypeptide. In some embodiments, determining expression of the polyQ tract expanded polypeptide comprises quantifying a level of expression, cellular distribution, subcellular localization, aggregation, absence or presence in a cell organelle, and/or cellular turnover of the polypeptide. In some embodiments, determining cellular morphology comprises quantifying cell volume; cell shape; cell size; area covered by a cell; cell context in a tissue; number, size, structure, morphology, and/or quality of cell-cell contacts or cell-cell connections; size, shape, volume, structure, and/or morphology of a cell organelle. In some embodiments, the cell is a neuronal or a glial cell and determining cellular morphology comprises quantifying axonal outgrowth, axon size, axon length, axonal connections, branching, blebbing, fasciculation, polypeptide aggregation, neuromere number, neuromere size, connection number, connection strength, projection length, branch point number, branch point distribution, or tissue organization. In some embodiments, determining is by cell imaging. In some embodiments, cell imaging is live-cell fluorescence imaging. In some embodiments, live-cell fluorescence imaging is performed by automated microscopy.

Some aspects of this invention provide a fusion protein, comprising (a) a polyQ tract expanded protein, or fragment thereof, wherein the fragment comprises the polyQ tract of the protein; and (b) a detectable protein or polypeptide. In some embodiments, the polyQ tract expanded protein is an ATN1 or DRPLA (NCBI RefSeq: NP 001007027.1) protein comprising a polyQ tract of more than 35 Q residues, an HTT (Huntingtin) protein (NCBI RefSeq: NP 002102) comprising a polyQ tract of more than 35 Q residues, an Androgen receptor protein (NCBI RefSeq: NP 000035) comprising a polyQ tract of more than 36 Q residues, an ATXN1 protein (NCBI RefSeq: NP 000323.2) comprising a polyQ tract of more than 35 Q residues, an ATXN2 protein (NCBI RefSeq: NP 002964.3) comprising a polyQ tract of more than 32 Q residues, an ATXN3 (NCBI RefSeq: NP 001121168.1) protein comprising a polyQ tract of more than 40 Q residues, a CACNA1A (NCBI RefSeq: NP 000059.3) protein comprising a polyQ tract of more than 18 Q residues, an ATXN7 protein (NCBI RefSeq: NP 001170858.1) comprising a polyQ tract of more than 17 Q residues, a TBP protein (NCBI RefSeq: NP 001165556.1) comprising a polyQ tract of more than 42 Q residues, or a PPP2R2B or SCA12 (NCBI RefSeq: NP 001120853.1) protein comprising a polyQ tract of more than 28 Q residues. In some embodiments, the polyQ tract expanded protein is an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the poly-Q tract is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 90, 91, 92, 93, 94, 95, 96, 97, 98, 99, 100,101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140 residues long. In some embodiments, the detectable protein or polypeptide is a fluorescent protein or polypeptide. In some embodiments, the fluorescent protein or polypeptide is GFP, eGFP, YFP, RFP, mRFP, mTomato, mCherry, dsRed, or CFP.

Some aspects of this invention provide a modified cell, comprising (a) a nucleic acid construct comprising a nucleic acid sequence encoding a polyQ tract expanded protein fused to a fluorescent protein under the control of a promoter; and (b) a detectable marker allowing for visualization of cell morphology. In some embodiments, the detectable marker allowing for visualization of cell morphology is a fluorescent protein. In some embodiments, the fluorescent protein is membrane-binding fluorescent protein. In some embodiments, the fluorescent protein is GFP, eGFP, YFP, RFP, mRFP, or CFP. In some embodiments, the detectable marker is a dye. In some embodiments, the dye is a vital dye. In some embodiments, the vital dye is 5-carboxy-fluorescein diacetate AM. In some embodiments, the detectable marker is a detectably labeled antibody that binds to the surface of the cell. In some embodiments, the detectably labeled antibody is an antibody conjugated to a Cy dye. In some embodiments, the polyQ tract expanded protein is an ATN1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of

more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the polyQ tract expanded protein is an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the poly-Q tract is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 90, 91, 92, 93, 94, 95, 96, 97, 98, 99, 100,101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140 residues long.

In some embodiments, a cell culture is provided that comprises a cell described herein. In some embodiments, the culture consists of a substantially homogeneous population of cells.

Some aspects of this invention provide methods for the use of the agents, compounds, molecules, and compositions in the preparation of a medicament, particularly a medicament for the treatment of polyQ tract expansion diseases, for example, HD, are also provided.

Additional aspects, embodiments, advantages, features, and uses of the invention will become apparent from the following detailed description of non-limiting embodiments of the invention when considered in conjunction with the accompanying drawings.

## BRIEF DESCRIPTION OF THE DRAWINGS

**Figure 1.** Confocal microscopy images of Htt-Q15 (**A-C**) and Htt-Q138 (**D-F**) expressing *Drosophila* primary neural cultures plated on glass coverslips. The subcellular distribution of Htt (red channel), morphology of Htt expressing primary cultures (green channel, UAS-CD8GFP), and merged images are shown. Htt-Q15 (**B**) has a diffuse cytoplasmic distribution and fills most processes of cultured neurons, while Htt-Q138 forms large insoluble aggregates that accumulate in neurites and within cell bodies of neuromere clusters (**E**). Htt-Q15 and HttQ-138 expressing cultures also display different neuronal morphologies. Htt-Q15 cultures have long straight neurites (**A**), while Htt-Q138 cultures have shorter neurites that fail to extend from neuromere clusters leading to a club-like appearance (**D**). Scale bar: 100 μm. (G) Quantitative Western blot (n=4) showing that the relative expression level of $Htt15Q^1$ and $Htt138Q^1$ is comparable in strains used for primary culture screening (compare lanes 2 and 3). A Htt138Q strain with weaker expression is also shown (lane 4, $Htt138Q^2$). The strain genotypes that are listed on the bar graph (left to right) correspond to lanes 1-4 of the blot. Tubulin was used as a loading control. $p < 0.05$. Scale bar: 100 μm.

**Figure 2.** Htt-Q138 aggregation-inhibition screening in primary neural cultures using custom algorithms. (**A**) Scatter plot indicating the extent of Htt-Q138 aggregation following treatment with ~ 2600 small molecules. Loge ratio of HttQ-138 aggregates (small molecule treated well/DMSO treated well from the same screen plate) is plotted. The line denotes two standard deviations from the mean level of aggregates observed in the screen data set. Circled wells correspond to compounds that suppress aggregate formation and were subsequently analyzed in downstream validation studies. (**B,C**) Representative data set images collected via automated microscopy and analyzed with algorithms. (**B**) Htt-Q15 control cultures have few aggregates, while mutant Htt-Q138 cultures (**C**) have numerous aggregates. The exposure time used for image collection was optimized for Htt aggregate detection, which has a higher signal intensity than soluble Htt. This avoided pixel saturation at the upper end of the aggregate dynamic range, ensuring accurate aggregate quantification, although soluble Htt is not readily detectable in automated microscopy images. Image analysis was performed as described in the materials and methods. Scale bar: 200 μm.

**Figure 3.** Morphological analysis of Htt-Q138 aggregation inhibitors. (**A**) P-value scatter plot illustrating the ability of a subset of Htt-Q138 aggregation inhibitors to revert culture morphology towards Htt-Q15 controls. Circled compounds are the Camptothecin aggregation inhibitors. For morphological analysis, neurite (short, medium, long and average neurite length) and neuromere features (small, medium, large, average neuromere area) were used to compute statistical significance. (**B-E**) Representative automated microscopy images showing the neuronal morphology profiles of the *Drosophila* primary neural cultures plated on plastic, optical-bottom, 384-well plates. (**C**) Htt-Q138 primary neural cultures have dysmorphic neuronal profiles relative to Htt-Q15 controls (**B**). (**D, F**) Rescue of Htt-Q138 mutant morphology by treatment with 10-Hydroxy Camptothecin or Lkb-1 knockdown via RNAi. (**E**) An

example of a small molecule (Okadaic acid) found to suppress Htt-Q138 aggregation, but was found to have a 15Q morphology score since it exacerbated the mutant Htt-Q138 mutant morphology. Scale bar: 200 μm.

**Figure 4.** *In vitro* validation of small molecule screen hits. Confocal microscopy images of primary cultures plated on glass coverslips and treated with either DMSO (**A,B**) or test compounds (**C,D**). Primary neural cultures expressing Htt-Q138 have numerous aggregates in neurite processes and surrounding the cell bodies (**B**), while control HttQ15 expressing cultures do not (**A**). Htt15 is soluble and fills most neurite processes. Treatment of Htt-Q138 expressing cultures with Camptothecin (**C**) or 10-OH-Camptothecin (**D**) at 56 μM reduces aggregate formation and increases the proportion of soluble Htt-Q138 which fills neurite processes. Camptothecin treatment does not alter expression levels of Htt-Q138. (**E**) Quantification of altered Htt138Q distribution following Camptothecin treatment. An increase in the number of Htt138Q pixels/neuronal area (Htt-RFP pixels/neuromere and neurite GFP pixels) is observed in mutant cultures, suggesting an increase in Htt138Q solubility after drug treatment. * $p < 0.05$, $n = 4$, Scale bar: 100 μm.

**Figure 5.** *In vivo* validation of screen hits in HD model. (**A**) Viability scores (survival frequency scores) for HD larvae ($Elav^{c115}$-GAL4; UAS-Htt-Q138/+) after 5-day drug dosing in liquid culture. (**B**) Chemical structures of the Camptothecin and 18β-Glycyrrhetinic acid class of small molecules found to rescue Htt138Q toxicity in vivo. Shown are the structures for 10-OH-Camptothecin (Camptothecin class) and carbenoxolone (Na-salt, 18β-Glycyrrhetinic acid class). (**C-E**) Genetic interaction studies to assess the effect of lkb1 kinase reduction on Htt138Q toxicity. (**C**) Rescue of pupal lethality caused by Htt-Q138 following introduction of *lkb1* heterozygous background. Pan-neuronal expression of $Htt138Q^{1}$ causes pupal lethality (left) which can be rescued with the introduction of an lkb1 heterozygous background. (**D**) Quantitative Western blot analysis demonstrating *lkb1*-rescued HD adults have normal Htt-Q138 expression levels. A control deficiency, Df(3L)vin, which reduces Htt138Q expression is shown for comparison. (**E**) Lkb1 mutation rescues the climbing behavior of HD flies. 25 day-old Htt138Q flies (C155;UAS-$Htt138QmRFP^{2}$) have impaired climbing behavior as compared to controls. Introduction of an $Lkb1^{4A4-2}$ trans-heterozygous mutation into the $Htt138Q^{2}$ background improves climbing ability. * $p < 0.05$.

**Figure 6.** Exemplary structures of compounds tested for their ability to suppress Htt138Q neuronal toxicity.

## DETAILED DESCRIPTION OF CERTAIN EMBODIMENTS

A number of neurologic disorders are known to be caused by an increased number of CAG repeats in a genetic region encoding a protein. During protein synthesis, the expanded CAG repeats are translated into a series of uninterrupted glutamine (Q) residues forming what is known as a polyglutamine ("polyQ") tract. Without wishing to be bound by theory, it is believed that proteins comprising expanded polyglutamine tracts may be subject to increased aggregation. Such increased aggregation of proteins with expanded polyQ tract has been reported in various diseases and is believed to be causally connected to the specific disease. For example, in Huntington's disease (HD), it is believed that expansion of the polyQ tract in the coding region of the gene encoding the Huntingtin (Htt) protein beyond a number translating to a polyQ tract of about 35 Q residues results in HTT aggregation which in turn is associated with HD. Table 1 below lists exemplary diseases known to be associated with polyQ tract expansion, the gene/protein involved, or implicated, and the normal and pathogenic numbers of Q residues, or repeats, in the polyQ tract of the respective protein.

Table 1. Examples of polyQ tract expansion diseases.

| Disease | Gene | Normal polyQ repeats | Pathogenic polyQ repeats |
|---|---|---|---|
| DRPLA (Dentatorubropallidoluysian atrophy) | ATN1 or DRPLA | 6 - 35 | 49 - 88 |
| HD (Huntington's disease) | HTT (Huntingtin) | 10 - 35 | 35+ |
| SBMA (Spinobulbar muscular atrophy or Kennedy disease) | Androgen receptor on the X chromosome. | 9 - 36 | 38 - 62 |
| SCA1 (Spinocerebellar ataxia Type 1) | ATXN1 | 6 - 35 | 49 - 88 |
| SCA2 (Spinocerebellar ataxia Type 2) | ATXN2 | 14 - 32 | 33 - 77 |
| SCA3 (Spinocerebellar ataxia Type 3 or Machado-Joseph disease) | ATXN3 | 12 - 40 | 55 - 86 |
| SCA6 (Spinocerebellar ataxia Type 6) | CACNA1A | 4 - 18 | 21 - 30 |
| SCA7 (Spinocerebellar ataxia Type 7) | ATXN7 | 7 - 17 | 38 - 120 |
| SCA17 (Spinocerebellar ataxia Type 17) | TBP | 25 - 42 | 47 - 63 |
| SCA12 (Spinocerebellar ataxia Type 12) | PPP2R2B or SCA12 | 7 - 28 | 66 - 78 |

To identify Huntington's Disease therapeutics, we conducted high-content compound and RNAi suppressor screens for dystrophic neurites induced by Huntingtin with an expanded polyglutamine track expressed in *Drosophila* primary neuronal cultures. The screen identified *lkb1*, an upstream kinase in the mTOR/Insulin pathway, and a number of novel, FDA-approved drugs that were strong suppressors of mutant Huntingtin-induced neurotoxicity. These suppressors also restored viability in a *in vivo Drosophila* Huntington's Disease model.

***Methods and compositions for treating a polyQ tract expansion disease or disorder***

Some aspects of the invention relates to compounds and compositions for the treatment of polyQ tract expansion diseases or disorders, for example, the diseases and disorders described in Table 1. In some embodiments, a compound is provided that modulates a phenotype observed in a polyQ tract expansion associated disease in a desirable way. For example, in some embodiments, a compound or composition is provided that ameliorates aggregation of a polyQ tract expansion disease associated protein, or fragment thereof, comprising a polyQ tract of pathologic length. In some embodiments, the compound does not have significant cytotoxic side effects on the target cells. In some embodiments, the compound does have tolerable cytotoxic side effects on the target cells. In some embodiments, a compound of composition is provided that ameliorates a morphological change observed in cells expressing a polyQ tract expansion disease associated protein, or fragment thereof, comprising a polyQ tract of pathologic length.

Some aspects of this invention as disclosed are based on the surprising discovery that topoisomerase inhibitors are able to ameliorate cellular phenotypes typical for polyQ tract expansion disease, for example, Huntington's disease, while not exhibiting significant cytotoxicity in the target cells. Some aspects of this invention are based on the surprising discovery that compounds of the camptothecin class of topoisomerase inhibitors are able to ameliorate cellular phenotypes typical for polyQ tract expansion disease, for example, Huntington's disease, while not exhibiting significant cytotoxicity in the target cells.

Camptothecin is a cytotoxic quinoline alkaloid which inhibits the DNA enzyme topoisomerase I (also known as topo I, topoisomerase 1, topo 1, top 1, or top I). Because camptothecin can induce adverse side reaction in some subjects and at some dosages, various camptothecin derivatives have been developed. Camptothecins are in clinical use for the treatment of cancer. Currently, two camptothecins, topotecan and irinotecan, are FDA approved and are used in the clinic for cancer treatment.

Some aspects of this invention provide a method for treating a polyQ tract expansion disease or disorder, comprising administering to a subject having or suspected of having a polyQ tract expansion disorder or disease an effective amount of a compound provided herein. In some embodiments of the disclosure the compound being administered is camptothecin or a camptothecin derivative. In some embodiments, the camptothecin or camptothecin derivative is a compound described by Formula 1:

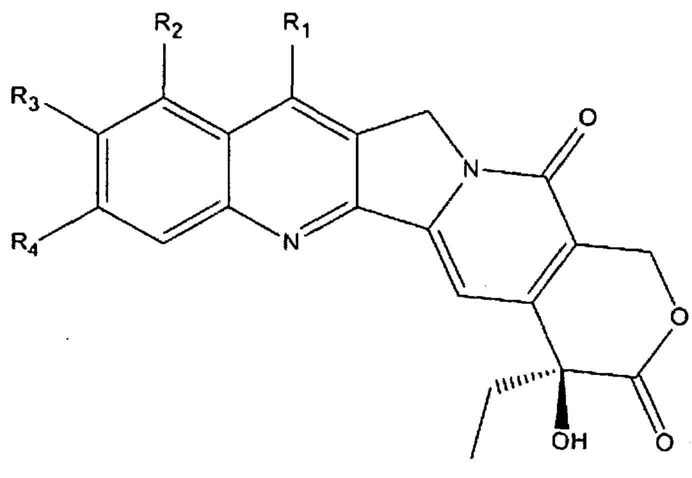

(Formula 1)

wherein

$R_1$ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; $-OR_C$; =O; $-C(=O)R_A$; $-CO_2R_A$; -CN; -SCN; $-SR_A$; $-SOR_A$; $-SO_2R_A$; $-NO_2$; $-N(R_A)_2$; $-NHC(O)R_A$; or $-C(R_A)_3$; wherein each occurrence of $R_A$ is independently hydrogen, a protecting group, aliphatic, heteroaliphatic, acyl, aryl, heteroaryl, alkoxy, aryloxy, alkylthio, arylthio, amino, alkylamino, dialkylamino,

heteroaryloxy, or heteroarylthio;

$R_2$ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; $-OR_B$; =O; $-C(=O)R_B$; $-CO_2R_B$; -CN; -SCN; $-SR_B$; $-SOR_B$; $-SO_2R_B$; - $NO_2$; $-N(R_B)_2$; $-NHC(O)R_B$; or $-C(R_B)_3$; wherein each occurrence of $R_B$ is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio;

$R_3$ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; $-OR_C$; =O; $-C(=O)R_C$; $-CO_2R_C$; -CN; -SCN; $-SR_C$; $-SOR_C$; $-SO_2R_C$; - $NO_2$; $-N(R_C)_2$; $-NHC(O)R_C$; or $-C(R_C)_3$; wherein each occurrence of $R_C$ is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio; and

$R_4$ is hydrogen; halogen; cyclic or acyclic, substituted or unsubstituted, branched or unbranched aliphatic; cyclic or acyclic, substituted or unsubstituted, branched or unbranched heteroaliphatic; substituted or unsubstituted, branched or unbranched acyl; substituted or unsubstituted, branched or unbranched aryl; substituted or unsubstituted, branched or unbranched heteroaryl; $-OR_D$; =O; $-C(=O)R_D$; $-CO_2R_D$; -CN; -SCN; $-SR_D$; $-SOR_D$; $-SO_2R_D$; - $NO_2$; $-N(R_D)_2$; $-NHC(O)R_D$; or $-C(R_D)_3$; wherein each occurrence of $R_D$ is independently hydrogen, a protecting group, aliphatic, heteroaliphatic, acyl, aryl, heteroaryl, alkoxy, aryloxy, alkylthio, arylthio, amino, alkylamino, dialkylamino, heteroaryloxy, or heteroarylthio.

In some embodiments, the camptothecin administered to a subject having or suspected of having a poly-Q tract expansion disorder or disease is substituted at position 7, 9, 10 and/or 11 (C atom having a covalent bond to R1, R2, R3, and R4, respectively). For example, in some embodiments, the camptothecin is 10-Hydroxycamptothecin. In some embodiments, the camptothecin comprises an enlarged lactone ring, for example, a lactone ring that is enlarged by one methylene unit (e.g., homocamptothecin). In some embodiments, the camptothecin comprises an electron-withdrawing group, for example, an amino, nitro, bromo or chloro group, at position 9 and/or 10 and/or a hydroxyl group at position 10 and/or 11. In some embodiments, the camptothecin is a hexacyclic camptothecin analog, comprising, for example, a methylenedioxy or ethylenedioxy group connected between position 10 and 11 to form a 5 or 6 membered ring. In some embodiments, the camptothecin is Lurtotecan, a 10, 11-ethylenedioxy camptothecin analogue with a 4-methylpiperazino-methylene at position 7.

Some exemplary camptothecin derivatives that are useful according to some embodiments of the invention are given in Table 2 below.

TABLE 2. Exemplary Camptothecin derivatives.

| Analogue | R1 | R2 | R3 | R4 |
|---|---|---|---|---|
| Topotecan | -H | $CH_2N(CH_3)_2$ | -OH | H |
| Irinotecan | $CH_2CH_3$ | H | 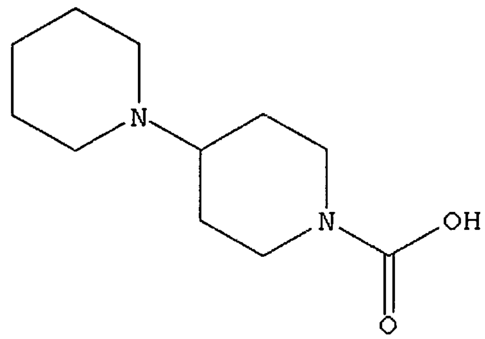 | H |
| DB 67 | 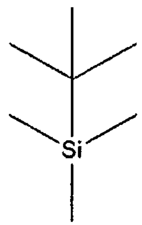 | H | OH | H |
| BNP 1350 | $CH_2CH_2Si(CH_3)_3$ | H | H | H |
| Exatecan | 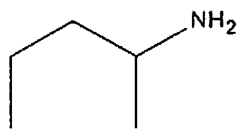 | | $CH_3$ | F |
| Lurtotecan | 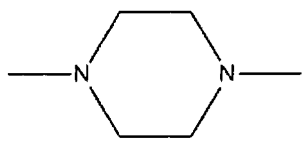 | H | 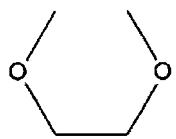 | |
| ST 1481 | $CH{=}NOC(CH_3)_3$ | H | H | H |
| CKD 602 | $CH_2CH_2NHCH(CH_3)_2$ | H | H | H |

Some aspects of this invention provide a method for treating a polyQ tract expansion disease or disorder, comprising administering to a subject having or suspected of having a polyQ tract expansion disorder or disease a compound provided herein. In some embodiments, the method comprises administering a compound provided in Table 3, Table 4, or Table 5. In some embodiments, the compound is chosen from the group of camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds.

In some embodiments of the disclosure a method for treating a polyQ tract expansion disease or disorder is provided, comprising administering to a subject having or suspected of having a polyQ tract expansion disorder or disease, for example, HD, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds at a dosage that is sufficient to achieve a desirable clinical result in the subject, but is non-toxic to the subject. In some embodiments, the compound, analog, salt, or solvate is administered to a subject having or suspected of having a polyQ tract expansion disease at a dose in the range of 0.1 mg to 10,000 mg per day. In some embodiments, the compound, analog, salt, or solvate is administered to a subject having or suspected of having a polyQ tract expansion disease at a dose of more than 10,000 mg per day.

For example, in some embodiments, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds is administered to a subject having or suspected of having a polyQ tract expansion disease at a dose of about 10 mg/day, about 20 mg/day, about 30 mg/day, about 40 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day, about 400 mg/day, about 450 mg/day, about 500 mg/day, about 550 mg/day, about 600 mg/day, about 650 mg/day, about 700 mg/day, about 750 mg/day, about 800 mg/day, about 850 mg/day, about 900 mg/day, about 950 mg/day, about 1000 mg/day, about 1050 mg/day, about 1100 mg/day, about 1150 mg/day, about 1200 mg/day, about 1250 mg/day, about 1300 mg/day, about 1350 mg/day, about 1400 mg/day, about 1450 mg/day, about 1500 mg/day, about 1550 mg/day, about 1600 mg/day, about 1650 mg/day, about 1700 mg/day, about 1750 mg/day, about 1800 mg/day, about 1850 mg/day, about 1900 mg/day, about 1950 mg/day, or about 2000 mg/day.

In some embodiments, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds is administered to a subject having or suspected of having a polyQ tract expansion disease at a dose that is determined based on the body weight of the subject (e.g., mg of compound (e.g., carbenoxolone) per kg of body weight of the subject), for example, at a dose of about 0.01 mg/kg, about 0.02 mg/kg, about 0.025 mg/kg, about 0.03 mg/kg, about 0.04 mg/kg, about 0.05 mg/kg, about 0.06 mg/kg, about 0.07 mg/kg, about 0.08 mg/kg, about 0.09 mg/kg, about 0.1mg/kg, about 0.2 mg/kg, about 0.25mg/kg, about 0.3 mg/kg, about 0.4 mg/kg, about 0.5 mg/kg, about 0.6 mg/kg, about 0.7 mg/kg, about 0.8 mg/kg, about 0.9 mg/kg, or about 1 mg/kg. In some embodiments, the dosage is at more than 1mg/kg. In some embodiments, for example, in some embodiments, in which carbenoxolone, or an analog, salt, or solvate thereof, is administered to a subject, the amounts in mg/kg provided herein are given as a daily dose, e.g., as 0.01 mg/kg/day, 0.02 mg/kg/day, *etc.*

In some embodiments, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds is administered to a subject having or suspected of having a polyQ tract expansion disease at a dose in the range of about 0.1 mg/day - about 1 mg/day, about 1 mg/day - about 10 mg/day, about 10 mg/day - about 100 mg/day, about 100 mg/day - about 300 mg/day, about 100mg/day - about 1g/day, about 100mg/day - about 750 mg/day, about 100mg/day - about 700 mg/day, about 100mg/day - about 500 mg/day, about 300 mg/day - about 500 mg/day, about 500 mg/day - about 600 mg/day, about 600 mg/day - about 650 mg/day, about 650 mg/day - about 700 mg/day, about 700 mg/day - about 750 mg/day, about 750 mg/day - about 800 mg/day, about 800 mg/day - about 900 mg/day, about 900 mg/day - about 1000 mg/day, about 1000 mg/day - about 1250 mg/day, about 1250 mg/day - about 1500 mg/day, about 1500 mg/day - about 2000 mg/day, about 2000 mg/day - about 5000 mg/day, or about 5000 mg/day - about 10000 mg/day. In some embodiments, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds is administered to a subject having or suspected of having a polyQ tract expansion disease orally, for example, via a pill or tablet. In some embodiments, oral administration is performed once, twice, or three times daily. In some embodiments, a compound described herein, for example, camptothecin, 10-hydroxycamptothecin, topotecan, irinotecan, 18β-Glycyrrhetinic acid, carbenoxolone, Etoposide, Ouabain, Proscillaridin A, and/or Ethacrynic acid, or a pharmaceutically acceptable analog, salt, or solvate of any of these compounds is administered to a subject having or suspected of having a polyQ tract expansion disease at a dose of about

30 mg/day, about 60 mg/day, about 90 mg/day, about 120 mg/day, about 150 mg/day, about 180 mg/day, about 210 mg/day, about 240 mg/day, about 270 mg/day, about 300 mg/day, about 330 mg/day, about 360 mg/day, about 390 mg/day, about 420 mg/day, about 450 mg/day, about 480 mg/day, about 510 mg/day, about 540 mg/day, about 570 mg/day, about 600 mg/day, about 630 mg/day, about 660 mg/day, about 690 mg/day, about 720 mg/day, about 750 mg/day, about 780 mg/day, about 810 mg/day, about 840 mg/day, about 870 mg/day, about 900 mg/day, about 930 mg/day, about 960 mg/day, about 990 mg/day, about 1020 mg/day, about 1050 mg/day, about 1080 mg/day, about 1110 mg/day, about 1140 mg/day, about 1170 mg/day, about 1200 mg/day, about 1230 mg/day, about 1260 mg/day, about 1290 mg/day, about 1320 mg/day, about 1350 mg/day, about 1380 mg/day, about 1410 mg/day, about 1440 mg/day, about 1470 mg/day, about 1500 mg/day, about 1530 mg/day, about 1560 mg/day, about 1590 mg/day, about 1620 mg/day, about 1650 mg/day, about 1680 mg/day, about 1710 mg/day, about 1740 mg/day, about 1770 mg/day, about 1800 mg/day, about 1830 mg/day, about 1860 mg/day, about 1890 mg/day, about 1920 mg/day, about 1950 mg/day, about 1980 mg/day, about 2010 mg/day, about 2040 mg/day, about 2070 mg/day, about 2100 mg/day, about 2130 mg/day, about 2160 mg/day, about 2190 mg/day, about 2220 mg/day, about 2250 mg/day, about 2280 mg/day, about 2310 mg/day, about 2340 mg/day, about 2370 mg/day, or about 2400 mg/day.

In some embodiments, a method for treating a polyQ tract expansion disease or disorder is provided, comprising administering to a subject having or suspected of having a polyQ tract expansion disorder or disease an 18β-Glycyrrhetinic acid analog. In some embodiments, a method for treating a polyQ tract expansion disease or disorder is provided, comprising administering to a subject having or suspected of having a polyQ tract expansion disorder or disease the 18β-Glycyrrhetinic acid analog carbenoxolone, a carbenoxolone analog or derivative, or a salt of carbenoxolone or of a carbenoxolone analog or derivative.

Carbenoxolone is also known to those of skill in the art as (3β)-3-[(3-carboxy-propanoyl)oxy]-11-oxoolean-12-en-30-oic acid; as (3β,20β)-3-(3-carboxy-1-oxopropoxy)-11-oxoolean-12-en-29-oic acid; as (2S,4aS,6aS,6bR,8aR,10S,12aS,12bR,14bR) -10-(3-carboxy-propanoyloxy)-2,4a,6a,6b,9,9,12a-heptamethyl-13-oxo-1,2,3,4,4a,5,6,6a,6b,7,8,8a,9,10,11, 12,12a,12b,13,14b-icosahydropicene-2-carboxylic acid; as butanedioic acid, mono[(3beta)-30-hydroxy-11,30-dioxoolean-12-en-3-yl] ester; as glycerrhetinic acid hydrogen succinate; as glycyrrhetic acid hydrogen succinate; as enoxolone succinate; and as CBX).

Salts of carbenoxolone, or of its analogs or derivatives, that are useful according to some aspects of this invention are well known to those of skill in the art and include, but are not limited to sodium and disodium salts (e.g., carbenoxolone sodium and carbenoxolone disodium salts). Other salts that are useful according to some aspects of the invention include pharmaceutically acceptable salts (e.g., pharmaceutically acceptable carbenoxolone salts).

Useful carbenoxolone analogs and derivatives will be apparent to those of skill in the art. Such useful analogs and derivatives include, but are not limited to, BX24, oleanoic acid sodium hydrogen succinate (OSS), acetoxolone, and cicloxolone. Useful carbenoxolone analogs further include, but are not limited to deuterated carbenoxolone analogs, in which one or more H atoms of the carbenoxolone molecule, or of a carbenoxolone analog molecule, is substituted with a deuterium atom.

The structure of carbenoxolone is well known to those of skill in the art and an exemplary representation of the structure of carbenoxolone is provided in Formula 2:

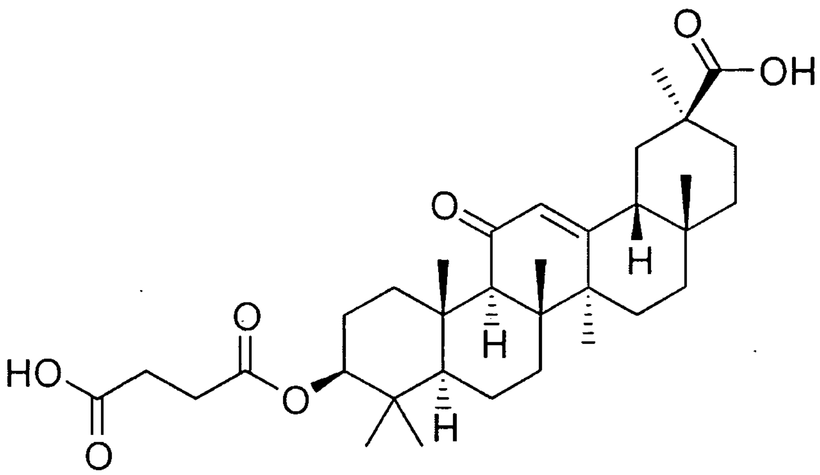

(Formula 2)

Carbenoxolone is in clinical use, for example, for the treatment of oesophagal ulceration, inflammation, and for the treatment of oral and perioral lesions. Some aspects of this invention are based on the surprising recognition that carbenoxolone is also useful for treating a polyQ tract expansion disease or disorder.

In some embodiments, a method for treating a polyQ tract expansion disease or disorder is provided, comprising administering to a subject having or suspected of having a polyQ tract expansion disorder or disease carbenoxolone, or a carbenoxolone analog or derivative, or a pharmaceutically acceptable salt of carbenoxolone or a carbenoxolone analog or derivative. In some embodiments, the method includes administering to a subject having or suspected of having Huntington's Disease an amount of carbenoxolone, or of a carbenoxolone analog or derivative, that is sufficient, either alone or in combination with additional administered amounts, to achieve a reduction in the aggregation of Htt protein, a reduction in the number or size of inclusion bodies, a normalization of brain tissue homeostasis (e.g. improved survival of neuronal cells and/or reduction in astrocytes), an improvement in cognitive and motor function, and/or a slowing or reversal of a personality change commonly associated with HD.

In some embodiments, a method for treating a polyQ tract expansion disease or disorder is provided, comprising administering to a subject having or suspected of having a polyQ tract expansion disorder or disease, for example, HD, carbenoxolone, or a carbenoxolone analog or derivative, or a pharmaceutically acceptable salt of carbenoxolone or a carbenoxolone analog or derivative, via an enteral administration route. For example, in some embodiments, carbenoxolone, an analog or derivative, or salt thereof, is administered orally to the subject.

Formulations of carbenoxolone, or a carbenoxolone analog or derivative, for oral administration are well known to those of skill in the art and include, but are not limited to those formulations of carbenoxolone in the drugs used under the trade names BIOGASTRONE™; BIOPLEX™; BIORAL™; CARBOSAN™; DUOGASTRONE™; GASTRAUSIL™; HERPESAN™; NEOGEL™; ROWADERMAT™; SANODIN™; ULCUS-TABLINEN™, and PYROGASTRONE™. Additional suitable formulations of carbenoxolone or a carbenoxolone analog or derivative, for oral administration to a subject having or suspected of having a polyQ tract disorder or disease will be apparent to those of skill in the art and include, but are not limited to formulation in capsules, tablets, lozenges, suspensions, syrups, elixirs, and emulsions.

In some embodiments, a method for treating a polyQ tract expansion disease or disorder is provided, comprising administering to a subject having or suspected of having a polyQ tract expansion disorder or disease, for example, HD, carbenoxolone, or a carbenoxolone analog or derivative, or a pharmaceutically acceptable salt of carbenoxolone or a carbenoxolone analog or derivative, via a parenteral administration route, for example, via subcutaneous, intramuscular, intraperitoneal, or intravenous injection. Some methods and formulation for parenteral administration of carbenoxolone or carbenoxolone analogs and derivatives are provided herein and additional methods and formulations are well known to those of skill in the art.

In some embodiments, a method for treating a polyQ tract expansion disease or disorder is provided, comprising administering to a subject having or suspected of having a polyQ tract expansion disorder or disease, for example, HD, carbenoxolone, or a carbenoxolone analog or derivative, or a pharmaceutically acceptable salt of carbenoxolone or of a carbenoxolone analog or derivative, at a dosage that is sufficient to achieve a desirable clinical result in the subject, but is non-toxic to the subject. In some embodiments, carbenoxolone or a carbenoxolone analog or derivative, or a salt thereof, is administered to a subject having or suspected of having a polyQ tract expansion disease at a dose in the range of 0.1 mg to 10,000 mg per day. In some embodiments, carbenoxolone or a carbenoxolone analog or derivative, or a salt thereof, is administered to a subject having or suspected of having a polyQ tract expansion disease at a dose of more than 10,000 mg per day.

For example, in some embodiments, carbenoxolone or a carbenoxolone analog or derivative, or a salt thereof, is administered to a subject having or suspected of having a polyQ tract expansion disease at a dose of about 10 mg/day, about 20 mg/day, about 30 mg/day, about 40 mg/day, about 50 mg/day, about 60 mg/day, about 70 mg/day, about 80 mg/day, about 90 mg/day, about 100 mg/day, about 150 mg/day, about 200 mg/day, about 250 mg/day, about 300 mg/day, about 350 mg/day, about 400 mg/day, about 450 mg/day, about 500 mg/day, about 550 mg/day, about 600 mg/day, about 650 mg/day, about 700 mg/day, about 750 mg/day, about 800 mg/day, about 850 mg/day, about 900 mg/day, about 950 mg/day, about 1000 mg/day, about 1050 mg/day, about 1100 mg/day, about 1150 mg/day, about 1200 mg/day, about 1250 mg/day, about 1300 mg/day, about 1350 mg/day, about 1400 mg/day, about 1450 mg/day, about 1500 mg/day, about 1550 mg/day, about 1600 mg/day, about 1650 mg/day, about 1700 mg/day, about 1750 mg/day, about 1800 mg/day, about 1850 mg/day, about 1900 mg/day, about 1950 mg/day, or about 2000 mg/day.

In some embodiments, carbenoxolone or a carbenoxolone analog or derivative, or a salt thereof, is administered to a subject having or suspected of having a polyQ tract expansion disease at a dose in the range of about 0.1 mg/day - about 1 mg/day, about 1 mg/day - about 10 mg/day, about 10 mg/day - about 100 mg/day, about 100 mg/day - about 300 mg/day, about 300 mg/day - about 500 mg/day, about 500 mg/day - about 600 mg/day, about 600 mg/day - about 650 mg/day, about 650 mg/day - about 700 mg/day, about 700 mg/day - about 750 mg/day, about 750 mg/day - about 800 mg/day, about 800 mg/day - about 900 mg/day, about 900 mg/day - about 1000 mg/day, about 1000 mg/day - about 1250 mg/day, about 1250 mg/day - about 1500 mg/day, about 1500 mg/day - about 2000 mg/day, about 2000 mg/day - about 5000 mg/day, or about 5000 mg/day - about 10000 mg/day.

In some embodiments, carbenoxolone or a carbenoxolone analog or derivative, or a salt thereof, is administered to a subject having or suspected of having a polyQ tract expansion disease orally, for example, via a pill or tablet. In some embodiments, oral administration is performed once, twice, or three times daily. In some embodiments, carbenoxolone or a carbenoxolone analog or derivative, or a salt thereof, is administered to a subject having or suspected of having a polyQ tract expansion disease at a dose of about 30 mg/day, about 60 mg/day, about 90 mg/day, about 120 mg/day, about 150 mg/day, about 180 mg/day, about 210 mg/day, about 240 mg/day, about 270 mg/day, about 300 mg/day, about 330 mg/day, about 360 mg/day, about 390 mg/day, about 420 mg/day, about 450 mg/day, about 480 mg/day, about 510 mg/day, about 540 mg/day, about 570 mg/day, about 600 mg/day, about 630 mg/day, about 660 mg/day, about 690 mg/day, about 720 mg/day, about 750 mg/day, about 780 mg/day, about 810 mg/day, about 840 mg/day, about 870 mg/day, about 900 mg/day, about 930 mg/day, about 960 mg/day, about 990 mg/day, about 1020 mg/day, about 1050 mg/day, about 1080 mg/day, about 1110 mg/day, about 1140 mg/day, about 1170 mg/day, about 1200 mg/day, about 1230 mg/day, about 1260 mg/day, about 1290 mg/day, about 1320 mg/day, about 1350 mg/day, about 1380 mg/day, about 1410 mg/day, about 1440 mg/day, about 1470 mg/day, about 1500 mg/day, about 1530 mg/day, about 1560 mg/day, about 1590 mg/day, about 1620 mg/day, about 1650 mg/day, about 1680 mg/day, about 1710 mg/day, about 1740 mg/day, about 1770 mg/day, about 1800 mg/day, about 1830 mg/day, about 1860 mg/day, about 1890 mg/day, about 1920 mg/day, about 1950 mg/day, about 1980 mg/day, about 2010 mg/day, about 2040 mg/day, about 2070 mg/day, about 2100 mg/day, about 2130 mg/day, about 2160 mg/day, about 2190 mg/day, about 2220 mg/day, about 2250 mg/day, about 2280 mg/day, about 2310 mg/day, about 2340 mg/day, about 2370 mg/day, or about 2400 mg/day.

In some embodiments, a compound described herein (e.g., a compound of Formula 1 or Formula 2) is administered to a subject carrying a mutation associated with a polyQ tract expansion disease (e.g., a pathologic polyQ tract expansion of a gene product described in Table 1), or expressing a polyQ tract expanded polypeptide implicated in a polyQ tract expansion disease, for example 35+ polyQ Huntingtin, before a clinical symptom of the polyQ tract expansion disease manifests. For example, some embodiments provide methods of administering a 18β-Glycyrrhetinic acid analog, for example, carbenoxolone, to a subject expressing a polyQ tract-expanded Huntingtin polypeptide, before the patient manifests a clinical symptom of HD. In some embodiments, the compound is administered based on the subject carrying the polyQ tract expansion mutation or expressing the polyQ tract-expanded polypeptide. In some embodiments, the compound is administered to prevent or delay the onset of, or mitigate the severity of a clinical symptom of the polyQ tract disease.

In some such pre-symptomatic treatment embodiments, the administration of the compound, for example, of an 18β-Glycyrrhetinic acid analog (e.g., carbenoxolone), prevents the onset of clinical symptoms of the disease (e.g., HD) in the subject, while in other embodiments, the onset of a clinical manifest symptom of the disease is merely delayed as compared to an untreated subject. In some pre-symptomatic treatment embodiments, the administration of the compound, for example, of the 18β-Glycyrrhetinic acid analog (e.g., carbenoxolone), mitigates the severity of a symptom of the disease, for example, the severity of a motor, behavioral, or cognitive impairment associated with the polyQ tract expansion disease. In some embodiments, the administration of the compound prior to clinical symptom manifestation delays the progression of the polyQ tract expansion disease once symptoms develop.

In some embodiments, a compound described herein (e.g., in Formula 1 or Formula 2) is chronically administered to a subject carrying a pathologic polyQ tract expansion mutation, or expressing a polyQ tract-expanded polypeptide, for example, for at least 1 month, at least 2 months, at least 3 months, at least 4 months, at least 5 months, at least 6 months, at least 12 months, at least 18 months, at least 2 years, at least 3 years, at least 4 years, at least 5 years, at least 6 years, at least 7 years, at least 8 years, at least 9 years, at least 10 years, at least 15 years, at least 20 years, at least 25 years, at least 30 years, at least 35 years, at least 40 years, or at least 50 years. In some such embodiments, the compound is administered at a dose that is non-toxic in long-term administration. In some such embodiments, the compound is administered at the highest, non-toxic dose. In some embodiments, the compound is administered at the lowest dose effective to prevent, delay, or mitigate the severity of a clinically manifest symptom of the disease. In some embodiments, the compound, for example, 18β-Glycyrrhetinic acid, carbenoxolone, or a pharmaceutically acceptable salt, solvate, analog, or derivative thereof, is administered at a dose described herein, for example, at a dose of about 30 mg/day, about 60 mg/day, about 90 mg/day, about 120 mg/day, about 150 mg/day, about 180 mg/day, about 210 mg/day, about 240 mg/day, about 270 mg/day, about 300 mg/day, about 330 mg/day, about 360 mg/day, about 390 mg/day, about 420 mg/day, about 450 mg/day, about 480 mg/day, about 510 mg/day, about 540 mg/day, about 570 mg/day, about 600 mg/day, about 630 mg/day, about 660 mg/day, about 690 mg/day, about 720 mg/day, about 750 mg/day, about 780 mg/day, about 810 mg/day, about 840 mg/day, about 870 mg/day, about 900 mg/day, about 930 mg/day, about 960 mg/day, about 990 mg/day, about 1020 mg/day, about 1050 mg/day, about 1080 mg/day, about 1110 mg/day, about 1140 mg/day, about 1170 mg/day, about 1200 mg/day, about 1230 mg/day, about 1260 mg/day, about 1290 mg/day, about 1320 mg/day, about 1350 mg/day, about 1380 mg/day, about 1410 mg/day, about 1440 mg/day, about 1470 mg/day, about 1500 mg/day, about 1530 mg/day, about 1560 mg/day, about 1590 mg/day, about 1620 mg/day, about 1650 mg/day, about 1680 mg/day, about 1710 mg/day, about 1740 mg/day, about 1770 mg/day, about 1800 mg/day, about 1830 mg/day, about 1860 mg/day, about 1890 mg/day, about 1920 mg/day, about 1950 mg/day, about 1980 mg/day, about 2010 mg/day, about 2040 mg/day, about 2070 mg/day, about 2100 mg/day, about 2130 mg/day, about 2160 mg/day, about 2190 mg/day, about 2220 mg/day, about 2250 mg/day, about 2280 mg/day,

about 2310 mg/day, about 2340 mg/day, about 2370 mg/day, or about 2400 mg/day. In some embodiments, the compound, for example, carbenoxolone, is administered at a dose recommended by the manufacturer, or approved or accepted by those of skill in the art to be safe for long-term administration.

In some embodiments, the pre-symptomatic treatment methods provided herein further comprise monitoring the subject for a clinical manifestation of a symptom associated with the polyQ tract disorder. Clinical symptoms of polyQ tract diseases and methods for their assessment in subjects having or suspected to have such a disease are well known to those of skill in the art. For example, clinical symptoms of HD and methods for their diagnosis and quantification have been published in the Unified Huntington's Disease Rating Scale (UHDRS, Huntington Study Group (Kieburtz K, primary author). The Unified Huntington's Disease Rating Scale: Reliability and Consistency. Mov Dis 1996;11:136-142; the entire contents of which are incorporated herein by reference).

Some of the compounds disclosed herein, for example, some 18β-Glycyrrhetinic acid analogs (e.g., carbenoxolone), can decrease the level of a glucocorticoid, for example, a cortisol level, when administered to a subject, for example, to a subject exhibiting an elevated glucocorticoid level. Recent studies have shown that neuroendocrine defects occur in HD patients (Hult et al., 2011). Specifically, HPA-axis (hypothalamus-pituitary-adrenal gland) dysregulation causes systemic elevation of the stress hormone cortisol. Increased cortisol levels are observed in both pre-symptomatic and symptomatic HD patients (van Duijn et al., 2010; Heuser et al., 1991; Aziz et al., 2009; Saleh et al., 2009). Stress research has shown that chronic cortisol exposure is neurotoxic and causes brain shrinkage particularly in the hippocampus (Lupien et al., 1998). The toxic effects of chronic cortisol exposure are not fully understood but they may be related to altered glucocorticoid receptor signaling. Chronic cortisol exposure is known to suppresses neuronal BDNF production, exacerbate glutamate toxicity and to oppose the action of insulin. It is well established that decreased BDNF levels, and glutamate toxicity from increased NMDA receptor signaling contributes to basal ganglia degeneration in HD (McEwen, 2010). Several studies have also found that there is decreased glucose metabolism in the basal ganglia of presymptomatic and symptomatic HD patients (Kuhl, 1982; Young, 1986; Hayden, 1986; Kuwert, 1990; Antonini, 1996). Together these results suggest, that restoring cortisol balance in HD patients has the potential for both immediate and long-term benefits. 11-beta-hydroxysteroid dehydrogenase 1 (HSD1) is a brain enzyme that regulates the production of cortisol in the brain. HSD1 is expressed widely in the forebrain (including the basal ganglia), hippocampus and cerebellum by both neurons and glia. Although the majority of cortisol produced in the body is generated by the adrenal glands, after activation of the HPA-axis, cortisol is unstable and is rapidly catabolized into the inactive analogue cortisone soon after it is released into the blood. The action of brain HSD1 then locally converts inactive cortisone to cortisol to sustain the effects of the active molecule for extended periods of time. One example of a compound disclosed herein that can decrease elevated corticosteroid levels is carbenoxolone.

Carbenoxolone is a steroid-like molecule with lipophilic properties that can cross the blood brain barrier (BBB) and reduce the production of active cortisol. Knock-out studies in mice have shown that loss of HSD1 enhances cognition in aged animals (Yau et al., 2007). Similarly, effects have been observed in rodent and human studies after pharmacological inhibition of the enzyme. For references, see Antonini A, Leenders KL, Spiegel R, Meier D, Vontobel P, Weigell-Weber M, Sanchez-Pernaute R, de Yébenez JG, Boesiger P, Weindl A, Maguire RP (1996) Striatal glucose metabolism and dopamine D2 receptor binding in asymptomatic gene carriers and patients with Huntington's disease. Brain Dec;119(Pt 6):2085-2095; Aziz NA, Pijl H, Frölich M, van der Graaf AW, Roelfsema F, Roos RA (2009) Increased hypothalamic -pituitary-adrenal axis activity in Huntington's disease. J Clin Endocrinol Metab 94:1223-1228; Hayden MR, Martin WR, Stoessl AJ, Clark C, Hollenberg S, Adam MJ, Ammann W, Harrop R, Rogers J, Ruth T, et al. (1986) Positron emission tomography in the early diagnosis of Huntington's disease. Neurology 36:888-894; Heuser IJ, Chase TN, Mouradian MM (1991) The limbic-hypothalamic-pituitary-adrenal axis in Huntington's disease. Biol Psychiatry 30:943-952; Hult S, Soylu R, Björklund T, Belgardt BF, Mauer J, Brüning JC, Kirik D, Petersén Å (2011) Mutant huntingtin causes metabolic imbalance by disruption of hypothalamic neurocircuits. Cell Metab 13:428-39; Kuhl DE, Phelps ME, Markham CH, Metter EJ, Riege WH, Winter J (1982) Cerebral metabolism and atrophy in Huntington's disease determined by 18FDG and computed tomographic scan. Ann Neurol 12:425-434; Kuwert T, Lange HW, Langen KJ, Herzog H, Aulich A, Feinendegen LE (1990) Cortical and subcortical glucose consumption measured by PET in patients with Huntington's disease. Brain Oct;113(Pt 5):1405-1423; Lupien SJ, de Leon M, de Santi S, Convit A, Tarshish C, Nair NP, Thakur M, McEwen BS, Hauger RL, Meaney MJ (1998) Cortisol levels during human aging predict hippocampal atrophy and memory deficits. Nat Neurosci 1:69-73; McEwen BS (2010) Stress, sex, and neural adaptation to a changing environment: mechanisms of neuronal remodeling. Ann NY Acad Sci Sep;1204 Suppl:E38-59; Saleh N, Moutereau S, Durr A, Krystkowiak P, Azulay JP, Tranchant C, Broussolle E, Morin F, Bachoud-Lévi AC, Maison P (2009) Neuroendocrine disturbances in Huntington's disease. PLoS One 4(3):e4962. Epub 2009 Mar 25; Schulte J, Sepp KJ, Wu C, Hong P, Littleton JT (2011) High-content chemical and RNAi screens for suppressors of neurotoxicity in a Huntington's disease model. PLoS One 6(8):e23841. Epub 2011 Aug 31; van Duijn E, Selis MA, Giltay EJ, Zitman FG, Roos RA, van Pelt H, van der Mast RC (2010) Hypothalamic-pituitary-adrenal axis functioning in Huntington's disease mutation carriers compared with mutation-negative first-degree controls. Brain Res Bull 83:232-237; Yau JL, McNair KM, Noble J, Brownstein D, Hibbernd C, Morton N, Mullins JJ, Morris RG, Cobb S, Seckl JR (2007) Enhanced hippocampal long-term potentiation

and spatial learning in aged 11beta-hydroxysteroid dehydrogenase type 1 knock-out mice. J Neurosci 27:10487-10496; and Young AB, Penney JB, Starosta-Rubinstein S, Markel DS, Berent S, Giordani B, Ehrenkaufer R, Jewett D, Hichwa R (1986) PET scan investigations of Huntington's disease: cerebral metabolic correlates of neurological features and functional decline. Ann Neurol 20:296-303; the entire contents of each of which are incorporated herein by reference.

It is important to note that the Drosophila data described herein is surprising in this regard, because there is no ortholog of the 11beta-HSD1 enzyme in Drosophila. Further, administering a different 11beta-HSD1 inhibitor to the Drosophila model described herein failed to produce the same result as carbenoxolone. Accordingly, carbenoxolone likely effects suppression of the toxicity of human mutant Huntingtin protein in the Drosophila model by an unknown, 1 1beta-HSD1-independent pathway.

Our experimental result that early dosing of carbenoxolone extends Drosophila HD model lifespan and improves motor control (Schulte et al., 2011) suggests that carbenoxolone may be useful as a prevention in pre-symptomatic individuals carrying the mutant Huntingtin gene.

Some aspects of this invention are based on the recognition that, controlling cortisol levels has clear benefits to pre-symptomatic as well as symptomatic subjects having a polyQ tract expansion disease or carrying a polyQ tract expansion mutation associated with a polyQ tract expansion disease. In some embodiments, a compound described herein, for example, carbenoxolone or an analog thereof, is administered to a subject carrying a polyQ tract expansion mutation in a gene that is associated with a polyQ tract expansion disease or disorder, or expressing a polyQ tract expanded polypeptide implicated in a polyQ tract expansion disease or disorder (see, e.g., Table 1 for exemplary genes and polypeptides), and exhibiting an elevated level of a glucocorticoid, for example, of cortisol. For example, in some embodiments, a compound described herein, for example, carbenoxolone or an analog thereof, is administered to a subject carrying a polyQ tract expansion mutation in the Huntingtin gene that is associated with HD, or expressing a polyQ tract expanded polypeptide implicated in HD, and exhibiting an elevated level of a glucocorticoid, for example, of cortisol. In some embodiments, the compound is administered to treat a symptom of a polyQ tract expansion disease, e.g., HD, for example, an impairment in motor or cognitive function (e.g., chorea). Some aspects of this invention are based on the recognition that the psychiatric and cognitive disturbances of polyQ tract expansion diseases may be largely due to chronic high cortisol in patients, and that at least part of the beneficial effect of some of the compounds described herein, e.g., carbenoxolone, is due to the reduction of cortisol levels effected by those compounds. This is consistent with the data from Drosophila disclosed herein, which shows that carbenoxolone improves chorea (defective motor coordination). This effect of carbenoxolone on chorea is unexpected given known cortisol effects on psychiatric and cognitive health. Chronically elevated cortisol is linked to hippocampal-dependent memory deficits and a decline in hippocampal volume. This is observed in hypercortisolemic (chronic high cortisol) but otherwise normal humans, hypercortisolemia rodent models, HD rodent models, and HD patients (see., e.g., Lupien SJ, de Leon M, de Santi S, Convit A, Tarshish C, Nair NP, Thakur M, McEwen BS, Hauger RL, Meaney MJ (1998) Cortisol levels during human aging predict hippocampal atrophy and memory deficits. Nat Neurosci 1:69-73; Grote HE, Bull ND, Howard ML, Van Dellen A, Blakemore C, Bartlett PF, Hannan AJ (2005) Cognitive disorders and neurogenesis deficits in Huntington's disease mice are rescued by fluoxetine. Eur J Neurosci 22:2081-2088; Spargo E, Everall IP, Lantos PL (1993) Neuronal loss in the hippocampus in Huntington's disease: a comparison with HIV infection. J Neurol Neurosurg Psychiatry 56:487-491; Schubert MI, Kalisch R, Sotiropoulos I, Catania C, Sousa N, Almeida OF, Auer DP (2008) Effects of altered corticosteroid milieu on rat hippocampal neurochemistry and structure - an in vivo magnetic resonance spectroscopy and imaging study. J Psychiatr Res 42:902-912; and Butters N, Sax D, Montgomery K, Tarlow S (1978) Comparison of the neuropsychological deficits associated with early and advanced Huntington's disease. Arch Neurol 35:585-589; the entire contents of each of which are incorporated herein by reference). Psychiatric effects that are linked to chronic elevated cortisol are: depression, anxiety, insomnia, compulsive behavior, and mood swings, and these changes are observed in both HD patients and hypercortisolemic but otherwise normal humans (see, e.g., Dubrovsky B (1993) Effects of adrenal cortex hormones on limbic structures: some experimental and clinical correlations related to depression. J Psychiatry Neurosci 18:4-16; Van Duijn E, Kingma EM, van der Mast RC (2007) Psychopathology in verified Huntington's disease gene carriers. J Neuropsychiatry Clin Neurosci 19:441-448; Amulf I, Nielsen J, Lohmann E, Schiefer J, Wild E, Jennum P, Konofal E, Walker M, Oudiette D, Tabrizi S, Durr A (2008) Rapid eye movement sleep disturbances in Huntington's disease. Arch Neurol 65:482-488; and Carroll BJ, Cassidy F, Naftolowitz D, Tatham NE, Wilson WH, Iranmanesh A, Liu PY, Veldhuis JD (2007) Pathophysiology of hypercortisolism in depression. Acta Psychiatr Scand Suppl 433-90-103; the entire contents of each of which are incorporated herein by reference)

Some aspects of this invention are based on the surprising discovery from the Drosophila experiments described herein that administration of a compound described herein to a subject carrying a polyQ tract expansion mutation in a gene implicated in a polyQ tract disease, e.g., an Htt polyQ tract expansion mutation, before the polyQ tract symptoms appeared, prevented or delayed onset of such symptoms in vivo. Accordingly, the compounds described herein are useful for the treatment of symptomatic subjects, but also for administration to pre-symptomatic subjects. In some embodiments, a compound described herein, for example, carbenoxolone or an analog thereof, is administered to a subject carrying a polyQ tract expansion mutation in a gene that is associated with a polyQ tract expansion disease or

disorder, or expressing a polyQ tract expanded polypeptide implicated in a polyQ tract expansion disease or disorder (see, e.g., Table 1 for exemplary genes and polypeptides), before a clinical symptom of the disease or disorder, for example, a motor impairment, cognitive impairment, behavioral impairment, restriction of independence, functional impairment, or and impairment in Total Functional Capacity (TFC) is clinically manifest. For example, in some embodiments, a compound described herein, for example, carbenoxolone or an analog or salt thereof, is administered to a subject carrying a polyQ tract expansion mutation in the Huntingtin gene that is associated with HD, or expressing a polyQ tract expanded polypeptide implicated in HD, before a clinical symptom of HD, for example, a motor impairment, cognitive impairment, behavioral impairment, restriction of independence, functional impairment, or and impairment in Total Functional Capacity (TFC) is clinically manifest. Accordingly, some embodiments provide methods of treating a polyQ tract expansion disease or disorder in pre-symptomatic subjects. (e.g., subjects that do not show outward signs of chorea, psychiatric disturbances or cognitive decline), in order to prevent or delay the onset of a symptom of the disease or disorder.

In some embodiments, a compound described herein, for example, carbenoxolone or an analog thereof, is administered to a subject carrying a polyQ tract expansion mutation in a gene that is associated with a polyQ tract expansion disease or disorder, or expressing a polyQ tract expanded polypeptide implicated in a polyQ tract expansion disease or disorder (see, e.g., Table 1 for exemplary genes and polypeptides), that exhibits an elevated glucocorticoid level, for example, an elevated cortisol level, before a clinical symptom of the disease or disorder, for example, a motor impairment, cognitive impairment, behavioral impairment, restriction of independence, functional impairment, or and impairment in Total Functional Capacity (TFC) is clinically manifest. For example, in some embodiments, a compound described herein, for example, carbenoxolone or an analog or salt thereof, is administered to a subject carrying a polyQ tract expansion mutation in the Huntingtin gene that is associated with HD, or expressing a polyQ tract expanded polypeptide implicated in HD, and exhibiting an elevated level of a glucocorticoid, for example, of cortisol, before a clinical symptom of HD, for example, a motor impairment, cognitive impairment, behavioral impairment, restriction of independence, functional impairment, or and impairment in Total Functional Capacity (TFC) is clinically manifest. Such treatment of pre-symptomatic subjects with elevated glucocorticoid levels allows for the prevention or the delay of the onset of symptoms associated with the disease or disorder.

The structure of numerous glucocorticoids, for example, of cortisol, as well as methods of measuring the level of glucocorticoids in a subject, and normal and elevated glucocorticoid levels, e.g., levels of cortisol present or expected to be present in a healthy subject or above the range deemed normal for a healthy subject, respectively, are well known to those of skill in the art. Elevated cortisol levels according to some aspects of this invention can be measured in body fluids including, but not limited to, urine, saliva, blood, blood plasma, and cerebrospinal fluid. Methods for such measurements are well known to those of skill in the art, and the invention is not limited in this regard.

For example, in some embodiments, normal blood plasma cortisol levels are between 70nmol/l - 700nmol/l (between 2.5 μg/dL - 25 μg/dL), or between 70mol/l - 350mol/l (between 2.5 μg/dL - 12.5 μg/dL), depending on the parameters of the assay. Methods for measuring cortisol levels, e.g., in the blood or urine of a subject, and normal ranges in addition to the ranges provided herein, are known to those of skill in the art and the invention is not limited in this respect. In some embodiments, a level above the normal range of cortisol levels, for example, a blood plasma cortisol level of more than 350nmol/l, 400nmol/l, 500nmol/l, 600nmol/l, 700nmol/l (e.g., a level of more than 750nmol/l, more than 800nmol/l, more than 900nmol/l, more than 1μmol/l, more than 2μmol/l, more than 2.5μmol/l, more than 5μmol/l, more than 10μmol/l, more than 20μmol/l, more than 25μmol/l, more than 50μmol/l, more than 100μmol/l, or more than 500μmol/l) is an elevated cortisol level. In some embodiments, a compound described herein, for example, carbenoxolone or an analog or salt thereof, is administered to the subject in an amount effective to reduce an elevated glucocorticoid level, for example, an elevated cortisol level, in the subject. In some embodiments, the 18β-Glycyrrhetinic acid or an analog thereof, for example, carbenoxolone, is administered to the subject in an amount effective to reduce an elevated glucocorticoid level, for example, an elevated cortisol level, in the subject to a level that is less than 90%, less than 80%, less than 75%, less than 70%, less than 60%, less than 50%, less than 40%, less than 30%, less than 25%, less than 20%, less than 10%, less than 5%, less than 2.5%, less than 1%, or less than 0.1 % of the level exhibited by the subject prior to administration of the 18β-Glycyrrhetinic acid or an analog thereof. In some embodiments, the 18β-Glycyrrhetinic acid or an analog thereof, for example, carbenoxolone, is administered to the subject in an amount effective to reduce an elevated glucocorticoid level, for example, an elevated cortisol level, in the subject to a non-pathogenic level, or a level not deemed to be elevated, or a level expected to be present in a healthy subject. For example, in some embodiments, carbenoxolone is administered to a subject carrying a polyQ tract expansion mutation of the Huntingtin gene, and exhibiting an elevated cortisol level (e.g., a blood plasma level of more than 350nmol/l or more than 700nmol/l), but not exhibiting a clinical symptom of HD, in an amount effective to reduce the cortisol level to a normal level (e.g., to a blood plasma level within the range of 70-350nnmol/l or 70-700nmol/l). In some embodiments, the cortisol level is monitored in the subject after administration of carbenoxolone, and the dosage is adjusted, e.g., increased if the cortisol level is determined to still be elevated, or decreased if the cortisol level is lower than desired (e.g., lower than 70nmol/l). In some embodiments, the lowest dose of carbenoxolone required to maintain a normal cortisol level (e.g., a blood plasma cortisol

level of 70nmol/l - 700nmol/l) is determined by repeated administration of carbenoxolone to the subject and monitoring of the cortisol level. In some embodiments, the lowest dose required to maintain a normal cortisol level is used for long-term administration in the subject.

In some embodiments, carbenoxolone or a carbenoxolone analog or derivative is administered to a subject having or suspected of having a polyQ tract expansion disease or disorder, or carrying a polyQ tract expansion mutation, or expressing a polyQ tract-expanded polypeptide, in combination with one or more additional drug In some embodiments, the one or more additional drug is a compound described herein, for example, camptothecin, 10-hydroxycamptothecin, topotecan, irinotecan, 18β-Glycyrrhetinic acid, Etoposide, Ouabain, Proscillaridin A, and/or Ethacrynic acid, or a pharmaceutically acceptable analog, salt, or solvate of any of these compounds. In some embodiments, the one or more additional drug is a compound identified in Formula 1. In some embodiments, the one or more additional drug is a drug that ameliorates an undesired side-effect of carbenoxolone or the analog, salt, or solvate thereof that is administered. For example, in some embodiments, the one or more additional drug is a drug that ameliorates hypertension, hypoalkaemia, and electrolyte retention (e.g., sodium retention). Non-limiting examples of such drugs are antihypertensive drugs, potassium supplements, and diuretics. Antihypertensive drugs, potassium supplements, and diuretics as well as effective amounts and suitable administration routes of such drugs are well known to those of skill in the art and the invention is not limited in this respect. For example, in some embodiments, a combination of carbenoxolone and an antihypertensive drug are administered to a subject having or suspected of having a polyQ tract expansion disease. In some embodiments, a combination of carbenoxolone and a potassium salt are administered to a subject having or suspected of having a polyQ tract expansion disease. In some embodiments, a combination of carbenoxolone and a diuretic drug are administered to a subject having or suspected of having a polyQ tract expansion disease.

Some aspects of this invention provide a method for treating a polyQ tract expansion disease or disorder, comprising administering to a subject having or suspected of having a polyQ tract expansion disorder or disease an lkb1 inhibitor, Topoisomerase 1 inhibitor, Topoisomerase 2 inhibitor, Topoisomerase 3 inhibitor, Topoisomerase 3α inhibitor, Na+/K+ ATPase inhibitor, or GST inhibitor. Exemplary inhibitors of these types are provided herein and additional inhibitors are well known to those of skill in the art and include, for example, RNAi agents (e.g. siRNA, shRNA, antisense RNA), small molecule compounds, antibodies, or antigen-binding fragments thereof, aptamers, and adnectins.

In some embodiments, the method comprises administering a single compound provided herein, for example, a single compound provided in Table 3, Table 4, or Table 5. In some embodiments, the method comprises administering a combination of compounds as provided herein, or a combination of one or more compounds as provided herein with a compound known in the art to be useful in the treatment of a polyQ tract expansion disease or disorder.

In some embodiments, the polyQ tract expansion disease or disorder is Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12). In some embodiments, the method of treating comprises administering a compound as provided herein to a subject diagnosed with any of the aforementioned diseases. In some embodiments, the method of treating comprises administering a compound as described herein to a subject based on the subject being diagnosed with the disease or based on the subject being suspected to have the disease.

In some embodiments, the polyQ tract expansion disease or disorder is causally related to a polyQ tract expansion mutation in the ATN1, DRPLA, HTT, Androgen receptor on the X chromosome, ATXN1, ATXN2, ATXN3, ATXN12, CACNA1A, ATXN7, TBP, PPP2R2B, or SCA12 gene. In some embodiments, the method comprises administering a compound provided herein to a subject based on the subject being diagnosed with having a polyQ tract expansion mutation, for example, a polyQ tract expansion mutation in any of the aforementioned genes.

In some embodiments, the subject expresses an ATN1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the subject expresses an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the method comprises administering a compound provided herein to a subject based on the subject expressing any of the aforementioned polyQ tract expanded proteins.

Some aspects of this invention provide methods for treating a subject In some embodiments, the subject is human. In some embodiments, the subject is a non-human mammal, for example, a non-human primate, a mouse, a rat, a pig, a dog, or a cat. In some embodiments, the subject is a non-mammal, for example, an insect, or a fish, an amphibian, or a reptile.

Some aspects of this invention also provide methods for preparing a medicament or a formulation for the treatment of a polyQ tract expansion disorder. In some embodiments, a compound or composition described herein is formulated for administration to a subject in need of such treatment.

Some aspects of the invention relate to methods of treating polyQ tract expansion diseases or disorders, or treating a subject carrying a polyQ tract expansion mutation, or expressing a polyQ tract-expanded polypeptide prior to the manifestation of clinical symptoms of a polyQ tract disease or disorder associated with the mutation or polypeptide. In some embodiments, a compound or composition as provided herein is administered to a subject having or suspected of having a polyQ tract expansion disease or disorder. In some embodiments, the compounds or compositions as provided herein are administered in an "effective amount". An "effective amount" in the context of treatment of a polyQ tract expansion disease or disorder is an amount of a compound or composition as described herein that alone, or together with further doses, produces a desired response, e.g. modulation of polyQ tract polypeptide aggregation, cell morphology, and/or amelioration of any functional symptoms associated with the polyQ tract expansion disease or disorder. For example, desired responses to treatment in the context of Huntington's disease include, but are not limited to, a reduction in the aggregation of Htt protein, reduction in the number or size of inclusion bodies, a normalization of brain tissue homeostasis (e.g. improved survival of neuronal cells and/or reduction in astrocytes), an improvement in cognitive and motor function, and/or is slowing or reversal of the personality change commonly associated with HD.

In some embodiments, in the case of treating a particular disease or condition described herein the desired response is inhibiting the progression of the disease or condition. In some embodiments, this involves only slowing the progression of the disease temporarily, although more preferably, it involves halting the progression of the disease permanently. In some embodiments, the response of the subject to the administration of a compound provided herein is monitored by routine diagnostic methods known to one of ordinary skill in the art for the particular disease. In some embodiments, the desired response to treatment of the disease or condition is delaying the onset or even preventing the onset of the disease or condition, or reversing the physiological effects of the disease.

The effective amount will depend on the particular condition being treated, the severity of the condition, the individual patient parameters including age, physical condition, size and weight, the duration of the treatment, the nature of concurrent therapy (if any), the formulation of the compound, the specific route of administration and like factors within the knowledge and expertise of the health practitioner. These factors are well known to those of ordinary skill in the art and can be addressed with no more than routine experimentation. It is generally preferred that a maximum dose of the agent that modulates a polyQ tract expansion disease or disorder (alone or in combination with other therapeutic agents) be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of one or more compounds or compositions as described herein for producing the desired response in a unit of weight or volume suitable for administration to a patient.

The doses of compounds or compositions administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

Various modes of administration will be known to one of ordinary skill in the art which effectively deliver the compounds or compositions to a desired tissue, cell or bodily fluid. Administration includes: topical, intravenous, oral, intracavity, intrathecal, intrasynovial, buccal, sublingual, intranasal, transdermal, intravitreal, subcutaneous, intramuscular and intradermal administration. The invention is not limited by the particular modes of administration disclosed herein. Standard references in the art (e.g., Remington's Pharmaceutical Sciences, 18th edition, 1990) provide modes of administration and formulations for delivery of various pharmaceutical preparations and formulations in pharmaceutical carriers. Other protocols which are useful for the administration of compounds or compositions will be known to one of ordinary skill in the art, in which the dose amount, schedule of administration, sites of administration, mode of administration (e.g., intra-organ) and the like vary from those presented herein.

Administration to mammals other than humans of compounds or compositions, e.g. for testing purposes or veterinary therapeutic purposes, is carried out under substantially the same conditions as described above. It will be understood by one of ordinary skill in the art that this invention is applicable to both human and animal diseases that can be treated by the compounds or compositions as described herein. Thus this invention is intended to be used in husbandry and veterinary medicine as well as in human therapeutics.

In general, a therapeutically effective amount of a compound or composition provided herein typically varies from about 0.01 ng/kg to about 1000 μg/kg, preferably from about 0.1 ng/kg to about 200 μg/kg and most preferably from about 0.2 ng/kg to about 20 μg/kg, in one or more dose administrations daily, for one or more days. Lesser or greater amounts may be found to be therapeutically effective and thus also are useful in accordance with the invention.

The pharmaceutical preparations of the invention may be administered alone or in conjunction with standard treatment(s) of the disorders described herein, e.g., polyQ tract expansion diseases or disorders such as Huntington's disease.

Pharmaceutical preparations of the invention are administered in effective amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

The compounds or compositions described herein may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the compounds or compositions, and with each other, in a manner such that there is no interaction which would substantially impair the desired pharmaceutical efficacy.

The pharmaceutical compositions may contain suitable buffering agents, as described above, including: acetate, phosphate, citrate, glycine, borate, carbonate, bicarbonate, hydroxide (and other bases) and pharmaceutically acceptable salts of the foregoing compounds.

The pharmaceutical compositions also may contain, optionally, suitable preservatives, such as: benzalkonium chloride; chlorobutanol; parabens; and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions suitable for parenteral administration may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulation suitable for oral, subcutaneous, intravenous, intramuscular, etc. administrations can be found in Remington's Pharmaceutical Sciences, Mack Publishing Co., Easton, PA.

A long-term sustained release implant also may be used for administration of the pharmaceutical agent composition. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above. Such implants can be particularly useful in treating conditions by placing the implant near portions of a subject affected by such activity, thereby effecting localized, high doses of the compounds of the invention.

### *Methods, compositions, and reagents for identifying an agent for the treatment of a polyQ tract expansion disease*

In some aspects, the invention provides methods that are useful for identifying compounds and compositions for use in treating polyQ tract expansion diseases or disorders. In some aspects, the invention provides methods that

are useful for identifying lead compounds and testing modified compounds that are useful in treating polyQ tract expansion diseases or disorders. In some aspects, and invention provides methods that are useful for identifying molecular targets, for example, druggable members of molecular pathways involved in the pathogenesis of polyQ tract expansion diseases or disorders.

In some embodiments, candidate agents, compounds and compositions are derived from combinatorial libraries, for example, from combinatorial peptide libraries, small molecule libraries, or natural product libraries. Candidate agents and compositions may encompass numerous chemical classes. In some embodiments, candidate compounds are small organic compounds. In some embodiments, candidate agents are small organic compounds, e.g., organic compounds having a molecular weight of more than 50 yet less than about 2500 Daltons. In some embodiments, at least some candidate agents comprise functional chemical groups necessary for structural interactions with polypeptides (e.g., kinase sites), for example, an amine, carbonyl, hydroxyl or carboxyl group. In some embodiments, at least some candidate agents comprise at least two, three, four, or more functional chemical groups. In some embodiments, at least some candidate agents comprise cyclic carbon or heterocyclic structure and/or aromatic or polyaromatic structures substituted with one or more of the above-identified functional groups. In some embodiments, candidate agents can be biomolecules such as peptides, saccharides, fatty acids, sterols, isoprenoids, purines, pyrimidines, derivatives or structural analogs of the above, or combinations thereof and the like. In some embodiments, a candidate agent is a nucleic acid (e.g., a siRNA, shRNA, microRNA, ribozyme, DNAzyme, or aptamer). In some embodiments, a candidate agent is a DNA or RNA molecule, a hybrid molecule, or a nucleic acid molecule comprising modified nucleotides, and/or non-naturally occurring bonds or subunits.

Candidate agents can be obtained from a wide variety of sources including libraries of synthetic or natural compounds. For example, numerous methods are available and known to one of ordinary skill in the art for random and directed synthesis of a wide variety of organic compounds and biomolecules, including expression of randomized oligonucleotides, random or non-random peptide libraries, synthetic organic combinatorial libraries, phage display libraries of random peptides, and the like. Alternatively, libraries of natural compounds in the form of bacterial, fungal, plant and animal extracts are available or readily produced. Additionally, natural and synthetically produced libraries and compounds can be readily be modified through conventional chemical, physical, and biochemical means. Further, known pharmacological agents may be subjected to directed or random chemical modifications such as acylation, alkylation, esterification, amidification, etc. to produce structural analogs of the agents.

*Screening methods*

Some aspects of this invention relate to screening methods for the identification of therapeutic agents or therapeutic targets in polyQ tract expansion diseases. In some embodiments, a screening method is provided that comprises contacting a cell expressing a polyQ tract expansion disease associated protein, or a fragment thereof that includes the polyQ tract, wherein the polyQ tract is a polyQ tract of pathological length, with a candidate agent, for example, a chemical compound, a nucleic acid, or a polypeptide. In some embodiments, the screening methods further comprises monitoring a phenotype of the cell that is associated with a polyQ tract expansion disease, for example, aggregation of the polyQ tract expansion disease associated protein, or fragment thereof, cell morphology, or cell physiology. In some embodiments, the screening method includes monitoring cell morphology and aggregation of the polyQ tract expansion disease associated protein. In some embodiments, cell morphology is monitored by microscopy. In some embodiments, a cell used in the screening method expresses a detectable marker that facilitates morphology determination, for example, a membrane associated fluorescent protein, for example membrane associated GFP. In some embodiments, a cell used in the screening method expresses a polyQ tract expansion disease associated protein, or fragment thereof that includes the polyQ tract, fused to a fluorescent protein, for example a monomeric red fluorescent protein. In some embodiments, a cell used in the screening method expresses both a detectable marker facilitating morphology determination and a polyQ tract expansion disease associated protein, or fragment thereof, fused to a detectable moiety, for example a fluorescent protein. In some embodiments, both the detectable marker facilitating morphology determination and the detectable moiety fused to the polyQ tract expansion disease associated protein are fluorescent proteins. In some such embodiments, the fluorescent proteins are chosen so that they can be a distinctly identified, for example by fluorescent microscopy, when coexpressed in the cell. For example, fluorescent proteins with distinguishable emission spectra may be employed, and those of skill in the art will be able to identify fluorescent proteins in the expression spectrum of which are sufficiently distinct.

Fluorescent proteins are well known to those of skill in the art. Exemplary fluorescent proteins useful in the methods provided herein include GFP, RFP, YFP, BFP, CFP, enhanced versions of fluorescent proteins (e.g., eGFP, eRFP, eCFP, etc.), destabilized versions of fluorescent proteins (e.g., dsREd), and other variations (Tomato, mCherry, etc.). Those of skill in the art will be able to ascertain additional fluorescent proteins and the invention is not limited in this respect.

The invention provides various methods for identifying compounds or compositions that are useful as pharmacological agents for the treatment of polyQ tract expansion diseases or disorders. The methods provided by the invention also are useful for identifying compounds or compositions that modulate aggregation of polyQ tract polypeptides, particularly of polyQ tract expanded polypeptides.

Some aspects of this invention provide a method for identifying an agent for the treatment of a polyQ tract expansion disease. In some embodiments, the method comprises a step of (a) contacting a cell expressing a polyQ tract expanded polypeptide fused to a detectable agent with a candidate agent. In some embodiments, the method comprises a step of (b) determining expression of the polyQ tract expanded polypeptide and/or cellular morphology of the cell contacted with the candidate agent. In some embodiments, the method comprises a step of (c) determining expression of the polyQ tract expanded polypeptide and/or cellular morphology representative of a cell expressing the polyQ tract expanded polypeptide, but not contacted with the candidate agent. In some embodiments, the method comprises a step of (d) comparing the expression and/or the cellular morphology determined in (b) and (c) to a reference or control expression and morphology representative of a cell not expressing the polyQ tract expanded polypeptide. In some embodiments, if the expression and the cellular morphology determined in (b) is more similar to the reference or control expression and morphology than the expression and the cellular morphology determined in (c), then the candidate agent is identified to be an agent for the treatment of a polyQ tract expansion disease. In some embodiments if the expression and the cellular morphology determined in (b) is not more similar to the reference or control expression and morphology than the expression and the cellular morphology determined in (c), then the candidate agent is identified to not be an agent for the treatment of a polyQ tract expansion disease.

In some embodiments, the polyQ tract expanded polypeptide is a polyQ tract expanded polypeptide implicated in Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12), or a fragment of such a peptide. In some embodiments, the polyQ tract expanded polypeptide is a gene product of the ATN1, DRPLA, HTT, Androgen receptor on the X chromosome, ATXN1, ATXN2, ATXN3, CACNA1A, ATXN7, TBP, PPP2R2B, or SCA12 gene, or a fragment of such a gene product.

In some embodiments, the cell is a neuronal or glial cell. In some embodiments, determining the expression of a polyQ tract expanded polypeptide is determining the level of aggregation of the polyQ tract expanded polypeptide. In some embodiments, the cell not expressing the polyQ tract expanded polypeptide is a cell expressing a non-pathogenic version of the polyQ tract expanded polypeptide.

In some embodiments, determining expression of the polyQ tract expanded polypeptide comprises quantifying a level of expression, cellular distribution, subcellular localization, aggregation, absence or presence in a cell organelle, and/or cellular turnover of the polypeptide. In some embodiments, determining cellular morphology comprises quantifying cell volume; cell shape; cell size; area covered by a cell; cell context in a tissue; number, size, structure, morphology, and/or quality of cell-cell contacts or cell-cell connections; size, shape, volume, structure, and/or morphology of a cell organelle. In some embodiments, for example, in some embodiments, where the cell is a neuronal or a glial cell, determining cellular morphology comprises quantifying axonal outgrowth, axon size, axon length, axonal connections, branching, blebbing, fasciculation, polypeptide aggregation, neuromere number, neuromere size, connection number, connection strength, projection length, branch point number, branch point distribution, or tissue organization. In some embodiments, determining is by cell imaging. In some embodiments, the cell imaging is live-cell fluorescence imaging. In some embodiments, the live-cell fluorescence imaging is performed by automated microscopy.

Some aspects of this invention relate to reagents useful in screening methods for the identification of therapeutic agents or therapeutic targets in polyQ tract expansion diseases. In some embodiments, a nucleic acid is provided which comprises the coding sequence of a gene associated with a polyQ tract expansion disease, or a fragment thereof that includes the sequence encoding the polyQ tract, and a nucleotide sequence encoding a detectable polypeptide. In some embodiments, the nucleic acid is a part of a nucleic acid construct, for example, an expression construct. In some embodiments, the gene associated with a polyQ tract expansion disease is a gene chosen from the group of ATN1 or DRPLA, HTT, Androgen receptor on the X chromosome, ATXN1, ATXN2, ATXN3, CACNA1A, ATXN7, TBP, or PPP2R2B or SCA12. In some embodiments, the nucleic acid encodes a fusion protein of a protein associated with a polyQ tract expansion disease, or a fragment thereof that includes the polyQ tract, and a detectable polypeptide. In some embodiments, the nucleic acid comprises a sequence encoding a polyQ tract of normal length for the specific protein or fragment thereof, for example of a normal length according to the ranges given in Table 1. In some embodiments, the nucleic acid comprises a sequence encoding a polyQ tract that is longer than the normal length for the specific protein or fragment thereof, for example of a normal length according to the ranges given in Table 1. In some embodiments, the nucleic acid comprises a sequence encoding a polyQ tract of pathologic length for the specific protein or fragment thereof, for example of a pathologic length according to the ranges given in Table 1.

*Fusion proteins and encoding nucleic acids*

For example, in some embodiments, a nucleic acid is provided, which comprises the coding sequence of the Htt gene, or a fragment thereof that includes the polyQ tract, and a sequence encoding a fluorescent protein as the detectable moiety. In some embodiments, a nucleic acid is provided, which encodes a fusion protein of the HTT protein, or the fragment thereof that includes the polyQ tract, and the fluorescent protein. In some embodiments, the nucleic acid encodes a polyQ tract of normal length, for example, a polyQ tract comprising 15 Q residues (HttQ15). In some embodiments, the nucleic acid encodes a polyQ tract of pathologic length, for example, a polyQ tract comprising 138 Q residues (HttQ138). In some embodiments, the fluorescent protein is a monomeric red fluorescent protein.

Some aspects of this invention provide a fusion protein, comprising (a) a polyQ tract expanded protein, or fragment thereof, wherein the fragment comprises the polyQ tract of the protein, and (b) a detectable protein or polypeptide. Some aspects of this invention provide a nucleic acid encoding such a fusion protein.

In some embodiments, the polyQ tract expanded protein is an ATN 1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the polyQ tract expanded protein is an ATN 1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the poly-Q tract is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 90, 91, 92, 93, 94, 95, 96, 97, 98, 99, 100,101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140 residues long.

In some embodiments, the detectable protein or polypeptide is a fluorescent protein or polypeptide. In some embodiments, the fluorescent protein or polypeptide is GFP, eGFP, YFP, RFP, mRFP, mTomato, mCherry, dsRed, or CFP.

*Cells*

Some aspects of this invention relate to cells useful in screening methods for the identification of therapeutic agents or therapeutic targets in polyQ tract expansion diseases. In some embodiments, a cell is provided, which comprises a nucleic acid or a nucleic acid construct as provided by some aspects of this invention. For example, in some embodiments, a transgenic cell is provided that expresses a nucleic acid constructs encoding a protein associated with polyQ tract expansion disease, or a fragment thereof that includes the polyQ tract, and further encoding a detectable polypeptide, for example, as a fusion with the polyQ tract disease-related protein.

In some embodiments, the provided methods utilize cells that are genetically or otherwise modified. In some embodiments, the cells preferably are modified to express, are contacted with, or contain molecules that permit analysis of polyQ tract protein expression, aggregation, and cellular morphology. Such molecules provide contrast with the surrounding environment and facilitate imaging. In some embodiments, the cells express one or more fluorescent proteins, for example, green fluorescent protein, and protein expression, aggregation, and/or cell morphology is readily imaged with fluorescent detection equipment. Fluorescent proteins are well known in the art and include, but are not limited to GFP, YFP, RFP, BFP, enhanced versions of fluorescent proteins (e.g., eGFP, eYFP, etc.), destabilized fluorescent proteins (e.g., dsRed), monomeric fluorescent proteins (e.g., mRFP, mOrange, mCherry, etc.) dimeric fluorescent proteins (dTomato, etc.). In some embodiments, the provided methods utilize cells that express two or more fluorescent proteins, for example, one as a marker facilitating the determination of cell morphology and another for the determination of polyQ tract protein expression or aggregation. Those of skill in the art will be able to readily select suitable fluorescent proteins and combinations of fluorescent proteins other than the ones disclosed herein for appropriate excitation and emission characteristics.

Some aspects of this invention provide a modified cell, comprising (a) a nucleic acid construct comprising a nucleic acid sequence encoding a polyQ tract expanded protein fused to a fluorescent protein under the control of a

promoter; and (b) a detectable marker allowing for visualization of cell morphology. In some embodiments, the detectable marker allowing for visualization of cell morphology is a fluorescent protein. In some embodiments, the fluorescent protein is membrane-binding fluorescent protein. In some embodiments, the fluorescent protein is GFP, eGFP, YFP, RFP, mRFP, or CFP. In some embodiments, the detectable marker is a dye. In some embodiments, the dye is a vital dye. In some embodiments, the vital dye is 5-carboxy-fluorescein diacetate AM. In some embodiments, the detectable marker is a detectably labeled antibody that binds to the surface of the cell. In some embodiments, the detectably labeled antibody is an antibody conjugated to a Cy dye.

In some embodiments, the polyQ tract expanded protein is an ATN1 or DRPLA protein comprising a polyQ tract of more than 35 Q residues, an HTT (Huntingtin) protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues. In some embodiments, the polyQ tract expanded protein is an ATN1 or DRPLA protein comprising a polyQ tract of 49-88 Q residues, a HTT (Huntingtin) protein comprising a polyQ tract of 35-140 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63, or a PPP2R2B or SCA12 protein comprising a polyQ tract of 66-78 Q residues. In some embodiments, the poly-Q tract is 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 90, 91, 92, 93, 94, 95, 96, 97, 98, 99, 100,101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, or 140 residues long.

In some embodiments, cells are treated with exogenously added molecules, for example, with a dye that facilitates cellular imaging. In some embodiments, cells are contacted with a dye that binds the cell membrane and provides contrast for imaging fine morphology, for example, neurite outgrowth or axonal fine structure of neuronal cells. In some embodiments, a dye is added to cell media for binding cell bodies prior to imaging. In some embodiments, the cell media may be substituted with media that does not contain the dye subsequent to staining but prior to imaging, to increase contrast between the background and the cells. Dyes suitable for cell staining are well known to those of skill in the art, and include, but are not limited to, dyes for living cells (e.g., vital dyes), such as 5-carboxy-fluorescein diacetate AM (Molecular Probes, Eugene, OR). In some embodiments, cells are labeled with antibodies that bind a cellular surface antigen and are detectably labeled to permit visualization. In some embodiments, antibodies are fluorescently labeled, e.g., by conjugation to a Cy dye (e.g. Cy3).

In some embodiments, the cells utilized in the assays described herein are cells that express a polyQ tract expanded polypeptide. In some embodiments, the cells do not endogenously express a polyQ tract expanded polypeptide, and the cells are modified to express the polyQ tract expanded polypeptide. Methods for modifying cells to express exogenous polypeptides are well known to those of skill in the art. For example, as described in more detail elsewhere herein, cells can be transfected or transduced with an expression vector that directs the expression of the polyQ tract expanded polypeptide.

A variety of cells are useful in the methods and assays of the invention. For example, suitable cells include, but are not limited to, neuronal and glial cells.

In some embodiments, methods are provided that include contacting a cell expressing a polyQ tract expanded polypeptide with a candidate agent, and comparing the cellular response to the candidate agent to an appropriate negative control. Appropriate negative controls are typically cells or samples of the same type and treated under the same conditions, but not contacted with the specific, or any, candidate agent. In some embodiments, control assays are performed by substituting the vehicle (e.g., water, or DMSO) for the candidate agent. In some embodiments, control assays are performed by substituting a control agent, (e.g., a scrambled nucleic acid or amino acid sequence, or a compound with known effect) for the candidate agent (e.g., a specific siRNA, polypeptide, or compound). In some embodiments, a plurality of cell populations are contacted in parallel with different candidate agents, and/or concentrations of candidate agents. In some embodiments, one of these concentrations serves as a negative control, for example, at zero concentration of candidate agent or at a concentration of agent below the limits of assay detection.

Various methods for determining cell morphology and/or expression of polyQ tract expanded protein or polypeptide are known in the art. In preferred embodiments, cell morphology and/or polyQ tract expanded polypeptide expression, for example, polyQ tract expanded polypeptide aggregation, is determined by cell imaging, for example, live-cell fluorescence imaging. In some embodiments, cells are grown in multiwell plates such as 96-well or 384-well plates, and

imaging is performed using an automated microscope. In some embodiments, pixel maps are generated by an analysis software (e.g., MetaXpress™). Some embodiments, cell bodies are identified as pixel blocks, for example, pixel blocks off a specified area range, for example, with an area smaller than 120$\mu m^2$ but greater than 25$\mu m^2$.

The invention also provides cultures and cell populations of the cells and cell lines described herein.

The function and advantage of these and other embodiments of the present invention will be more fully understood from the examples below. The following examples are intended to illustrate the benefits of the present invention, but do not exemplify the full scope of the invention.

EXAMPLES

**Materials and Methods**

**Primary Cell Culture.** *Elav$^{c155}$-GAL4* virgins were collected en-masse and crossed to either *UAS-Htt-Q138-mRFP$^1$, UAS-mCD8-GFP* or *UAS-HttQ15-mRFP$^1$, UAS-mCD8-GFP.* males to generate embryos for primary culture preparation. Neuroblasts were isolated as described (Sepp et al., 2008).

**Western Blotting.** Embryonic lysates (n = 4/genotype) were prepared from control and Htt expressing strains (50 mM Tris, pH 8.0, 150 mM NaCl, 0.1% SDS, 1.0 % NP-40 (IgePal), 0.1% sodium deoxycholate plus protease inhibitors (cOmplete-mini, Roche)), and protein content was quantified using a BCA kit (Pierce). Protein samples (10 $\mu$g/lane) were analyzed using standard SDS-PAGE/Western blotting techniques, and quantified using an Odyssey Infrared Imaging System (Li-Cor). For immunoblotting, antibodies were used at the following concentrations: mouse anti-Tubulin (6-11B-1, Sigma-Aldrich T7451) at 1/60,000, mouse anti-human Htt (MAb 2166, Chemicon) at 1/1,000, and goat-anti-mouse IR800 secondary (LI-COR 926-32210) at 1/3,000.

**Compound screening:** Primary cultures were re-suspended directly in Shields and Sang M3 media (Sigma) supplemented with 10 U/mL penicillin, 10 $\mu$g/mL streptomycin, 200 ng/mL insulin, and 5% fetal bovine serum. 100 nL of compounds from arrayed small-molecule libraries (NINDS Custom Collection 2, Prestwick1 Collection, BIOMOL2 ICCB-Longwood Known Bioactives High Concentration, various concentration from 1-15 mM in DMSO) were applied to 50 $\mu$L of cultures 24 hours after plating on optical bottom 384-well plates (Corning 3712). The neuroblast density was 18,500 cells/well. The primary screen was carried out in duplicate and hits were validated with 12 additional replicate wells.

**RNAi Screening:** dsRNAs (250 ng/well) were aliquoted onto microscopy plates and then 10 $\mu$L of neuroblasts were applied to a density of 18,500 cells/well. Cultures were incubated for 3 days with dsRNAs to achieve gene knockdown. Shields and Sang M3 media (Sigma) supplemented with 10 U/mL penicillin, 10 $\mu$g/mL streptomycin, 200 ng/mL insulin, and 5% fetal bovine serum was then added to cultures to bring assay volume to 50 $\mu$L. The *Drosophila* RNAi Screening Center (DRSC) whole genome kinase/phosphatase library (468 genes, 3 amplicons/gene) was screened in duplicate, and hits were validated using additional dsRNA amplicons containing no off-targets. For RNAi validation studies, dsRNAs were synthesized from T7-tailed DNA templates using the MEGAshortscript T7 transcription kit (Ambion). Synthesized dsRNAs were purified with RNeasy kits (Qiagen) before use in cell culture experiments. The T7-tailed oligonucleotides used to generate DNA templates from $w^{1118}$ genomic DNA are as follows : Lkb-1: DRSC16481 (GCCGTCAAGATCCTGACTA/CTCCGCTGGACCAGATG, SEQ ID NO: 1 and 2), DRSC36925 (GCAACTCCACGGTGATACCT/ATGCAGGACGTCAGCTTCTT, SEQ ID NO: 3 and 4), DRSC36926 (ATTGCGGCGAACTTACTTTG/TAATCCTCACCAGGCACACA, SEQ ID NO: 5 and 6); Top-1: DRSC36056 (GAGAATGTGCAGGGACAGGT/GTCGATGAAGTAAAGGGCCA, SEQ ID NO: 7 and 8), DRSC20295 (GGAGGAGGAGAAGCGTG/GCGCCGCTTGATCATG, SEQ ID NO: 9 and 10); Top2: DRSC36057 (CACAGCGACAGAAGCATCAT/TTCTTGTATTCCCTCGTGGC, SEQ ID NO: 11 and 12), DRSC3459 (TTTGCCAGAGCGATATCTC/CCATAGTGGCTCGATCTTTT, SEQ ID NO: 13 and 14); Top3$\alpha$: DRSC3460 (TTAAACGTGGCTGAGAAGAA/GCCCACGCCCTTTTTCA, SEQ ID NO: 15 and 16), DRSC37672(GTGGTCCTGACCGAACAGAT/AGGTTTTGTACCAACCGCTG, SEQ ID NO: 17 and 18); Top3$\beta$: DRSC18724(GCGGACTTCGGTGAGGA/CGCTGGCAGATGTTGTTG, SEQ ID NO: 19 and 20).

**Microscopy:** For high-content screening mature 7-day old cultures were imaged with an ImageXpress$^{MICRO}$ robotic microscope (Molecular Devices, Sunnyvale, CA) using a 10X objective and FITC/Cy3 filter sets. Images were 1392 x 1040 pixels, or 897 x 670 micrometers. Laser-based autofocusing was used to locate plate bottoms, and then image-based focusing used to resolve fluorescently labeled neurons over a 48 $\mu$m range. The GFP and mRFP channels were imaged in the same focal plane, with exposure times of 850 and 400 ms respectively. Three sites were imaged per well for each treatment group, and the screen was done in duplicate. For confocal microscopy of primary cultures, neuroblasts were plated on poly-L-lysine coated chambered cover slips (LabTekII, 0.8$cm^2$/well) at 18,300 cells/well in 50 $\mu$L volume. Small molecules were added to cultures 24 hours after plating, incubated for 7 days, and then imaged with a Leica TCS-SP2 confocal LSM microscope.

**Digital Image Analysis of High Content Screening Data Sets:** Neuronal morphological analysis and Htt aggregate quantification for automated microscopy images was performed as previously described (Schulte et al., Wu et al.). In brief, Htt-Q138 aggregates were quantified as the total number of pixels/image with an intensity higher than

an empirically set threshold. Statistical analysis was conducted using a two sample *t*-test. To quantify neuronal morphologies, cell body clusters (neuromeres) and neurites were extracted from images using our custom algorithms (Wu et al.). The $log_2$ transformed areas of cell body clusters were found to fit a Gaussian mixture model (GMM) and therefore were separated into three bins (small, medium, and large). Absolute counts of neuromeres/bin were tabulated for all images. Neurite segment lengths were similarly clustered into three groups (short, medium, and long) using the *K*-means method and then quantified. Cell cluster and neurite counts were converted into percentages to control for any variation in cell number between wells arising from pipetting error. Mean neuromere area and neurite length for each image were also calculated to give a total of eight morphological metrics for image morphology quantification. For statistical analysis, *p*-values for each morphological feature were calculated using a two sample *t*-test (e.g. small neuromere feature of Htt-Q138 drug-treated cultures *versus* small neuromere feature of Htt-Q15 DMSO cultures). The resultant morphological *p*-values for individual features were then integrated into a single *p*-value using the Fisher method (Fisher 1932) defined by the equation $\chi^2 = -2\sum_{i=1}^{k} log_e(p_i)$, where *k* represents independent tests, $p_i$ is the *p*-value of the *i*-th feature. The combined statistic has a $\chi^2$ distribution with 2*k* degrees of freedom under the joint null hypothesis. This method works well in cases where the evidence against the null-hypothesis is spread across different features. Excluded from analysis were compound-treated wells with < 6 images, out-of-focus images, or images that lacked cell profiles altogether.

***In vivo* Rescue studies:** For small molecule *in vivo* rescue studies, $Elav^{c155}$; *UAS-Htt-Q138-mRFP$^1$,UAS-mCD8-GFP*/+ 1$^{st}$ instar larvae were collected en-masse and dispensed into liquid yeast media (10 larvae/well) containing therapeutic agents at three different concentrations as described in the results (n=8 replicas/concentration). Cultures were reared at 21°C and larval viability was assessed after 5 days and the mean number of living Htt138Q larvae (i.e. $GFP^+$, $Htt138QmRFP^+$, and mobile) was tabulated, and expressed as a percentage of total larvae/well. For genetic rescue studies with lkb1, two Htt138Q strains were utilized: a strong expressing line, $UAS\text{-}Htt138QmRFP^1$ which is pharate lethal when crossed to $Elav^{c155}$, and a weaker expressing line, $UAS\text{-}Htt138QmRFP^2$ which survives to adulthood and is viable for a number of weeks. Using the strong line, pharate lethality at 25°C was calculated after lkbl ($lkb1^{4B1-11}$ and $lkb1^{4A4-2}$ alleles) was introduced into an $Elavc^{155}$,UAS-Htt/+ background. *$Lkb1^{4B1-11}$* is a premature truncation allele (Q98 > Stop), and *$lkb1^{4A4-2}$* is an EMS null allele (589 b.p. deletion removing 150 b.p. of the 5' UTR, the start codon and the beginning of the open reading frame). For Top1 *in vivo* analysis, $Elav^{c155}$,*$Top^{112}$* recombinants were generated and crossed to *$UAS\text{-}Htt138QmRFP^1$*.

**Negative Geotaxis Assay:** $Lkb1^{4A4-2}$ was crossed into the $Elav^{c155}$, $UAS\text{-}Htt138QmRFP^2$ which is adult viable and has weaker Htt138Q expression. Virgin female Drosophila were collected and flipped onto fresh media two times per week until the start of the assay. 25 day-old flies (10-15 flies/vial, 4 vials/genotype) were gently tapped to the base of vials, and climbing behavior was video-recorded for 18 seconds (trial). The percentage of flies to reached the top of a vial was tabulated and averaged after 4 trails. Vials were back-lit with a light box to enhance the resolution of the fly climbing trajectories. Statistical analysis was performed using a t-test.

## Example 1: a transgenic model to study polyQ expansion phenotypes

We have previously described a *Drosophila* model that displays many characteristics of HD, including neurodegeneration, disrupted axonal transport, and decreased longevity (Lee et al., 2004). To extend our studies of HD pathology, we generated a new monomeric Red Fluorescent Protein (mRFP) N-terminal tag variant for in vivo imaging of Htt distribution (Htt-RFP). The Htt-RFP construct encompasses the caspase-6 cleavage fragment important for Htt toxicity (Graham et al., 2006) and includes either a nonpathogenic (Q15) or pathogenic (Q138) poly-Q tract. This fragment corresponds to exons 1-12 of human Htt and is 588 amino acids in length (~80 kDa), excluding the polyQ domain and RFP tag.

For our studies, we used the GAL4/UAS system (Brand AH, Perrimon N (1993)) to drive expression of the constructs in the nervous system using the pan-neuronal GAL4 driver Elavc155 (C155). We selected UAS-Htt15QmRFP and UAS-Htt138QmRFP strains that had comparable expression levels ($Htt15Q^1$ and $Htt138Q^1$) when crossed to C155 as demonstrated by quantitative Western blotting (Figure 1G). Prominent bands of ~109 kDa and ~125 kDa were observed for the $Htt15Q^1$ and $Htt138Q^1$ strains, respectively, in agreement with the predicted molecular weights of the RFP-fusion proteins. Pan-neuronal expression of $Htt138Q^1$ using C155 causes pupal lethality, while $Htt15Q^1$ controls are viable and have normal longevity (see detailed analysis below). For downstream behavioral analysis, we selected an additional UAS-Htt138QmRFP strain ($Htt138Q^2$) that has reduced Htt138Q protein expression (Figure 1G) and is adult viable. The decreased longevity observed in the $Htt138Q^1$ strain is more severe than that observed in our earlier studies (Lee W-CM, Yoshihara M, Littleton JT (2004) Cytoplasmic aggregates trap polyglutamine-containing proteins and block axonal transport in a Drosophila model of Huntington's disease. Proc Natl Acad Sci USA. pp. 3224-3229) and may be related to an increased polyQ length in the new construct (138Q vs. 128Q), a larger Htt N-terminal fragment (588 amino acids vs. 548 amino acids), or differences in Htt expression levels. The severity of the $Htt138Q^1$ allele suggests that this model may correspond to juvenile-onset HD observed in humans (Roos RA (2010) Huntington's disease: a clinical review.

Orphanet J Rare Dis 5: 40.). In juvenile-onset HD, the CAG repeats often exceed 55 and phenotypes develop prior to adulthood.

To conduct a small molecule and RNAi screen to identify suppressors of Huntington toxicity we prepared primary neuronal cultures from control (C155;Htt15QmRFP[1], abbreviated as Htt15Q) and mutant (C155;UAS-Htt138QmRFP[1], abbreviated as Htt138Q) Huntingtin strains that simultaneously expressed membrane associated-GFP (UAS-CD8GFP) in all neurons. This dual labeling approach enabled us to track the subcellular distribution of mRFP-tagged Huntingtin, while simultaneously monitoring the general morphology of cultured neurons (Fig. 1 A-F). Visualization of Htt-RFP localization demonstrated that Htt138Q readily forms aggregates which accumulate in cell bodies and neurites, while Htt15Q is soluble and has a more uniform cytoplasmic distribution (Fig. 1, compare 1B, 1E). In addition, we found that Htt138Q-expressing neurons display morphological indicators of reduced neuronal health (Roos RA (2010) Huntington's disease: a clinical review. Orphanet J Rare Dis 5: 40.; DiFiglia M, Sapp E, Chase K, Davies S, Bates G, et al. (1997) Aggregation of Huntingtin in neuronal intranuclear inclusions and dystrophic neurites in brain. Science. pp. 1990), including smaller neuromeres, increased branching, and reduced axonal connectivity, as monitored by membrane associated-GFP (Fig. 1, compare 1A, 1D). Neurite morphology and Htt aggregation were quantified in cultures plated in 384-well format using custom algorithms developed to process digital images collected via automated microscopy (Wu C, Schulte J, Sepp KJ, Littleton JT, Hong P (2010) Automatic robust neurite detection and morphological analysis of neuronal cell cultures in high-content screening. Neuroinformatics 8: 83-100.). Population analysis of Htt15Q and Htt138Q replicate wells revealed that eight morphology features (small, medium, large, and average neuromere size, and short, medium, long, and average neurite length) provided robust data content to generate effective separation of Htt15Q control from Htt138Q mutant neuronal morphology. Differences in Htt aggregation were also readily detectable between mutant and control cultures using these algorithms. To screen for suppressors of HD toxicity we therefore monitored the presence of Htt aggregates, as well as morphology, to evaluate overall neuron health.

**Example 2: screening assays and identification of compounds**

We performed a dual RNAi and small molecule screen to identify HD toxicity suppressors, and assayed for suppression of Htt-138Q aggregate formation, in addition to reversion of mutant Htt-Q138 morphology back towards Htt-Q15 controls. For RNAi screening, we wanted to identify novel targets for HD therapeutic development, and focused on a kinase/phosphatase RNAi library (468 genes) that would potentially contain targets of high value for chemical inhibition. For small-molecule screening, we tested libraries enriched for FDA-approved drugs, including the NINDS Custom Collection 2, BIOMOL ICCB Known Bioactives Collection and the Prestwick 1 Collection. This allowed screening of ≈2600 approved compounds, potentially facilitating the advancement of screen hits to clinical trials. For compound screening, we verified that addition of 0.2% DMSO to primary cultures does not significantly alter neuronal morphology or Htt-Q138 aggregation characteristics (Table 3).

Table 3: Effect of positive control compounds on Htt138Q aggregate formation and neurite morphology (p-values listed):

| Compound | CAS# | Well Conc. (mM) | Htt Culture | Mutant Htt 138Q aggregation* | Muant Htt138Q morphology rescue** | n = |
|---|---|---|---|---|---|---|
| C2-8 | 300670-16-0 | 2 | 138Q | 0.0072745 | 0 | 6 |
| C2-8 | | 0.4 | 138Q | 0.216406 | 0 | 6 |
| C2-8 | | 0.08 | 138Q | 0.0679971 | 0 | 6 |
| C2-8 | | 0.016 | 138Q | 0.381982 | 0 | 6 |
| C2-8 | | 0.0032 | 138Q | 0.436453 | 0 | 6 |
| GW5074 | 220904-83-6 | 2 | 138Q | 0.000242646 | 0 | 6 |
| GW5074 | | 0.4 | 138Q | 0.414394 | 0 | 6 |
| GW5074 | | 0.08 | 138Q | 0.859258 | 0 | 6 |
| GW5074 | | 0.016 | 138Q | 0.193046 | 0 | 6 |
| GW5074 | | 0.0032 | 138Q | 0.062019 | 2.54E-14 | 6 |
| Juglone | 481-39-0 | 100 | 138Q | 0.00062262 | 0.00000361 | 6 |
| Juglone | | 20 | 138Q | 0.999965 | 0 | 6 |
| Juglone | | 4 | 138Q | 0.999994 | 0 | 6 |
| Juglone | | 0.8 | 138Q | 0.908447 | 0 | 6 |
| Juglone | | 0.16 | 138Q | 0.640557 | 1.26E-11 | 6 |

(continued)

| Compound | CAS# | Well Conc. (mM) | Htt Culture | Mutant Htt 138Q aggregation* | Muant Htt138Q morphology rescue** | n = |
|---|---|---|---|---|---|---|
| Radicicol | 12772-57-5 | 100 | 138Q | 9.17E-66 | 0 | 6 |
| Radicicol | | 20 | 138Q | 0.0000587 | 0 | 6 |
| Radicicol | | 4 | 138Q | 0.671473 | | 6 |
| Radicicol | | 0.8 | 138Q | 0.0313196 | 0 | 6 |
| Radicicol | | 0.16 | 138Q | 0.0116727 | 0 | 6 |
| Rapamycin | 53123-88-9 | 4 | 138Q | 0.00161737 | 0 | 6 |
| Rapamycin | | 0.8 | 138Q | 0.134587 | | 6 |
| Rapamycin | | 0.16 | 138Q | 0.151829 | 0 | 6 |
| Rapamycin | | 0.032 | 138Q | 0.382367 | 0 | 6 |
| Rapamycin | | 0.0064 | 138Q | 0.290667 | 0 | 6 |
| DMSO | 67-68-5 | 0.2% | 138Q | 1 | 0 | 360 |
| DMSO | 67-68-5 | 0.2% | 15Q | 0 | 1 | 144 |

* $p < 0.05$ indicates Htt138Q aggregate formation is inhibited (shaded).
**$p > 0.05$ indicates Htt138Q drug-treated culture have morphology similar to Htt 15Q control cultures

Known suppressors of Htt poly-Q aggregation, including *C2-8, GW5074*, *Juglone, Radicicol,* and *Rapamycin*, were tested for their efficacy in our assay (Chin et al., 2004; Hay et al., 2004; Ravikumar et al., 2004; Wang et al., 2005; Zhang et al., 2005). Although all control compounds reduced Htt-Q138 aggregation, none reverted the morphology profiles of Q138 expressing neurons towards normal (Table 3). Instead, these compounds caused reduced axon outgrowth, neuromere size, and suppressed GFP expression over a wide concentration range, suggesting these compounds have neurotoxic properties. The dual RNAi/compound screen was conducted in duplicate, and all wells were visually scored independently by two investigators to identify agents that either suppressed aggregation, or reverted mutant Htt-138Q neural profiles towards Htt-15Q controls. From the visual-based screens, three novel suppressors of Htt polyQ toxicity were identified: 1 RNAi hit (*lkb1*), and 2 compounds (Camptothecin and 10-Hydroxycamptothecin). Lkb1 is a known tumor suppressor and a negative regulator of the mTOR /Insulin pathway, which has important roles in autophagy and nutrient sensing (Shaw et al., 2004; Inoki et al., 2005), while the Camptothecins function as DNA Topoisomerase 1 (*Top1*) inhibitors (Hertzberg et al., 1990). In addition to visual inspection of the screen plates, automated microscopy was used to record images of the compound-treated plates for subsequent morphometric analysis using custom algorithms. For automated microscopy, three images per well were taken at different sites for each channel (GFP/RFP) to facilitate hit identification and increase statistical power. Htt-Q138 aggregation was first quantified which led to the identification of 62 compounds that significantly suppressed Htt aggregate formation (Fig. 2, Table 4). Subsequently, those wells that had inhibited aggregate formation were re-evaluated to determine if any treatments were able to revert the mutant Htt-138Q morphology profiles towards that of Htt-15Q controls. Of the 62 compounds that were found to inhibit Htt-138Q aggregate formation, 8 compounds were found to improve the Htt-138Q induced morphological defects (Fig. 3, Table 5). Unmasking of the identities of the 8 compounds revealed that 4 compounds were Camptothecins, in agreement with the visual scoring observations. In addition, two Na+/K+ ATPase inhibitors, and a Glutathione-S-Transferase inhibitor were also identified as being capable of suppressing aggregate formation and rescuing the mutant Htt138Q culture morphologies towards the control state (Table 5). We subsequently validated screen hits, focusing on the targets that overlapped in both the visual and morphometric analysis lists. The Lkb-1 target was validated with three independent dsRNAs (amplicons DRSC16481, DRSC36925, DRSC36926). Each amplicon improved Htt-138Q mutant morphology with statistical significance, but did not inhibit aggregate formation (Table 6). The Camptothecin and 10-Hydroxy-camptothecin small molecules are structural analogues, and were found to rescue aggregate formation in addition to partially reverting dystrophic morphology profiles over a range of concentrations (56 μM, 5.6 μM) (Fig. 4). These compounds alter the Htt-Q138 localization within neurons, such that it resembles, or more closely resembles, the distribution of Htt-Q15 in control cultures (compare Fig. 4C, D with E).

Table 4: List of compounds found to inhibit Htt138Q toxicity in Drosophila primary culture system:

| I.D. | Library | ICCB plate | Well | Library Conc | CAS# | Compound* | Function** | Aggregate Log₂ ratio | Morphological p value |
|---|---|---|---|---|---|---|---|---|---|
| 1 | BIOMOL2 | 1792 | P17 | 5mg/mL | G237 | Cerulenin | Fatty acid biosynthesis inhibitor | -3.139483 | 0 |
| 2 | BIOMOL2 | 1792 | B07 | 0.5mg/mL | EI181 | okadaic acid | PP1 PP2A inhibitor | -3.077206 | 0 |
| 3 | NINDS | 1922 | K06 | 10 mM | 22862-76-6 | ANISOMYCIN | antiprotozoal, antifungal, protein synthesis inhibitor | -3.055318 | 0 |
| 4 | NINDS | 1921 | L04 | 10 mM | 66-81-9 | CYCLOHEXIMIDE | protein synthesis inhibitor | -3.0364 | 0 |
| 5 | BIOMOL2 | 1791 | O18 | 5mg/mL | CA-100 | A-23187 | Calcium ionophore | -2.809242 | 0 |
| 6 | BIOMOL2 | 1792 | I13 | 5mg/mL | CA-201 | ionomycin | Calcium ionophore | -2.735893 | 0 |
| 7 | NINDS | 1921 | P06 | 10 mM | 58-27-5 | MENADIONE | prothrombogenic agent | -2.64595 | 0 |
| 8 | Prestwick | 1569 | N21 | 2 mg/ml | 20554-84-1 | Parthenolide | anti-inflammatory | -2.393522 | 0 |
| 9 | NINDS | 1921 | P18 | 10 mM | 622-78-6 | BENZYL ISOTHIOCYANATE | antineoplastic, antibacterial, antifungal | -2.385704 | 0 |
| 10 | NINDS | 1922 | H15 | 10 mM | 123-31-9 | HYDROQUINONE | antioxidant | -2.341888 | 0 |
| 11 | BIOMOL2 | 1792 | J07 | 5mg/mL | EI-156 | staurosporine | kinase inhibitor | -2.313348 | 0 |
| 12 | BIOMOL2 | 1791 | F08 | 5mg/mL | GR300 (Biomol) | actinomycin D | transcription inhibitor | -2.277917 | 0 |
| 13 | BIOMOL2 | 1792 | F03 | 5mg/mL | GR-312 | puromycin | protein synthesis inhibitor | -2.254039 | 0 |
| 14 | BIOMOL2 | 1791 | B06 | 5mg/mL | GR-316 (Biomol) | 10-hydroxycamptothecin | topoisomerase 1 inhibitor, antineoplastic | -2.21028 | 0.00242 |
| 15 | BIOMOL2 | 1791 | B08 | 5mg/mL | GR-308 (Biomol) | beta-lapachone | topoisomerase 1 inhibitor, antineoplastic | -2.193166 | 0 |
| 16 | NINDS | 1923 | E05 | 10 mM | 2752-65-0 | GAMBOGIC ACID | antiinflammatory, cytotoxic, inhibits HeLa cells in vitro; LD50(rat) | -2.173426 | 0 |
| 17 | NINDS | 1921 | I15 | 10 mM | 1404-88-2 | TYROTHRICIN | topical antibacterial (topical) | -2.17232 | 0 |
| 18 | BIOMOL2 | 1791 | N03 | 5mg/mL | PI-122 | Tosyl-Phe-CMK (TPCK) | serine protease inhibitor | -2.166435 | 0 |
| 19 | BIOMOL2 | 1792 | M09 | 5mg/mL | EI-293 | 5-iodotubercidin | ERK-2 inhibitor | -2.139152 | 0 |
| 20 | BIOMOL2 | 1792 | C09 | 5mg/mL | CN-240 | diphenyleneiodonium Cl | flavoprotein inhibitor | -2.113107 | 0 |
| 21 | NINDS | 1922 | B15 | 10 mM | 29767-20-2 | TENIPOSIDE | topoisomerase II inhibitor, antineoplastic | -2.103361 | 0 |
| 22 | BIOMOL2 | 1792 | E11 | 5mg/mL | CM120 | FCCP | mitochondrial uncoupler | -2.014179 | 0 |
| 23 | BIOMOL2 | 1792 | M07 | 5mg/mL | CN-200 | LY-83583 | inhibits NO-activation of guanylate cyclase | -1.879978 | 0 |
| 24 | BIOMOL2 | 1791 | H22 | 5mg/mL | EI-175 (Biomol) | aristolochic acid | phospholipase A2 inhibitor | -1.851602 | 0 |
| 25 | NINDS | 1920 | D07 | 10 mM | 70-30-4 | HEXACHLOROPHENE | antiinfective (topical) | -1.844784 | 0 |
| 26 | Prestwick | 1569 | C06 | 2 mg/ml | 22862-76-6 | Anisomycin | Antibiotic. Activator of p38 and MAP kinases. | -1.83072 | 0 |
| 27 | BIOMOL2 | 1791 | P21 | 5mg/mL | CA-421 | Nigericin | induces intracellular acidification | -1.821227 | 0 |
| 28 | NINDS | 1922 | G11 | 10 mM | 522-51-0, 6707-58-0 [dequalinium] | DEQUALINIUM CHLORIDE | antiinfectant | -1.811869 | 0 |
| 29 | NINDS | 1921 | E07 | 10 mM | 54-64-8 | THIMEROSAL | antiinfective, preservative | -1.798877 | 0 |
| 30 | BIOMOL2 | 1791 | F20 | 5mg/mL | EI-258 (Biomol) | AG-879 | NGF receptor inhibitor | -1.786601 | 0 |
| 31 | BIOMOL2 | 1792 | F21 | 5mg/mL | EI270 | Rottlerin | inhibitor of p38 activated kinases | -1.777457 | 0 |
| 32 | BIOMOL2 | 1792 | M13 | 5mg/mL | G-236 | manumycin A | ras farnesylation inhibitor | -1.755964 | 0 |
| 33 | BIOMOL2 | 1791 | N06 | 5mg/mL | GR301 | camptothecin | topoisomerase 1 inhibitor, antineoplastic | -1.637238 | 0.151249 |
| 34 | BIOMOL2 | 1792 | L21 | 5mg/mL | EI-215 | tyrphostin 9 | PDGF-R tyrosine kinase inhibitor | -1.622908 | 0 |
| 35 | BIOMOL2 | 1792 | E09 | 5mg/mL | GR307 | etoposide | topoisomerase II inhibitor, antineoplastic | -1.568362 | 0 |
| 36 | NINDS | 1920 | M13 | 10 mM | 6004-24-6, 123-03-5 (anhydrous) | CETYLPYRIDINIUM CHLORIDE | antiinfective (topical) | -1.560866 | 0 |
| 37 | BIOMOL2 | 1791 | B10 | 5mg/mL | T-113 (Biomol) | Parthenolide | IkappaB kinase inhibitor | -1.552586 | 0 |
| 38 | BIOMOL2 | 1792 | I03 | 5mg/mL | GR-303 | Hoechst 33342 | DNA minor groove binder | -1.534591 | 0 |
| 39 | NINDS | 1922 | P16 | 10 mM | 35069-70-6 | 2,6-DIMETHOXYQUINONE | antibacterial, induces dermatitis, mutagen | -1.507747 | 0 |
| 40 | Prestwick | 1569 | B22 | 2 mg/ml | 316-42-7 | Emetine dihydrochloride | inhibits RNA, DNA and protein synthesis | -1.468808 | 0 |
| 41 | Prestwick | 1570 | M22 | 2 mg/ml | 66-81-9 | Cycloheximide | Antibiotic. protein synthesis inhibitor | -1.445816 | 0 |
| 42 | Prestwick | 1569 | E18 | 2 mg/ml | 6487-30-5 | Cephaeline dihydrochloride heptahydrate | | -1.410059 | 0 |
| 43 | BIOMOL2 | 1791 | N11 | 5mg/mL | PI-110 | 3,4-dichloroisocoumarin | granzyme B inhibitor | -1.372644 | 0 |
| 44 | NINDS | 1923 | A13 | 10 mM | 34157-83-0 | CELASTROL | antineoplastic, antiinflamatory, NO synthesis inhibitor, chaperone | -1.348443 | 0 |
| 45 | NINDS | 1920 | K12 | 10 mM | 316-42-7, 483-18-1 [emetine] | EMETINE | inhibits RNA, DNA and protein synthesis | -1.288103 | 0 |
| 46 | Prestwick | 1568 | H21 | 2 mg/ml | 2112992 | Camptothecine (S,+) | topoisomerase 1 inhibitor, antineoplastic | -1.22608 | 0.109087 |
| 47 | NINDS | 1920 | M16 | 10 mM | 58-54-8 | ETHACRYNIC ACID | diuretic | -1.226055 | 0 |
| 48 | NINDS | 1923 | C05 | 10 mM | 518-75-2 | CITRININ | antibacterial | -1.218574 | 0 |
| 49 | BIOMOL2 | 1792 | L07 | 5mg/mL | AP-300 | TPEN | cell permable heavy metal chelator | -1.18808 | 0 |
| 50 | Prestwick | 1569 | M11 | 2 mg/ml | 70476-82-3 | Mitoxantrone dihydrochloride | topoisomerase II inhibitor, antineoplastic | -1.170389 | 0 |
| 51 | NINDS | 1921 | N08 | 10 mM | 2112992 | CAMPTOTHECIN | topoisomerase I inhibitor, antineoplastic | -1.152003 | 0.06008 |
| 52 | NINDS | 1922 | O21 | 10 mM | 70476-82-3, 65271-80-9 [mitoxantrone] | MITOXANTHRONE HYDROCHLORIDE | antineoplastic | -1.141116 | 0 |
| 53 | NINDS | 1920 | J10 | 10 mM | 19237-84-4, 19216-56-9 [prazosin] | PRAZOSIN HYDROCHLORIDE | antihypertensive | -1.098414 | 0 |
| 54 | NINDS | 1922 | O20 | 10 mM | 508-75-8 | CONVALLATOXIN | cardiotonic | -1.088732 | 0 |
| 55 | Prestwick | 1568 | D20 | 2 mg/ml | 58-54-8 | Ethacrynic acid | Glutathione S-transferase inhibitor | -1.088665 | 9/E-7 |
| 56 | BIOMOL2 | 1792 | O17 | 5mg/mL | AC-146 | prazocin | adrenoreceptor agonist | -1.074835 | 0 |
| 57 | BIOMOL2 | 1792 | B13 | 5mg/mL | CM109 | ouabain | Na+K+ATPase inhibitor | -1.050767 | 0.000246 |
| 58 | NINDS | 1922 | H22 | 10 mM | 119413-54-6 | TOPOTECAN HYDROCHLORIDE | topoisomerase I inhibitor, antineoplastic | -1.033386 | 0 |
| 59 | BIOMOL2 | 1792 | N07 | 5mg/mL | KC-140 | valinomycin | K+ ionophore | -0.984651 | 0 |
| 60 | NINDS | 1921 | D17 | 10 mM | 58-58-2, 53-79-2 [puromycin] | PUROMYCIN HYDROCHLORIDE | antineoplastic, antiprotozool | -0.889753 | 0 |
| 61 | Prestwick | 1569 | O13 | 2 mg/ml | 33419-42-0 | Etoposide | topoisomerase II inhibitor, antineoplastic | -0.881592 | 0.003217 |
| 62 | Prestwick | 1571 | E13 | 2 mg/ml | 466-06-8 | Proscillaridin A | Na+/K+-ATPase inhibitor. Cardiac glycoside. | -0.876806 | 5/E-6 |

* Shaded cells indcate validated compounds

** Colored cells highlight compounds that have similar biological activity.

Table 5. List of small molecules found to suppress Huntingtin toxicity. Compounds that inhibit mutant Htt138Q aggregation and revert primary culture morphology towards Htt15Q control are listed:

| I.D. | Compound | CAS# | Function | Htt Culture | Htt138Q Aggregate $Log_2$ ratio* | Aggregate Suppression Significance (p-value) | Morphology Statistical Significance** (p-value) | Library | ICCB Plate I.D., Well # | Well Conc |
|---|---|---|---|---|---|---|---|---|---|---|
| 1 | Camptothecin | 7689-03-4 | topoisomerase I inhibitor, antineoplastic | 138Q | -1.637238 | 0.00000365 | 0.151249 | BIOMOL2 | 1791, N06 | 26 uM |
| 2 | Camptothecin | 7689-03-4 | topoisomerase I inhibitor, antineoplastic | 138Q | -1.22608 | 0.00000927 | 0.109087 | Prestwick | 1568, H21 | 10 uM |
| 3 | Camptothecin | 7689-03-4 | topoisomerase I inhibitor, antineoplastic | 138Q | -1.152003 | 0.00000000 | 0.06008 | NINDS | 1921, N08 | 20 uM |
| 4 | Etoposide | 33419-42-0 | topoisomerase II inhibitor, antineoplastic | 138Q | -0.881592 | 0.00003212 | 0.003217 | Prestwick | 1569, O13 | 6.8 uM |
| 5 | 10-OH-Camptothecin | 64439-81-2 | topoisomerase 1 inhibitor, antineoplastic | 138Q | -2.21028 | 0.00000012 | 0.00242 | BIOMOL2 | 1791, B06 | 26 uM |
| 6 | Ouabain | 630-60-4 | Na+/K+-ATPase inhibitor | 138Q | -1.050767 | 0.00000268 | 0.000246 | BIOMOL2 | 1792, B13 | 17 uM |
| 7 | Proscillaridin A | 466-06-8 | Na+/K+-ATPase inhibitor | 138Q | -0.876806 | 0.00020794 | 0.000006 | Prestwick | 1571, E13 | 7.5 uM |
| 8 | Ethacrynic acid | 58-54-8 | GST inhibitor | 138Q | -1.088665 | 0.00003454 | 0.000001 | Prestwick | 1568, D20 | 13.2 uM |

* Compounds listed are a subset of those found to inhibit Htt138Q aggregate formation ( $P < 0.05$).

** $P > 0.05$ indicate mutant Htt138Q neurite morphology is reverted towards Htt15Q controls.

Table 6: RNAi validation. Effect of Lkb-1 or Top knockdown on Htt138Q aggregate formation and rescue of mutant culture morphology.

| Gene Target | dsRNA amplicon | Off-targets | Htt Culture | Aggregate Suppression Significance (p-value)* | Morphology Statistical Significance (p-value)** | n = |
|---|---|---|---|---|---|---|
| LKB-1 | DRSC16481 | 0 | 138Q | 0.995894 | 0.152692 | 12 |
| LKB-1 | DRSC36925 | 0 | 138Q | 0.730001 | 0.979141 | 12 |
| LKB-1 | DRSC36926 | 0 | 138Q | 0.89236 | 0.702101 | 12 |

(continued)

| Gene Target | dsRNA amplicon | Off-targets | Htt Culture | Aggregate Suppression Significance (p-value)* | Morphology Statistical Significance (p-value)** | n = |
|---|---|---|---|---|---|---|
| Top 1 | DRSC36056 | 0 | 138Q | 0.833703 | 0.428127 | 12 |
| Top 1 | DRSC20295 | 0 | 138Q | 0.968551 | 0.015374 | 12 |
| Top 2 | DRSC36057 | 0 | 138Q | 0.993983 | 0.208051 | 12 |
| Top 2 | DRSC03459 | 0 | 138Q | 0.960684 | 0.35244 | 12 |
| Top 3α | DRSC03460 | 0 | 138Q | 0.980167 | 0.102255 | 12 |
| Top 3α | DRSC37672 | 0 | 138Q | 1 | 0 | 12 |
| Top 3β | DRSC18724 | 0 | 138Q | 0.288516 | 0.331471 | 12 |
| Mock | N/A | N/A | 138Q | 1 | 0 | 12 |
| Mock | N/A | N/A | 15Q | 0 | 1 | 72 |

* Htt Aggregates. $P < 0.05$ Indicates suppression of Htt138Q aggregate formation.
** $P > 0.05$ indicate mutant Htt138Q neurite morphology is reverted towards Htt15Q controls.

**Example 3: identified compounds ameliorate polyQ tract expansion phenotypes *in vivo***

To further examine the RNAi/small molecule screen hits, we assayed *in vivo* efficacy by testing their ability to rescue lethality in our *Drosophila* HD model. Htt Q138-mRFP$^1$ expression in the nervous system results in pupal lethality when animals are reared on standard media. Animals undergo metamorphosis but fail to eclose. In liquid culture, the longevity of the Htt-Q138$^1$ expressing animals is reduced, and larvae perish during the 2$^{nd}$ instar stage, likely secondary to drowning from decreased motility. Rapamycin, a well characterized mTOR inhibitor (Ravikumar et al., 2004), suppresses neurodegeneration in various HD models, and we found it enhanced viability of Htt-Q138$^1$ expressing larvae reared in liquid culture in a dose-dependent fashion compared to DMSO-treated controls (Fig. 5a). Using this assay, we found that Camptothecin and 10-Hydroxycamptothecin also increased larval longevity *in vivo,* but to a lesser extent than Rapamycin (Fig. 5a). 10-Hydroxycamptothecin is more efficacious than Camptothecin, possibly due to solubility differences, as Camptothecin readily precipitates when added to cultures. Specific inhibitors of Lkb1 were not available for *in vivo* testing. Given its role as an upstream regulatory kinase of the mTOR/Insulin pathway, we tested additional pharmacological agents that regulate this pathway, including Metformin (an mTOR pathway activator and oral anti-diabetic drug) (Shaw et al., 2005; Hardie, 2006) and 18β-Glycyrrhetinic acid (a putative mTOR inhibitor and neuroprotective agent) (Kao et al., 2009). Metformin could not revert Htt-Q138 lethality. However, 18β-Glycyrrhetinic acid was almost as efficacious as Rapamycin (Fig. 5a). We also tested an analogue of 18β-Glycyrrhetinic acid that has increased solubility: Carbenoxolone, and found it to have comparable activity. 18β-Glycyrrhetinic acid is non-toxic and has been used as a commercial sweetener, making it an attractive candidate for future studies for suppressive effects in mammalian HD models. Similarly, carbenoxolone has been approved and used for the treatment of ulcers. Rapamycin, in contrast, has numerous cytotoxic side-effects that limit its potential as a therapeutic.

**Example 4: *lkb1* genetic interaction studies**

To further examine the role of the Lkb1/Insulin pathway in the suppression of HD toxicity, we conducted genetic interaction studies with *lkb1* loss-of-function mutations. While pan-neuronal expression of Htt-Q138$^1$ causes pupal lethality, the introduction of a heterozygous *lkb1* null mutation into the Htt-Q138 background suppresses lethality. We observed no C155/+; UAS-Htt138Q/+ adult escapers at 25°C (n=83 pupae), however, the introduction of an Lkb1$^{4B1-11}$/+ or Lkb1$^{4A4-2}$/+ allele into this background led to an adult escaper frequency of 1.8% (n=110 pupae) and 3.7% (n=81 pupae) respectively.Using quantitative Western blot analysis, we found that the introduction of *lkb1* trans-heterozygous alleles does not reduce Htt138Q protein levels (Fig. 5D), suggesting the suppressive effects are not tied to altering Htt expression. This is in contrast to another rescuing deficiency we identified in an independent screen, Df(3L)vin7, which significantly decreases Htt138Q expression and yields an escaper frequency of 25.9% (n=85 pupae). The Lkb1 heterozygous animals expressing Htt138Q are viable and have relatively normal walking ability, although they do not inflate their wings (Fig. 5C).

To further investigate the relationship between lkb1 and mutant Htt138Q toxicity, we introduced the Lkb1$^{4A4-2/+}$ allele into a weaker Htt138Q expressing strain (C155/+; UAS-Htt138QmRFP$^2$/+) which is adult viable so that we could evaluate climbing behavior as an indicator of motor performance. From negative geotaxis assays performed on 25-day

old flies, we found that introduction of an Lkb1$^{4Aa-2}$/+ mutant background enhanced performance only in the C155:Htt138Q background, but had no effect on either C155 or C155:Htt15Q control backgrounds (Fig. 5E). This suggests that the toxicity effects of Htt138Q in neurons, is associated with Lkb-1 signaling.

Since RNAi knockdown as performed in our primary culture screening assay, is representative of a hypomorphic situation, and the *in vivo lkb1* rescue studies we conducted were haplo-insufficient, partial knockdown screening can be advantageous to uncover therapeutic targets. Full knockdown of *lkb1* would not have revealed beneficial effects, as homozygous *lkb1* null mutants are lethal and have cell polarity defects (Martin and St Johnston, 2003).

**Example 5: Camptothecin acts through a Top1-independent pathway**

To investigate the mechanism of action of Camptothecins in suppression of Htt-Q138 neurotoxicity, we performed genetic loss-of-function studies with target effector proteins. Since Camptothecins function as *Top1* inhibitors, we reasoned that *Top1* RNAi knockdown in primary cultures should phenocopy Camptothecin treatment and suppress HD pathology. RNAi knockdown of *Top1* or other annotated *Drosophila Top* genes (*Top2, 3$\alpha$ or 3$\beta$*), either singularly or in combination, did not suppress Htt aggregation (Table 6). Knockdown of the Tops did, however, partially revert the mutant Htt138Q neurite morphology towards controls. To extend these studies *in vivo* we introduced a heterozygous *Top1* null allele into the HD model background, but this had no effect on Htt-Q138-induced pupal lethality, as no adult escapers were observed. Given that the Camptothecins have a robust effect on Htt138Q aggregation inhibition, while Top-knockdown does not, these results suggest that the Camptothecins may act through a Top1-independent pathway to suppress Htt-138Q aggregation. Given that Camptothecins, GW5074 and 18β-Glycyrrhetinic acid have partially overlapping backbone ring-structures, it will be interesting to conduct structure-function analysis to determine what minimal architecture is required for these compounds to elicit their effects.

**Summary**

In summary, we have used the presence of aggregates and neuronal morphology as biomarkers to identify RNAi and small molecule suppressors of HD toxicity. In contrast to non-neuronal cell culture screens for poly-Q protein aggregation, the use of neuronal cultures displaying complex morphological features provides sensitive indicators of alterations in cellular physiology. Our screening system has led to the identification of *lkb1*, an upstream kinase regulator of the mTOR/Insulin pathway, as a suppressor of mutant poly-Q Htt toxicity. We have also identified two new classes of compounds that have promising HD therapeutic efficacy: 18β-Glycyrrhetinic acid and its analogs, carbenoxolone and its analogs, and the Camptothecins. With improved methods for image analysis of complex morphologies as presented here, high-content screening in specialized cells such as neurons represents a favorable approach for identifying suppressors of neuropathology.

LKB-1 knockdown was found to suppress mutant Htt toxicity in our system, as it rescued the dysmorphic primary neural culture morphology in vitro and restored viability in vivo. LKB-1 has been extensively studied, and mutations in the locus result in the Peutz Jeghers Syndrome (PJS) (Jenne DE, Reimann H, Nezu J, Friedel W, Loff S, et al. (1998) Peutz-Jeghers syndrome is caused by mutations in a novel serine threonine kinase. Nat Genet 18: 38-43; and van Veelen W, Korsse SE, van de Laar L, Peppelenbosch MP (2011) The long and winding road to rational treatment of cancer associated with LKB1/AMPK/TSC/mTORC1 signaling). In Drosophila, loss of LKB-1 in the embryonic nervous system blocks apoptosis and results in hyperplasia (Lee JH, Koh H, Kim M, Park J, Lee SY, et al. (2006) JNK pathway mediates apoptotic cell death induced by tumor suppressor LKB1 in Drosophila. Cell Death Differ 13: 1110-1122. How a partial LKB-1 knockdown elicits its beneficial effect in our system is still uncertain, although decreased levels of LKB-1 may reduce apoptosis caused by mutant Htt. LKB-1 lies upstream of many pathways that have previously been implicated in HD, including the mTOR/autophagy pathway (Ravikumar B, Vacher C, Berger Z, Davies J, Luo S, et al. (2004) Inhibition of mTOR induces autophagy and reduces toxicity of polyglutamine expansions in fly and mouse models of Huntington disease. Nat Genet. pp. 585-595; Sarkar S, Perlstein EO, Imarisio S, Pineau S, Cordenier A, et al. (2007) Small molecules enhance autophagy and reduce toxicity in Huntington's disease models. Nat Chem Biol 3: 331-338. Epub 2007 May 2007; and Fleming A, Noda T, Yoshimori T, Rubinsztein DC (2011) Chemical modulators of autophagy as biological probes and potential therapeutics. Nat 7: 9-17) and the Insulin/AMPK signaling network (Yamamoto A, Cremona M, Rothman J (2006) Autophagy-mediated clearance of Huntingtin aggregates triggered by the insulin-signaling pathway. Journal of Cell Biology. pp. 719; David DC, Ollikainen N, Trinidad JC, Cary MP, Burlingame AL, et al. (2010) Widespread protein aggregation as an inherent part of aging in C. elegans. PLoS 8: e1000450). Recently our findings were corroborated in vertebrates as activation of AMPK, the main kinase target of LKB-1, was found to potentiate striatal neurodegeneration in HD (Ju TC, Chen HM, Lin JT, Chang CP, Chang WC, et al. (2011) Nuclear translocation of AMPK-{alpha} 1 potentiates striatal neurodegeneration in Huntington's disease. J Cell Biol 194: 209-227).

Several compounds that suppressed mutant Htt toxicity in our primary culture system have previously been shown to have neuroprotective effects in mammalian systems, indicating that the assay with Drosophila primary cultured

neurons has translational capacity. Compound GW5074 inhibited mutant Htt aggregate formation in our system, and also reduced striatal degeneration in the NP-3 mouse HD model (Chin PC, Liu L, Morrison BE, Siddiq A, Ratan RR, et al. (2004) The c-Raf inhibitor GW5074 provides neuroprotection in vitro and in an animal model of neurodegeneration through a MEK-ERK and Akt-independent mechanism. J Neurochem 90: 595-608). Similarly, 18β-Glycyrrhetinic acid, which rescued HD toxicity in vivo in our Drosophila assays, has been shown to suppresses neurotoxicity in a PC12 cellular stress model (Kao TC, Shyu MH, Yen GC (2009) Neuroprotective effects of glycyrrhizic acid and 18beta-glycyrrhetinic acid in PC 12 cells via modulation of the PI3K/Akt pathway. J Agric Food Chem 57: 754-761).

By analyzing the molecular structures and mechanisms of action of the small molecules identified as mutant Htt suppressors, new avenues to investigate the biology of HD pathogenesis have been uncovered. 18 β -Glycyrrhetinic acid, and carbenoxolone, which were used to pharmacologically manipulate LKB-1 dependent pathways and rescue HD toxicity in vivo in this work, have also been reported to block gap junction activity (Juszczak GR, Swiergiel AH (2009) Properties of gap junction blockers and their behavioural, cognitive and electrophysiological effects: animal and human studies. Prog Neuropsychopharmacol Biol Psychiatry 33: 181-198. Epub 2009 Jan 2001). Recently there have been several reports that mutant Htt expressed in glia can trigger neuronal defects (Shin JY, Fang ZH, Yu ZX, Wang CE, Li SH, et al. (2005) Expression of mutant Huntingtin in glial cells contributes to neuronal excitotoxicity. J Cell Biol 171: 1001-1012 ; Tamura T, Sone M, Yamashita M, Wanker EE, Okazawa H (2009) Glial cell lineage expression of mutant ataxin-1 and Huntingtin induces developmental and late-onset neuronal pathologies in Drosophila models. PLoS One 4: e4262. Epub 2009 Jan 4223; Bradford J, Shin JY, Roberts M, Wang CE, Sheng G, et al. (2010) Mutant Huntingtin in glial cells exacerbates neurological symptoms of Huntington disease mice. J 285: 10653-10661. Epub 12010 Feb 10659; Kretzschmar D, Tschape J, Bettencourt Da Cruz A, Asan E, Poeck B, et al. (2005) Glial and neuronal expression of polyglutamine proteins induce behavioral changes and aggregate formation in Drosophila. Glia 49: 59-72). In addition, postmortem analysis of HD patient brain samples revealed increased activated astrocytes and reactive microglia in the striatum and cortex compared to similar aged non-diseased brains (Sapp E, Kegel KB, Aronin N, Hashikawa T, Uchiyama Y, et al. (2001) Early and progressive accumulation of reactive microglia in the Huntington disease brain. J Neuropathol Exp Neurol 60: 161-172). Gap junctions allow astrocytes to communicate via elaborate networks, and there is evidence that cell death signals can be propagated through gap junction networks (Lin JH, Weigel H, Cotrina ML, Liu S, Bueno E, et al. (1998) Gap-junction-mediated propagation and amplification of cell injury. Nat Neurosci 1: 494-500).

These reports, in combination with the findings disclosed herein indicate that, surprisingly, modulating gap junction activity with non-toxic compounds such as 18β-Glycyrrhetinic acid or carbenoxolone results in neuroprotective benefits in an in vivo model of HD. 18β-Glycyrrhetinic acid derivatives are particularly attractive for clinical applications, because they have already been evaluated in two clinical trials for other indications (ClinicalTrials.gov Identifier: NCT00384384 and NCT00759525), and are widely used as commercial sweeteners. Recently, an 18β-Glycyrrhetinic acid derivative was found to be efficacious in the treatment of two mouse models of Amyotrophic Lateral Sclerosis (ALS) and an Alzheimer's Disease model, further supporting the therapeutic value of this class of compounds for neurodegenerative diseases (Takeuchi H, Mizoguchi H, Doi Y, Jin S, Noda M, et al. (2011) Blockade of gap junction hemichannel suppresses disease progression in mouse models of amyotrophic lateral sclerosis and Alzheimer's disease. PLoS One 6: e21108).

Camptothecins were very effective at suppressing the dystrophic neuronal profiles and mutant Htt aggregation in our assay. Camptothecins are potent anti-cancer drugs that block cell division through several mechanisms including the introduction of DNA replication-dependent double-stranded breaks which trigger apoptosis, and down regulation of Top-1 by activation of proteasome pathways. In quiescent neurons, Camptothecins most likely cause transcriptional repression as a result of collisions between RNA polymerase and immobilized Top-1/Camptothecin complexes linked to the DNA. In our system, the benefit of Camptothecin treatment could theoretically be related to decreased Htt transgene expression, although we did not observe any decrease in Htt-mRFP fluorescence, even after one week of continuous exposure to the drug. Since targeted knockdown of mutant Htt via siRNA has been found to be effective at reversing disease progression in mouse models, small molecule transcriptional repressors may offer another therapeutic avenue to control HD (DiFiglia M, Sena-Esteves M, Chase K, Sapp E, Pfister E, et al. (2007) Therapeutic silencing of mutant Huntingtin with siRNA attenuates striatal and cortical neuropathology and behavioral deficits. Proc Natl Acad Sci U S A 104: 17204-17209. Epub 12007 Oct 17216). Although toxicity issues have been reported in neural cultures following Camptothecin treatment, we did not observe morphological defects in our Drosophila HD model (Lang-Rollin IC, Rideout HJ, Noticewala M, Stefanis L (2003) Mechanisms of caspase-independent neuronal death: energy depletion and free radical generation. J Neurosci 23: 11015-11025). Camptothecins have been reported to regulate a number of different pathways, including activation of the ubiquitin/proteasome system and upregulation of mitochondrial biogenesis. These secondary Camptothecin effects could alleviate toxic Htt cellular stress by removing toxic Htt species or restoring energy homeostasis (Kluza J, Marchetti P, Gallego MA, Lancel S, Fournier C, et al. (2004) Mitochondrial proliferation during apoptosis induced by anticancer agents: effects of doxorubicin and mitoxantrone on cancer and cardiac cells. Oncogene 23: 7018-7030; Reipert S, Berry J, Hughes MF, Hickman JA, Allen TD (1995) Changes of mitochondrial mass in the hemopoietic stem cell line FDCP-mix after treatment with etoposide: a correlative study by multiparameter flow cytometry

and confocal and electron microscopy. Exp Cell Res 221: 281-288; Fu X, Wan S, Lyu YL, Liu LF, Qi H (2008) Etoposide induces ATM-dependent mitochondrial biogenesis through AMPK activation. PLoS One 3: e2009; and Thomas CJ, Rahier NJ, Hecht SM (2004) Camptothecin: current perspectives. Bioorg Med Chem 12: 1585-1604).

There has been debate in field about the contribution of Htt aggregates to disease pathology for many years. Several studies have shown that Htt aggregates accumulate in fine neuronal processes such as axons and dendrites, and block axon-transport to negatively impact cell heath (Sapp E, Penney J, Young A, Aronin N, Vonsattel JP, et al. (1999) Axonal transport of N-terminal Huntingtin suggests early pathology of corticostriatal projections in Huntington disease. J Neuropathol Exp Neurol 58: 165-173; Gunawardena S, Her LS, Brusch RG, Laymon RA, Niesman IR, et al. (2003) Disruption of axonal transport by loss of Huntingtin or expression of pathogenic polyQ proteins in Drosophila. Neuron 40: 25-40; and Trushina E, Dyer RB, Badger JD, 2nd, Ure D, Eide L, et al. (2004) Mutant Huntingtin impairs axonal trafficking in mammalian neurons in vivo and in vitro. Mol Cell Biol 24: 8195-8209). Real-time imaging experiments have suggested that soluble Htt, and not aggregates, correlate better with cellular toxicity (Arrasate M, Mitra S, Schweitzer ES, Segal MR, Finkbeiner S (2004) Inclusion body formation reduces levels of mutant Huntingtin and the risk of neuronal death. Nature 431: 805-810). Given this controversy, we chose not to use aggregate suppression as the sole metric to identify small molecules and RNAi knockdown probes that have therapeutic value and included an additional parameter: neurite morphology. We found, surprisingly, that neurite processes are sensitive to mutant polyQ-expanded Htt and offer a means of identifying drugs and RNAi knock-downs that have non-specific toxicity effects. Using this assay we were able to identify compounds and RNAi knockdowns that have potential therapeutic value and could not have been identified with conventional assays relying solely on aggregate suppression as the readout metric. Although the physiological link between aggregate inhibition and improved neuronal health remains to be investigated in more detail, we discovered compounds that improved neurite morphology in addition to reducing mutant Htt aggregation, providing evidence that aggregates can at least contribute to toxicity.

Drosophila models of neurodegenerative disease have been a powerful tool for understanding mechanisms of neurodegeneration for more than a decade, and have more recently been applied directly to drug discovery as well (Ambegaokar SS, Roy B, Jackson GR (2010) Neurodegenerative models in Drosophila: polyglutamine disorders, Parkinson disease, and amyotrophic lateral sclerosis. Neurobiol 40: 29-39. Epub 2010 May 2031; Lim KL (2010) Non-mammalian animal models of Parkinson's disease for drug discovery. Expert Opin Drug Discov 5: 165-176; O'Kane CJ (2011) Drosophila as a Model Organism for the Study of Neuropsychiatric Disorders; Bilen J, Bonini NM (2005) Drosophila as a model for human neurodegenerative disease. Annu Rev Genet 39: 153-171; Agrawal N, Pallos J, Slepko N, Apostol BL, Bodai L, et al. (2005) Identification of combinatorial drug regimens for treatment of Huntington's disease using Drosophila. Proc Natl Acad Sci U S A 102: 3777-3781; Steffan JS, Bodai L, Pallos J, Poelman M, McCampbell A, et al. (2001) Histone deacetylase inhibitors arrest polyglutamine-dependent neurodegeneration in Drosophila. Nature 413: 739-743; Auluck PK, Bonini NM (2002) Pharmacological prevention of Parkinson disease in Drosophila. Nat Med. pp. 1185-1186; Min KT, Benzer S (1999) Preventing neurodegeneration in the Drosophila mutant bubblegum. Science 284: 1985-1988). Aside from genetic tools in Drosophila and the host of neurodegenerative disease models available, it is an attractive model for conducting suppressor screens given the lack of gene redundancy often observed in mammals. While single gene knock-down studies often fail to produce robust phenotypes in mammals, this is not the case in Drosophila (Banovic D, Khorramshahi O, Owald D, Wichmann C, Riedt T, et al. (2010) Drosophila neuroligin 1 promotes growth and postsynaptic differentiation at glutamatergic neuromuscular junctions. Neuron 66: 724-738; and Williams R (2006) Development: Neuroligin knockouts: form but no function. Nature Reviews Neuroscience 7: 831). We have found that the complex neural morphologies of Drosophila primary cultures can also provide sensitive information about the general cell physiological status of a disease model. The algorithms that we have used in this study can help quantify complex morphologies can also facilitate the identification of disease modifying genes (Wu C, Schulte J, Sepp KJ, Littleton JT, Hong P (2010) Automatic robust neurite detection and morphological analysis of neuronal cell cultures in high-content screening. Neuroinformatics 8: 83-100). Live imaging, as presented here, has the advantage over traditional cell staining experiments in that the fine neurite morphology of cultures is preserved. Detergents and washes needed for immunofluorescence-based assays can disrupt fine cellular processes and introduce artifacts, which reduce assay sensitivity and introduce noise. Live-cell imaging also makes it possible to collect different time points in a single experiment, which not only reduces labor but also enables one to track the effect of a compound or gene knockdown over time. Because of the ease and speed of conducting RNAi and compound screens in Drosophila primary culture systems, this methodology offers an attractive approach to identify disease-modifying agents for neurodegenerative diseases.

**Example 6: mouse model of Huntington's disease**

Animal models are of particular value for neurodegenerative disease research, for example, pre-clinical investigations of lead compounds for polyQ tract expansion disease therapy, as it is very difficult to approximate the environment of an aging, degenerating neuron in vitro. In order to translate the strategy described herein for Drosophila to a mammalian model, several mouse strains were identified as a model for HD disease progression.

*Mouse strains:*_Mouse strain names provided herein adhere to the Guidelines for Nomenclature of Mouse and Rat Strains, Revised October 2011, International Committee on Standardized Genetic Nomenclature for Mice, accessible at www.informatics.jax.org/mgihome/nomen/strains.shtml. Official gene symbols, or, where appropriate, official mouse strain names of the Jackson Laboratory(www.jax.org, e.g., the Jackson Laboratory Mice Database at jaxmice.jax.org) are used. There are numerous HD mouse strains suitable for candidate compound or nucleic acid construct screening, and for pre-clinical evaluations of candidates for the treatment of HD such as the HD model strains R6/2, R6/1, N171-82Q, CAG140, HdhQ1 11, BACHD, and CAG150 (Crook ZR, Housman D (2011) Huntington's disease: can mice lead the way to treatment? Neuron. 69:423-35; the entire contents of which are incorporated herein by reference). The HD model strains differ in their manifestations and time line of HD-like symptoms, e.g., motor and cognitive dysfunctions. Depending on the HD strain, mice show morbidity as early as at 12 weeks of age or as late as at 30 weeks, while other strains do not show early morbidity. For pre-clinical evaluation of carbenoxolone, four cohorts of mice with 20 mice per cohort are treated as follows: Cohort 1: + drug; Cohort 2: no drug; Cohort 3: wild type, sex-, age-, and/or genetic background-matched, + drug; and Cohort 4: wild type, sex-, age-, and/or genetic background-matched, no drug. For example, for an exemplary experiment involving the R6/2 strain, which is bred on a C57BL/6 genetic background, the cohorts for an evaluation of carbenoxolone are: Cohort 1: 20 R6/2 mice, treated with carbenoxolone; Cohort 2: 20 R6/2 mice, mock treated with vehicle (no carbenoxolone); Cohort 3: 20 C57BL/6 mice, treated with carbenoxolone; and Cohort 4: 20 B57BL/6 mice, mock treated with vehicle (no carbenoxolone). Depending on the HD strain, different age groups are treated, for example, pre-symptomatic age groups and post-symptomatic age groups.

*Carbenoxolone:* In order to achieve precise dosing, carbenoxolone is administered intraperitoneally. Carbenoxolone, sodium salt, (SIGMA C4790) is dissolved in sterile, injectable saline, at a concentration of between 0.02 and 2 mg/mL. The solution is filtered (0.22 micron) prior to IP injection. Between 0.2 mg/kg and 35 mg/kg in a volume of 0.01 ml/g body weight are injected intraperitoneally (e.g., a 25 g mouse receives between 5 and 500 $\mu$g in 0.25 mL). Administration begins at 4 weeks of age, and is repeated every other day until the end of the experiment. Mice are tested weekly in behavioral assays (Morris water maze and accelerating rotarod).

*Behavioral tests:* HD involves motor, psychological and behavioral symptoms in human patients, all of which are progressive and eventually terminal. Many of these same symptoms are recapitulated to an extent in mouse models of HD, including the mouse model strains provided herein. For example, the strains R6/2 and N171-82Q express a fragment of the mutant protein responsible for HD and have been well studied, demonstrating a decline in performance at motor and cognitive tasks. Both strains have a shortened life span (~12-14 weeks for R6/2, and ~24 weeks for N171-82Q). Testing for both strains commences at age 4 weeks. The rotarod assay is used to assess motor deterioration and coordination loss, while the Morris water maze is used to measure spatial learning. Performance in both of these apparatuses is known to diminish with age in HD model mouse strains, for example, in the R6/2 and N171-82Q mice.

Progression of HD phenotype and the effect, if any, of a candidate HD therapeutic on HD phenotype and phenotype progression can be measured by various behavioral tests, including rotarod and Morris water maze assay (Lione LA, Carter RJ, Hunt MJ, Bates GP, Morton AJ, and Dunnett SB (1999) Selective Discrimination Learning Impairments in Mice Expressing the Human Huntington's Disease Mutation. J. Neuroscience. 19: 10428-10437; ; the entire contents of each of which are incorporated herein by reference). In order to assess the effect of a candidate nucleic acid construct, for example, in the context of pre-clinical evaluations of such a candidate, behavioral assessment is peformed for each experimental animal before administration of the respective candidate HD therapeutic nucleic acid construct or compound, and performance in the respective behavioral assay is compared to untreated control animals. In some experiments, behavioral assays are run repeatedly at different time points post-administration to determine whether a beneficial effect of the compound on an HD-like symptom can be observed.

*Rotarod assay:* Mice are habituated to balancing on a slowly rotating rotarod (5 rpm). Following one day of habituation, the mice are placed onto a rotating rotarod that is accelerating from 4 to 40 rpm over 10 minutes, the maximum time to be used. The time to fall off the rod, onto a platform located ~30cm below the rod, is measured. The increase in latency to fall during the course of training is compared between four groups of mice for each candidate compound to be evaluated (Mutant + candidate, mutant no candidate, wild type + candidate, wild type no candidate). For example, the increase in latency to fall is compared amongst the following groups of mice: Cohort 1: 20 R6/2 mice, treated with carbenoxolone; Cohort 2: 20 R6/2 mice, mock treated with vehicle (no carbenoxolone); Cohort 3: 20 C57BL/6 mice, treated with carbenoxolone; and Cohort 4: 20 B57BL/6 mice, mock treated with vehicle (no carbenoxolone). An increased latency to fall in a cohort treated with a candidate compound or nucleic acid construct, as compared to a non-treated control, indicates an ameliorating effect of the candidate on HD-related motor skill impairments.

*Morris water maze assay:* The water maze assay measures spatial learning in mice. The water maze apparatus is a circular pool (1.2 meters in diameter) filled with water made opaque by a small amount of non-toxic paint powder. A platform, when present, will be in the center of one of the four quadrants, and the test assesses working spatial learning and memory by measuring how quickly a mouse can find the platform hidden 0.5 cm below water level. Four quadrants are marked by different visual queues, and the tester is not visible during testing. The training protocol consists of daily sessions for 10 days (four, 60 sec. trials per session per day), tested starting at either 4, 8, or 12 weeks of age. The first

day is a training period where the platform is made more visible with a high-contrast flag. The sixth day has a single-trial probe test without the platform, and the time spent in each quadrant is recorded. The final three days measure the mouse's ability to reverse and re-learn the test by moving the platform to the opposite quadrant. The navigation of the mice is tracked by video camera, and the escape latency to the platform is recorded. Mice will be allowed to swim for a maximum of 60 seconds; mice remaining in the water at this point are manually placed on the platform. After reaching the platform, mice are left there for 15 seconds, removed, dried off, and placed in their home cage on a warming rack or mat for the intertrial interval (10 mins). Latency to escape is increased in HD model mouse strains due to an impairment in cognitive skills. A reduced latency to escape in a cohort treated with a candidate compound or nucleic acid construct, as compared to a non-treated control, indicates an ameliorating effect of the candidate on HD-related cognitive skill impairments.

Experimentals

HD model strain mice are treated with carbenoxolone or shRNAs targeting lkb1, starting at 4 weeks of age. At 8 weeks of age, motor and cognitive performance are tested in rotarod and water maze assays. Untreated mice are expected to show a significant impairment of motor and cognitive capabilities at 8 weeks of age, as measured by a shortened latency to fall in the rotarod assay, and a lengthened latency to escape in the water maze assy, respectively, as compared to wid type control mice. Treated mice, however, are expected to show a lesser degree of impairment of motor and cognitive capabilites, as measured by a shortened latency to fall in the rotarod assay, and a lengthened latency to escape in the water maze assay, respectively, as compared to untreated HD mice.

**Example 7: Use of Carbenoxolone as an HD therapeutic agent**

In some contemplated embodiments, the 18β-Glycyrrhetinic acid analog carbenoxolone is administered to a subject having or suspected of having the polyQ tract expansion disease HD. The carbenoxolone is administered orally in tablet form, for example, as carbenoxolone disodium salt. The dosage is within the range of about 100-700 mg/day, for example at about 150 mg/day, about 300 mg/day, or about 600 mg/day. The subject is assessed for symptoms of HD at the beginning or before administration of carbenoxolone. The subject is further monitored during the course of administration of carbenoxolone to determine whether an improvement in a symptom of HD is ameliorated or not. Such monitoring includes measuring cognitive and motor function in the subject, and/or determining whether a slowing or reversal of a personality change commonly associated with HD is observable. In some instances, the monitoring includes measuring the aggregation of Htt protein, the number or size of inclusion bodies, and/or brain tissue homeostasis (e.g. detection of improved survival of neuronal cells and/or reduction in astrocytes).

The method, in some embodiments, includes administering carbenoxolone at a dosage known to be non-toxic to humans, for example, at a dose of about 150 mg/day (e.g., 3 tablets comprising 50 mg carbenoxolone each per day). After a period of time sufficient for a desired change, e.g. an amelioration in an HD symptom, to manifest, the subject is then monitored for such a change. For example, the subject, in some embodiments, is monitored for cognitive function, for example, within a time frame of about a week to about 6 months (e.g., about one month or about two months) after administration is commenced. If no desirable change in clinical presentation is detected, e.g., if the subject does not show an improvement in cognition or still exhibits the same or an increased severity of symptoms, then the dose of carbenoxolone is increased. For example, in some embodiments, the dose may be increased from about 150 mg/day to about 300 mg/day. In some embodiments, the subject is monitored again for symptoms after dose adjustment and, if the symptoms persist at the same severity level, the dose is increased further. For example, in some embodiments, the dose may be increased from about 300 mg/day to about 450 mg/day. In some embodiments, multiple cycles of dose adjustment and monitoring are performed until a desired change in the severity of a symptom is observed. For example, in some embodiments, the dose may be increased from about 150 mg/day to about 300 mg/day in a first dose adjustment, then to about 450 mg/day, then to about 500 mg/day, then to about 600 mg/day. In some cases, the dose may be increased to an amount higher than 600 mg/day, particularly, where the treatment is well tolerated. If, on the other hand, the subject exhibits a desired change in the severity of HD symptoms, then the dose is maintained or even decreased. A decrease in carbenoxolone dosage is indicated, for example, if undesirable side effects (e.g., hypertension, hypoalkaemia, or sodium retention) are observed in the subject. In some cases, the dosage may be decreased below 150 mg/day, particularly, where a clinical improvement is still observed with lower doses. In some embodiments, the subject is monitored repeatedly and the dose of carbenoxolone is adjusted accordingly to find the minimal dose at which a desired change in HD symptoms is observed, but at which side effects are absent or tolerable. If side effects persist at the minimally effective dose, administration of one or more additional drugs for the treatment of the side effects (e.g., antihypertensive drugs, potassium supplements, or diuretics) is indicated.

An improvement of at least some HD symptoms, including, but not limited to an improvement of cognition, motor function, and an inhibition of the progression or a reversion of the personality change associated with HD, is

expected in HD subjects so treated.

**Example 8: Use of Carbenoxolone as an HD therapeutic agent in pre-symptomatic HD patients**

In some contemplated embodiments, the 18β-Glycyrrhetinic acid analog carbenoxolone is administered to a subject carrying a polyQ tract expansion mutation in the Huntingtin gene that is associated with HD, for example, a mutation resulting in a Huntingtin gene product comprising a pathogenic polyQ repeat length, for example, of 35 or more Q residues, or to a subject expressing a polyQ tract expanded polypeptide implicated in HD, for example, a Huntingtin polypeptide comprising a polyQ tract of more than 35 Q residues. In some embodiments, a 18β-Glycyrrhetinic acid analog, for example, carbenoxolone, is administered to a subject based on the subject carrying a polyQ tract expansion mutation in the Huntingtin gene that is associated with HD or expressing a polyQ tract expanded polypeptide implicated in HD. In some embodiments, a 18β-Glycyrrhetinic acid analog, for example, carbenoxolone, is administered to the subject before a clinical symptom of HD manifests, for example, before a motor impairment, cognitive impairment, behavioral impairment, restriction of independence, functional impairment, or and impairment in Total Functional Capacity (TFC) is clinically manifest. Clinical symptoms of HD and their manifestations are well known to those of skill in the art and can be measured and quantified according to methods well known to the skilled artisan (see., e.g., the Unified Huntington's Disease Rating Scale (UHDRS, Huntington Study Group (Kieburtz K, primary author). The Unified Huntington's Disease Rating Scale: Reliability and Consistency. Mov Dis 1996;11:136-142; the entire contents of which are incorporated herein by reference).

In some embodiments, 18β-Glycyrrhetinic acid or an analog thereof, for example, carbenoxolone, is administered to a subject carrying a polyQ tract expansion mutation in the Huntingtin gene that is associated with HD or expressing a polyQ tract expanded polypeptide implicated in HD, and exhibiting an elevated level of a glucocorticoid, for example, of cortisol, before a clinical symptom of HD, for example, a motor impairment, cognitive impairment, behavioral impairment, restriction of independence, functional impairment, or and impairment in Total Functional Capacity (TFC) is clinically manifest. In some embodiments, a 18β-Glycyrrhetinic acid analog, for example, carbenoxolone, is administered to the subject after a clinical symptom of HD has manifested, for example, after a motor impairment, cognitive impairment, behavioral impairment, restriction of independence, functional impairment, or and impairment in Total Functional Capacity (TFC) is clinically manifest. In some embodiments, the 18β-Glycyrrhetinic acid or an analog thereof, for example, carbenoxolone, is administered to a subject at an amount effective to reduce the elevated glucocorticoid level, for example, the cortisol level, in the subject, for example, to a non-pathogenic level, a level not deemed to be elevated, or a level expected to be present in a healthy subject.

The method, in some embodiments, includes administering carbenoxolone at a dosage known to be non-toxic to humans, for example, at a dose of about 150 mg/day (e.g., 3 tablets comprising 50 mg carbenoxolone each per day) to the subject. After a period of time sufficient for a change in a cortisol level to manifest, e.g. after about a week, about two weeks, about three weeks, or about a month, the cortisol level in the subject is measured. If no desirable change in the cortisol level is detected, e.g., if the cortisol level is unchanged (e.g., as compared to a prior measurement that determined an elevated cortisol level), or if the subject maintains an elevated cortisol level, then the dose of carbenoxolone is increased. For example, in some embodiments, the dose may be increased from about 150 mg/day to about 300 mg/day. In some embodiments, the cortisol level in the subject is measured again after dose adjustment and, if the cortisol level remains elevated, the dose is increased further. For example, in some embodiments, the dose may be increased from about 300 mg/day to about 450 mg/day. In some embodiments, multiple cycles of dose adjustment and cortisol level measurement are performed until a desired cortisol level is observed in the subject, for example, a blood plasma cortisol level within the range of 70-700nmol/l or 70-350nmol/l. For example, in some embodiments, the dose may be increased from about 150 mg/day to about 300 mg/day in a first dose adjustment, then to about 450 mg/day, then to about 500 mg/day, then to about 600 mg/day. In some cases, the dose may be increased to an amount higher than 600 mg/day, particularly, where the treatment is well tolerated. If, on the other hand, the subject exhibits a desired reduction in a cortisol level, for example, a reduction of an elevated cortisol level to a blood plasma cortisol level within the range of 70-700nmol/l, then the dose is maintained or even decreased. A decrease in carbenoxolone dosage is indicated, for example, if undesirable side effects (e.g., hypertension, hypoalkaemia, or sodium retention) are observed in the subject. In some cases, the dosage may be decreased below 150 mg/day, particularly, where a desired reduction in a cortisol level is still observed with lower doses. In some embodiments, the cortisol level in the subject is monitored repeatedly and the dose of carbenoxolone is adjusted accordingly to find the minimal dose at which a desired cortisol level is observed, but at which side effects are absent or tolerable. If side effects persist at the minimally effective dose, administration of one or more additional drugs for the treatment of the side effects (e.g., antihypertensive drugs, potassium supplements, or diuretics) is indicated.

A prevention or delay of the onset, and/or an amelioration of the severity of at least one HD symptom, including, but not limited to, an impairment of cognition, motor function, behavior, functionality, and Total Functional Capacity (TFC), is expected in subjects so treated. In some embodiments, in which the subject exhibits a symptom of HD at the

time of treatment, a delay in the progression of the disease, or an amelioration of at least one HD symptom, including, but not limited to, an impairment of cognition, motor function, behavior, functionality, and Total Functional Capacity (TFC), is expected in subjects so treated.

REFERENCES

Brand AH, Perrimon N (1993) Targeted gene expression as a means of altering cell fates and generating dominant phenotypes. Development 118: 401-415.

Chin PC, Liu L, Morrison BE, Siddiq A, Ratan RR, Bottiglieri T, D'Mello SR (2004) The c-Raf inhibitor GW5074 provides neuroprotection in vitro and in an animal model of neurodegeneration through a MEK-ERK and Akt-independent mechanism. J Neurochem 90:595-608.

DiFiglia M, Sapp E, Chase K, Davies S, Bates G, Vonsattel J, Aronin N (1997) Aggregation of Huntingtin in neuronal intranuclear inclusions and dystrophic neurites in brain. Science 1997 Sep 26;277(5334):1990-3.

Fisher, R.A. 1932. Statistical Methods for Research Workers. Oliver and Boyd, Edinburgh.

Graham R, Deng Y, Slow E, Haigh B, Bissada N, Lu G, Pearson J, Shehadeh J, Bertram L, Murphy Z (2006) Cleavage at the caspase-6 site is required for neuronal dysfunction and degeneration due to mutant Huntingtin. Cell 2006 Jun 16;125(6):1179-91.

Hardie D (2006) Neither LKB 1 nor AMPK are the direct targets of metformin. In: Gastroenterology, 2006 Sep;131 (3):973.

Hay DG, Sathasivam K, Tobaben S, Stahl B, Marber M, Mestril R, Mahal A, Smith DL, Woodman B, Bates GP (2004) Progressive decrease in chaperone protein levels in a mouse model of Huntington's disease and induction of stress proteins as a therapeutic approach. Hum Mol Genet 2004 13:1389-1405. Epub 2004 Apr 1328.

Hertzberg R, Busby R, Caranfa M, Holden K, Johnson R, Hecht S, Kingsbury W (1990) Irreversible trapping of the DNA-topoisomerase I covalent complex. Affinity labeling of the camptothecin binding site. In: Journal of Biological Chemistry, 1990 Nov 5;265(31):19287-95.

Inoki K, Corradetti MN, Guan KL (2005) Dysregulation of the TSC-mTOR pathway in human disease. Nat Genet 37:19-24.

Kao TC, Shyu MH, Yen GC (2009) Neuroprotective effects of glycyrrhizic acid and 18beta-glycyrrhetinic acid in PC 12 cells via modulation of the PI3K/Akt pathway. J Agric Food Chem 57:754-761.

Lee W-CM, Yoshihara M, Littleton JT (2004) Cytoplasmic aggregates trap polyglutamine-containing proteins and block axonal transport in a Drosophila model of Huntington's disease. In: Proc Natl Acad Sci USA, pp 3224-3229.

Martin S, St Johnston D (2003) A role for Drosophila LKB1 in anterior-posterior axis formation and epithelial polarity. In: Nature, pp 379-384.

Ravikumar B, Vacher C, Berger Z, Davies J, Luo S, Oroz L, Scaravilli F, Easton D, Duden R, O'Kane C (2004) Inhibition of mTOR induces autophagy and reduces toxicity of polyglutamine expansions in fly and mouse models of Huntington disease. In: Nat Genet, pp 585-595.

Roze E, Saudou F, Caboche J (2008) Pathophysiology of Huntington's disease: from Huntingtin functions to potential treatments. Curr Opin Neurol 21:497-503.

Schulte J, Sepp KJ, Jorquera RA, Wu C, Song Y, Hong P, Littleton JT (2010) Mob4/Phocein regulates synapse formation, axonal transport, and microtubule organization. J Neurosci. Apr 14;30(15):5189-203.

Sepp KJ, Hong P, Lizarraga SB, Liu JS, Mejia LA, Walsh CA, Perrimon N (2008) Identification of neural outgrowth genes using genome-wide RNAi. PLoS Genet 2008, Volume 4, Issue 7, e1000111.

Shaw R, Lamia K, Vasquez D, Koo S, Bardeesy N, DePinho R, Montminy M, Cantley L (2005) The kinase LKB1 mediates glucose homeostasis in liver and therapeutic effects of metformin. Science. 310(5754):1642-6.

Shaw RJ, Bardeesy N, Manning BD, Lopez L, Kosmatka M, DePinho RA, Cantley LC (2004) The LKB 1 tumor suppressor negatively regulates mTOR signaling. Cancer Cell 6:91-99.

Tsuchiya K, Kohda Y, Yoshida M, Zhao L, Ueno T, Yamashita J, Yoshioka T, Kominami E, Yamashima T (1999) Postictal blockade of ischemic hippocampal neuronal death in primates using selective cathepsin inhibitors. Exp Neurol 155:187-194.

Wang J, Gines S, MacDonald ME, Gusella JF (2005) Reversal of a full-length mutant Huntingtin neuronal cell phenotype by chemical inhibitors of polyglutamine-mediated aggregation. BMC Neurosci 6:1.

Wu C, Schulte J, Sepp KJ, Littleton JT, Hong P Automatic robust neurite detection and morphological analysis of neuronal cell cultures in high-content screening. Neuroinformatics 8:83-100.

Zhang X, Smith DL, Meriin AB, Engemann S, Russel DE, Roark M, Washington SL, Maxwell MM, Marsh JL, Thompson LM, Wanker EE, Young AB, Housman DE, Bates GP, Sherman MY, Kazantsev AG (2005) A potent small molecule inhibits polyglutamine aggregation in Huntington's disease neurons and suppresses neurodegeneration

in vivo. Proc Natl Acad Sci U S A 102:892-897. Epub 2005 Jan 2010.

The terms and expressions that have been employed are used as terms of description and not of limitation, and there is no intention in the use of such terms and expressions of excluding any equivalents of the features described, it being recognized that various modifications are possible within the scope of the invention. All definitions, as defined and used herein, should be understood to control over dictionary definitions, definitions in documents incorporated by reference, and/or ordinary meanings of the defined terms.

The indefinite articles "a" and "an", as used herein in the specification and in the claims, unless clearly indicated to the contrary, should be understood to mean "at least one." The phrase "and/or," as used herein in the specification and in the claims, should be understood to mean " either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified unless clearly indicated to the contrary. Thus, as a non-limiting example, a reference to "A and/or B", when used in conjunction with open-ended language such as "comprising" can refer, in one embodiment, to A without B (optionally including elements other than B); in another embodiment, to B without A (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as " only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of", when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently, "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the acts of the method are recited.

## Claims

1. Carbenoxolone or 18β-Glycyrrhetinic acid, or a salt, or solvate thereof, for use in a method for treating a polyQ tract expansion disease or disorder, said method comprising administering to a subject having or suspected of having a polyQ tract expansion disease or disorder, or carrying a polyQ tract expansion mutation of a gene implicated in a

polyQ tract expansion disease or disorder, or expressing a polyQ tract-expanded polypeptide implicated in a polyQ tract expansion disease or disorder, an effective amount of said carbenoxolone or 18β-Glycyrrhetinic acid, or salt, or solvate thereof.

2. The carbenoxolone or 18β-Glycyrrhetinic acid, or salt, or solvate thereof of claim 1, for the use of claim 1, wherein the polyQ tract expansion disease or disorder is Huntington's Disease (HD), Dentatorubropallidoluysian atrophy (DRPLA), Spinobulbar muscular atrophy or Kennedy disease (SBMA), Spinocerebellar ataxia Type 1 (SCA1), Spinocerebellar ataxia Type 2 (SCA2), Spinocerebellar ataxia Type 3 or Machado-Joseph disease (SCA3), Spinocerebellar ataxia Type 6 (SCA6), Spinocerebellar ataxia Type 7 (SCA7), Spinocerebellar ataxia Type 17 (SCA17), Spinocerebellar ataxia Type 12 SCA12 (SCA12).

3. The carbenoxolone or 18β-Glycyrrhetinic acid, or salt, or solvate thereof of any of claims 1-2, for the use of any of claims 1-2, wherein the polyQ tract expansion disease or disorder is a polyQ tract expansion mutation in the HTT, ATN1, DRPLA, Androgen receptor on the X chromosome, ATXN1, ATXN2, ATXN3, ATXN12, CACNA1A, ATXN7, TBP, PPP2R2B, or SCA12 gene.

4. The carbenoxolone or 18β-Glycyrrhetinic acid, or salt, or solvate thereof of any of claims 1-3, for the use of any of claims 1-3, wherein the subject expresses an HTT (Huntingtin) protein comprising a polyQ tract of more than 35 Q residues, an ATN1 or DRPLA protein comprising a polyQ tract of more than 35 Q residues, an Androgen receptor protein comprising a polyQ tract of more than 36 Q residues, an ATXN1 protein comprising a polyQ tract of more than 35 Q residues, an ATXN2 protein comprising a polyQ tract of more than 32 Q residues, an ATXN3 protein comprising a polyQ tract of more than 40 Q residues, a CACNA1A protein comprising a polyQ tract of more than 18 Q residues, an ATXN7 protein comprising a polyQ tract of more than 17 Q residues, a TBP protein comprising a polyQ tract of more than 42 Q residues, or a PPP2R2B or SCA12 protein comprising a polyQ tract of more than 28 Q residues.

5. The carbenoxolone or 18β-Glycyrrhetinic acid, or salt, or solvate thereof of any of claims 1-4, for the use of any of claims 1-4, wherein the subject expresses a HTT (Huntingtin) protein comprising a polyQ tract of 35-140 Q residues, an ATN1 or DRPLA protein comprising a polyQ tract of 49-88 Q residues, an Androgen receptor protein comprising a polyQ tract of 38-62 Q residues, an ATXN1 protein comprising a polyQ tract of 49-88 Q residues, an ATXN2 protein comprising a polyQ tract of 33-77 Q residues, an ATXN3 protein comprising a polyQ tract of 55-86 Q residues, a CACNA1A protein comprising a polyQ tract of 21-30 Q residues, an ATXN7 protein comprising a polyQ tract of 38-120 Q residues, a TBP protein comprising a polyQ tract of 47-63 Q residues, or a PPP2R2B or SCA12 protein comprising a polyQ tract of 66-78 Q residues.

6. The carbenoxolone or 18β-Glycyrrhetinic acid, or salt, or solvate thereof of any of claims 1-5, for the use of any of claims 1-5, wherein the polyQ tract expansion disease or disorder is HD, preferably wherein the subject expresses a HTT (Huntingtin) protein comprising a polyQ tract of 35-140 Q residues.

7. The carbenoxolone or 18β-Glycyrrhetinic acid, or salt, or solvate thereof of any of claims 1-6, for the use of any of claims 1-6, wherein the carbenoxolone or 18β-Glycyrrhetinic acid is administered orally.

8. The carbenoxolone or 18β-Glycyrrhetinic acid, or salt, or solvate thereof of any of claims 1-7, for the use of any of claims 1-7, wherein the carbenoxolone or 18β-Glycyrrhetinic acid is administered at a dose of 0.1 mg/day to 10000 mg/day.

## Patentansprüche

1. Carbenoxolon oder 18β-Glycyrrhetinsäure oder ein Salz oder Solvat davon zur Verwendung in einem Verfahren zur Behandlung einer polyQ-Trakt-Expansionserkrankung oder -störung, wobei das Verfahren das Verabreichen an ein Subjekt, das eine polyQ-Trakt-Expansionserkrankung oder -störung hat oder bei dem dieser Verdacht besteht, oder das eine polyQ-Trakt-Expansionsmutation eines Gens trägt, das an einer polyQ-Trakt-Expansionserkrankung oder -störung beteiligt ist, oder ein polyQ-Traktexpandiertes Polypeptid exprimiert, das an einer polyQ-Trakt-Expansionserkrankung oder -störung beteiligt ist, einer wirksamen Menge des Carbenoxolons oder der 18β-Glycyrrhetinsäure oder eines Salzes oder Solvats davon umfasst.

2. Carbenoxolon oder 18β-Glycyrrhetinsäure oder Salz oder Solvat davon gemäß Anspruch 1 für die Verwendung

gemäß Anspruch 1, wobei die polyQ-Trakt-Expansionserkrankung oder -störung Huntington-Erkrankung (Huntington's Disease, HD), Dentatorubropallidoluysische Atrophie (DRPLA), Spinobulbäre Muskelatrophie oder Kennedy-Erkrankung (SBMA), Spinozerebelläre Ataxie Typ 1 (SCA1), Spinozerebelläre Ataxie Typ 2 (SCA2), Spinozerebelläre Ataxie Typ 3 oder Machado-Joseph-Erkrankung (SCA3), Spinozerebelläre Ataxie Typ 6 (SCA6), Spinozerebelläre Ataxie Typ 7 (SCA7), Spinozerebelläre Ataxie Typ 17 (SCA17), Spinozerebelläre Ataxie Typ 12 SCA12 (SCA12) ist.

**3.** Carbenoxolon oder 18β-Glycyrrhetinsäure oder Salz oder Solvat davon gemäß irgendeinem der Ansprüche 1-2 für die Verwendung gemäß irgendeinem der Ansprüche 1-2, wobei die polyQ-Trakt-Expansionserkrankung oder -störung eine polyQ-Trakt-Expansionsmutation in dem HTT-, ATN1-, DRPLA-, Androgen-Rezeptor-auf-dem-X-Chromosom-, ATXN1-, ATXN2-, ATXN3-, ATXN12-, CACNA1A-, ATXN7-, TBP-, PPP2R2B- oder SCA12-Gen ist.

**4.** Carbenoxolon oder 18β-Glycyrrhetinsäure oder Salz oder Solvat davon gemäß irgendeinem der Ansprüche 1-3 für die Verwendung gemäß irgendeinem der Ansprüche 1-3, wobei das Subjekt exprimiert: ein HTT- (Huntingtin-) Protein, welches einen polyQ-Trakt von mehr als 35 Q-Resten umfasst, ein ATN1- oder DRPLA-Protein, welches einen polyQ-Trakt von mehr als 35 Q-Resten umfasst, ein Androgen-Rezeptor-Protein, welches einen polyQ-Trakt von mehr als 36 Q-Resten umfasst, ein ATXN1-Protein, welches einen polyQ-Trakt von mehr als 35 Q-Resten umfasst, ein ATXN2-Protein, welches einen polyQ-Trakt von mehr als 32 Q-Resten umfasst, ein ATXN3-Protein, welches einen polyQ-Trakt von mehr als 40 Q-Resten umfasst, ein CACNA1A-Protein, welches einen polyQ-Trakt von mehr als 18 Q-Resten umfasst, ein ATXN7-Protein, welches einen polyQ-Trakt von mehr als 17 Q-Resten umfasst, ein TBP-Protein, welches einen polyQ-Trakt von mehr als 42 Q-Resten umfasst, oder ein PPP2R2B- oder SCA12-Protein, welches einen polyQ-Trakt von mehr als 28 Q-Resten umfasst.

**5.** Carbenoxolon oder 18β-Glycyrrhetinsäure oder Salz oder Solvat davon gemäß irgendeinem der Ansprüche 1-4 für die Verwendung gemäß irgendeinem der Ansprüche 1-4, wobei das Subjekt exprimiert: ein HTT- (Huntingtin-) Protein, welches einen polyQ-Trakt von 35-140 Q-Resten umfasst, ein ATN1- oder DRPLA Protein, welches einen polyQ-Trakt von 49-88 Q-Resten umfasst, ein Androgen-Rezeptor-Protein, welches einen polyQ-Trakt von 38-62 Q-Resten umfasst, ein ATXN1-Protein, welches einen polyQ-Trakt von 49-88 Q-Resten umfasst, ein ATXN2-Protein, welches einen polyQ-Trakt von 33-77 Q-Resten umfasst, ein ATXN3-Protein, welches einen polyQ-Trakt von 55-86 Q-Resten umfasst, ein CACNA1A-Protein, welches einen polyQ-Trakt von 21-30 Q-Resten umfasst, ein ATXN7-Protein, welches einen polyQ-Trakt von 38-120 Q-Resten umfasst, ein TBP-Protein, welches einen polyQ-Trakt von 47-63 Q-Resten umfasst, oder ein PPP2R2B- oder SCA12-Protein, welches einen polyQ-Trakt von 66-78 Q-Resten umfasst.

**6.** Carbenoxolon oder 18β-Glycyrrhetinsäure oder Salz oder Solvat davon gemäß irgendeinem der Ansprüche 1-5 für die Verwendung gemäß irgendeinem der Ansprüche 1-5, wobei die polyQ-Trakt-Expansionserkrankung oder -störung HD ist, bevorzugt wobei das Subjekt ein HTT- (Huntingtin-) Protein exprimiert, welches einen polyQ-Trakt von 35-140 Q-Resten umfasst.

**7.** Carbenoxolon oder 18β-Glycyrrhetinsäure oder Salz oder Solvat davon gemäß irgendeinem der Ansprüche 1-6 für die Verwendung gemäß irgendeinem der Ansprüche 1-6, wobei das Carbenoxolon oder die 18β-Glycyrrhetinsäure oral verabreicht wird.

**8.** Carbenoxolon oder 18β-Glycyrrhetinsäure oder Salz oder Solvat davon gemäß irgendeinem der Ansprüche 1-7 für die Verwendung gemäß irgendeinem der Ansprüche 1-7, wobei das Carbenoxolon oder die 18β-Glycyrrhetinsäure in einer Dosis von 0,1 mg/Tag bis 10000 mg/Tag verabreicht wird.

## Revendications

**1.** Carbénoxolone ou acide 18β-glycyrrhétinique, ou un sel ou solvate de ceux-ci, pour une utilisation dans un procédé de traitement d'une maladie ou d'un trouble par expansion du tractus polyQ, ledit procédé comprenant l'administration à un sujet ayant ou suspecté d'avoir une maladie ou un trouble par expansion du tractus polyQ, ou portant une mutation d'un gène d'expansion du tractus polyQ impliqué dans une maladie ou un trouble par expansion du tractus polyQ, ou exprimant un polypeptide à expansion du tractus polyQ impliqué dans une maladie ou un trouble par expansion du tractus polyQ, d'une quantité efficace de ladite carbénoxolone ou dudit acide 18β-glycyrrhétinique, ou d'un sel ou solvate de ceux-ci.

2. Carbénoxolone ou acide 18β-glycyrrhétinique, ou un sel ou solvate de ceux-ci selon la revendication 1, pour l'utilisation selon la revendication 1, dans laquelle/lequel la maladie ou le trouble par expansion du tractus polyQ est la maladie de Huntington (MH), l'atrophie dentato-rubro-pallido-luysienne (ADRPL), l'atrophie musculaire spino-bulbaire ou maladie de Kennedy (AMSC), l'ataxie spinocérébelleuse de type 1 (ASC1), l'ataxie spinocérébelleuse de type 2 (ASC2), l'ataxie spinocérébelleuse de type 3 ou maladie de Machado-Joseph (ASC3), l'ataxie spinocérébelleuse de type 6 (ASC6), l'ataxie spinocérébelleuse de type 7 (ASC7), l'ataxie spinocérébelleuse de type 17 (ASC17) et l'ataxie spinocérébelleuse de type 12 (ASC12.)

3. Carbénoxolone ou acide 18β-glycyrrhétinique, ou un sel ou solvate de ceux-ci selon l'une quelconque des revendications 1 à 2, pour l'utilisation selon l'une quelconque des revendications 1 à 2, dans laquelle/lequel la maladie ou le trouble par expansion du tractus polyQ est une mutation par expansion du tractus polyQ dans le gène HTT, ATN1, DRPLA, récepteur androgénique sur le chromosome X, ATXN1, ATXN2, ATXN3, ATXN12, CACNA1A, ATXN7, TBP, PPP2R2B, ou SCA12.

4. Carbénoxolone ou acide 18β-glycyrrhétinique, ou un sel ou solvate de ceux-ci selon l'une quelconque des revendications 1 à 3, pour l'utilisation selon l'une quelconque des revendications 1 à 3, dans laquelle/lequel le sujet exprime une protéine HTT (huntingtine) comprenant un tractus polyQ de plus de 35 résidus Q, une protéine ATN1 ou DRPLA comprenant un tractus polyQ de plus de 35 résidus Q, une protéine de récepteur androgénique comprenant un tractus polyQ de plus de 36 résidus Q, une protéine ATXN1 comprenant un tractus polyQ de plus de 35 résidus Q, une protéine ATXN2 comprenant un tractus polyQ de plus de 32 résidus Q, une protéine ATXN3 comprenant un tractus polyQ de plus de 40 résidus Q, une protéine CACNA1A comprenant un tractus polyQ de plus de 18 résidus Q, une protéine ATXN7 comprenant un tractus polyQ de plus de 17 résidus Q, une protéine TBP comprenant un tractus polyQ de plus de 42 résidus Q, ou une protéine PPP2R2B ou SCA12 comprenant un tractus polyQ de plus de 28 résidus Q.

5. Carbénoxolone ou acide 18β-glycyrrhétinique, ou un sel ou solvate de ceux-ci selon l'une quelconque des revendications 1 à 4, pour l'utilisation selon l'une quelconque des revendications 1 à 4, dans laquelle/lequel le sujet exprime une protéine HTT (huntingtine) comprenant un tractus polyQ de 35 à 140 résidus Q, une protéine ATN1 ou DRPLA comprenant un tractus polyQ de 49 à 88 résidus Q, une protéine de récepteur androgénique comprenant un tractus polyQ de 38 à 62 résidus Q, une protéine ATXN1 comprenant un tractus polyQ de 49 à 88 résidus Q, une protéine ATXN2 comprenant un tractus polyQ de 33 à 77 résidus Q, une protéine ATXN3 comprenant un tractus polyQ de 55 à 86 résidus Q, une protéine CACNA1A comprenant un tractus polyQ de 21 à 30 résidus Q, une protéine ATXN7 comprenant un tractus polyQ de 38 à 120 résidus Q, une protéine TBP comprenant un tractus polyQ de 47 à 63 résidus Q, ou une protéine PPP2R2B ou SCA12 comprenant un tractus polyQ de 66 à 78 résidus Q.

6. Carbénoxolone ou acide 18β-glycyrrhétinique, ou un sel ou solvate de ceux-ci selon l'une quelconque des revendications 1 à 5, pour l'utilisation selon l'une quelconque des revendications 1 à 5, dans laquelle/lequel la maladie ou le trouble par expansion du tractus polyQ est la MH, de préférence dans laquelle/lequel le sujet exprime une protéine HTT (huntingtine) comprenant un tractus polyQ de 35 à 140 résidus Q.

7. Carbénoxolone ou acide 18β-glycyrrhétinique, ou un sel ou solvate de ceux-ci selon l'une quelconque des revendications 1 à 6, pour l'utilisation selon l'une quelconque des revendications 1 à 6, dans laquelle/lequel la carbénoxolone ou l'acide 18β-glycyrrhétinique est administré(e) par voie orale.

8. Carbénoxolone ou acide 18β-glycyrrhétinique, ou un sel ou solvate de ceux-ci selon l'une quelconque des revendications 1 à 7, pour l'utilisation selon l'une quelconque des revendications 1 à 7, dans laquelle/lequel la carbénoxolone ou l'acide 18β-glycyrrhétinique est administré(e) à une dose de 0,1 mg/jour à 10 000 mg/jour.

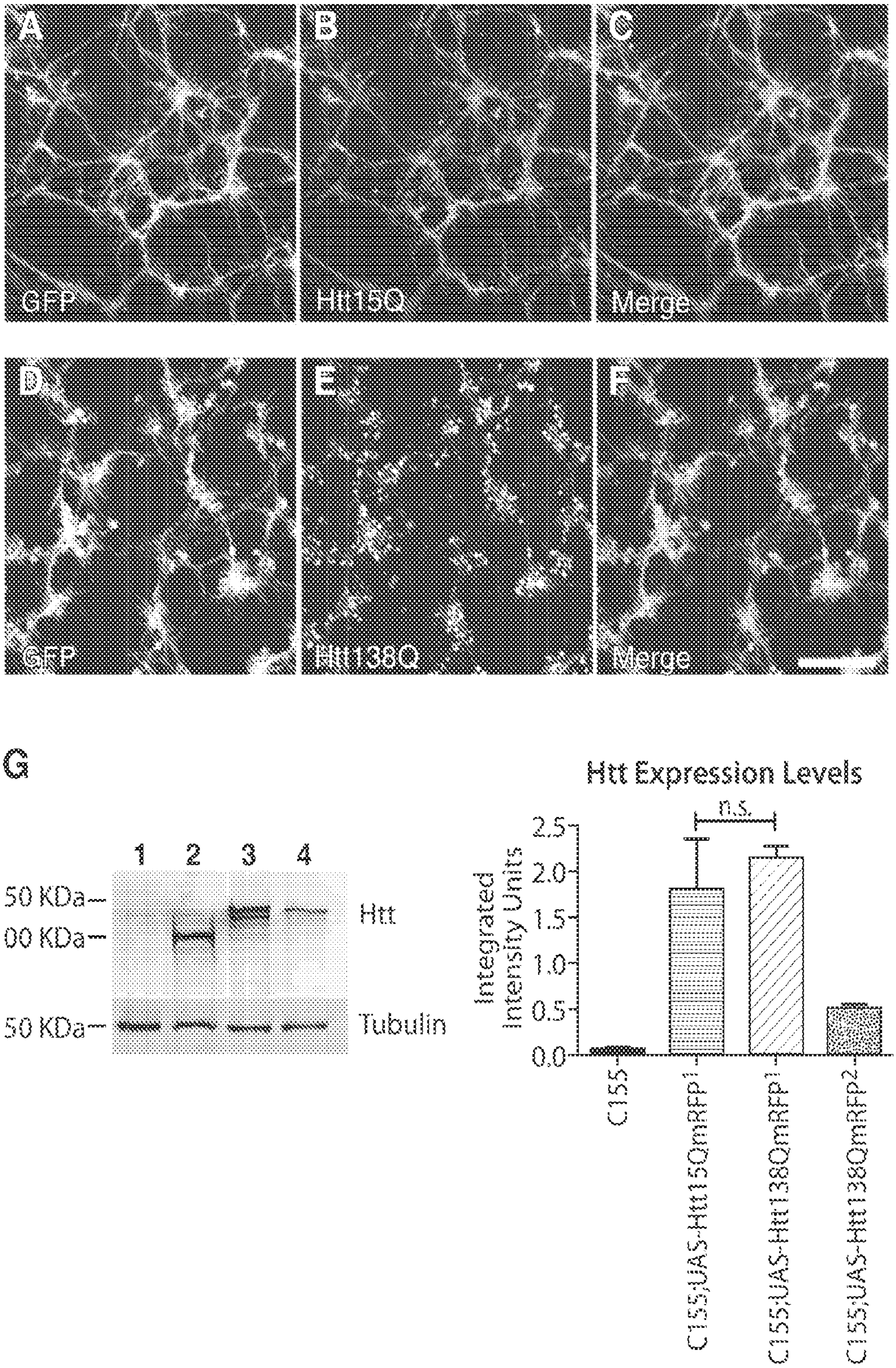
A
B
C
GFP
Htt15Q
Merge
D
E
F
GFP
Htt138Q
Merge
G
1 2 3 4
150 KDa
100 KDa
50 KDa
Htt
Tubulin
Htt Expression Levels
n.s.
Integrated Intensity Units
2.5
2.0
1.5
1.0
0.5
0.0
C155
C155;UAS-Htt15QmRFP1
C155;UAS-Htt138QmRFP1
C155;UAS-Htt138QmRFP2

Fig. 1

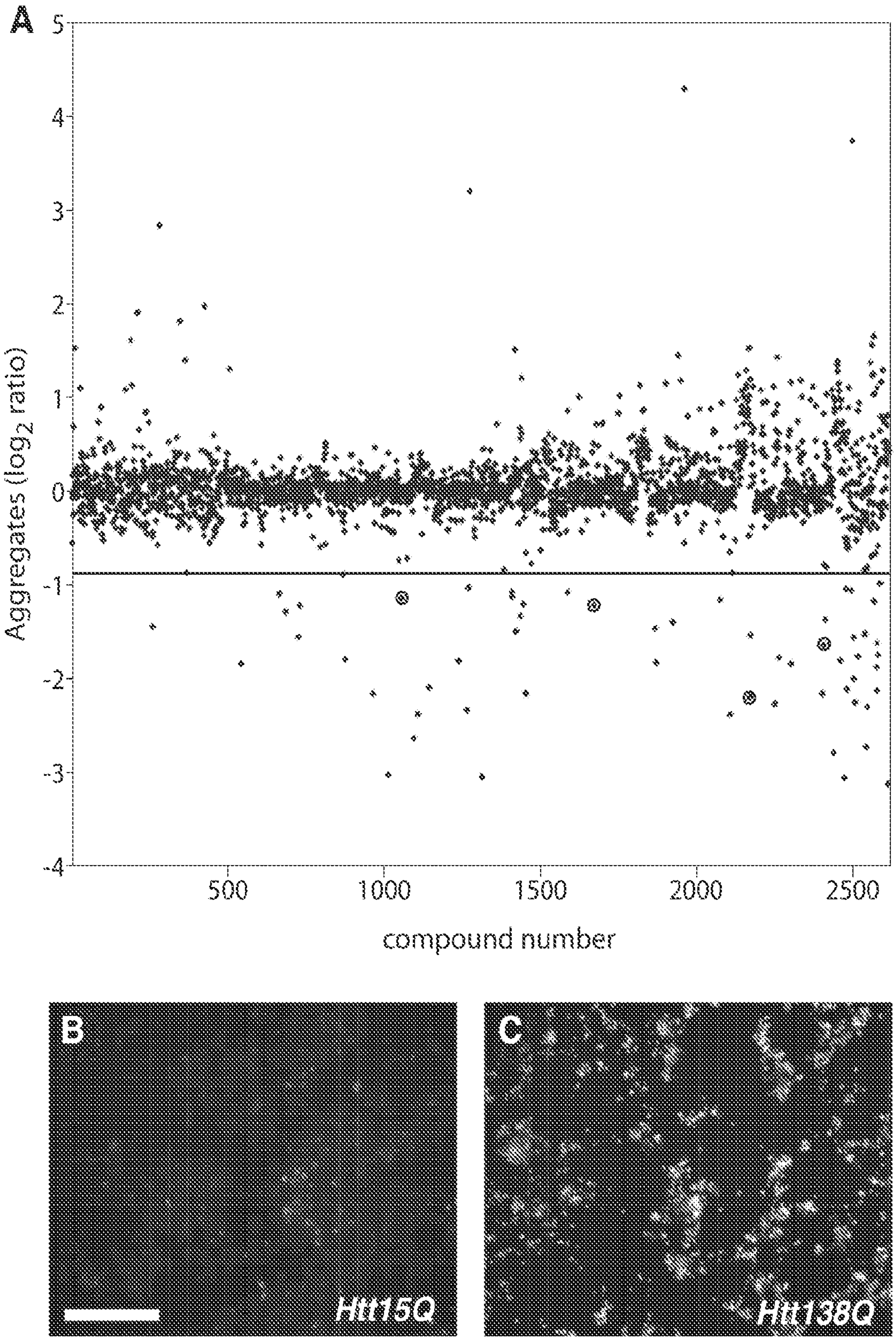
A
Aggregates (log2 ratio)
5
4
3
2
1
0
-1
-2
-3
-4
500
1000
1500
2000
2500
compound number
B
Htt15Q
C
Htt138Q

Fig. 2

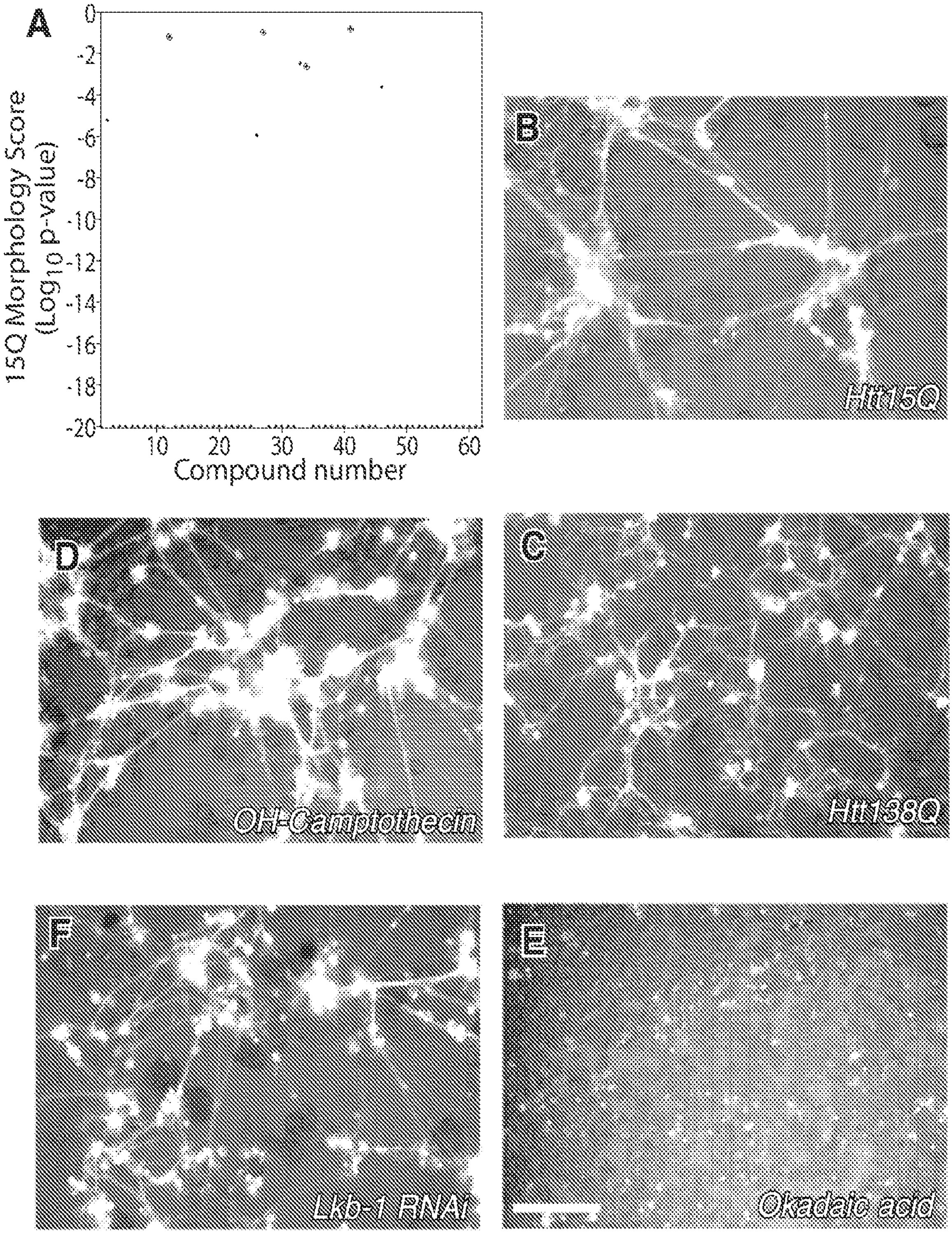
A
15Q Morphology Score
(Log10 p-value)
0
-2
-4
-6
-8
-10
-12
-14
-16
-18
-20
10
20
30
40
50
60
Compound number
B
Htt15Q
D
OH-Camptothecin
C
Htt138Q
F
Lkb-1 RNAi
E
Okadaic acid

Fig. 3

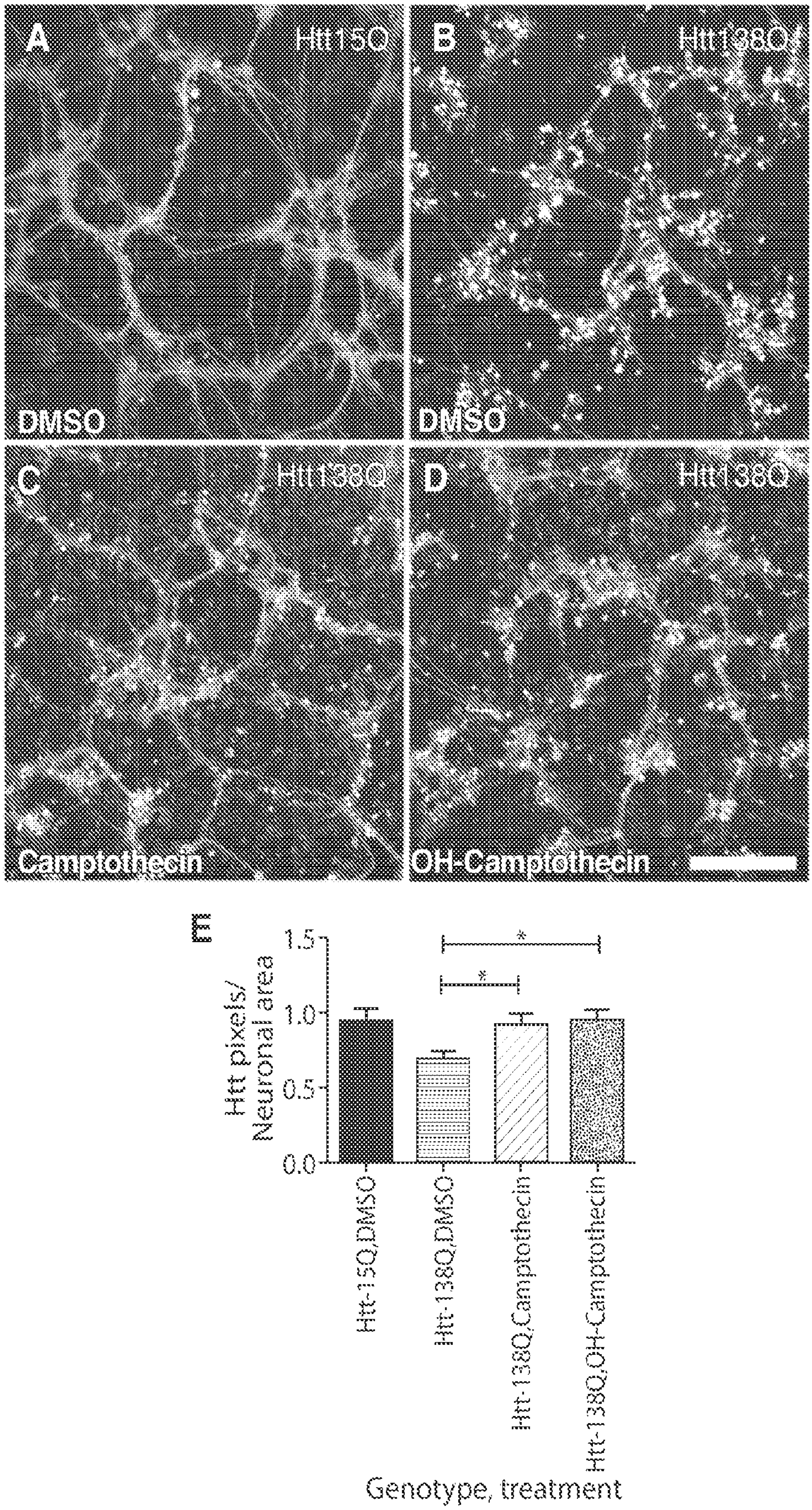
A
Htt15Q
DMSO
B
Htt138Q
DMSO
C
Htt138Q
Camptothecin
D
Htt138Q
OH-Camptothecin
E
Htt pixels/
Neuronal area
1.5
1.0
0.5
0.0
*
*
Htt-15Q,DMSO
Htt-138Q,DMSO
Htt-138Q,Camptothecin
Htt-138Q,OH-Camptothecin
Genotype, treatment

Fig. 4

A

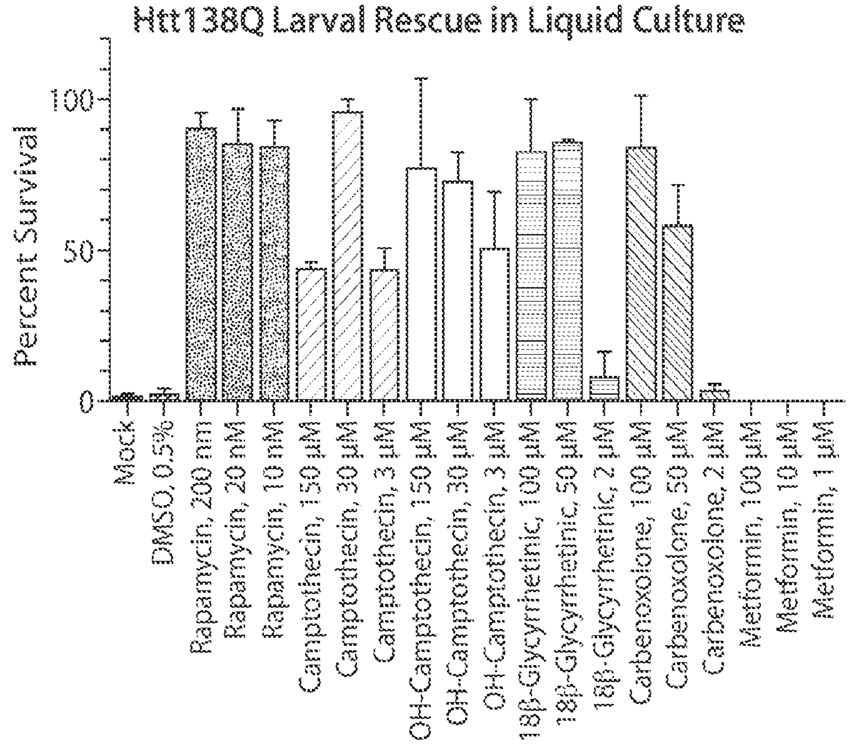


B

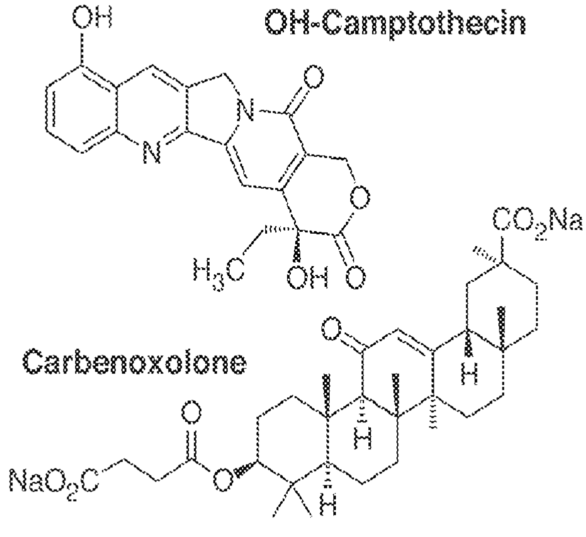

OH-Camptothecin

Carbenoxolone

C

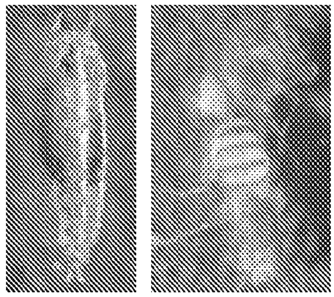

Fig. 5-1

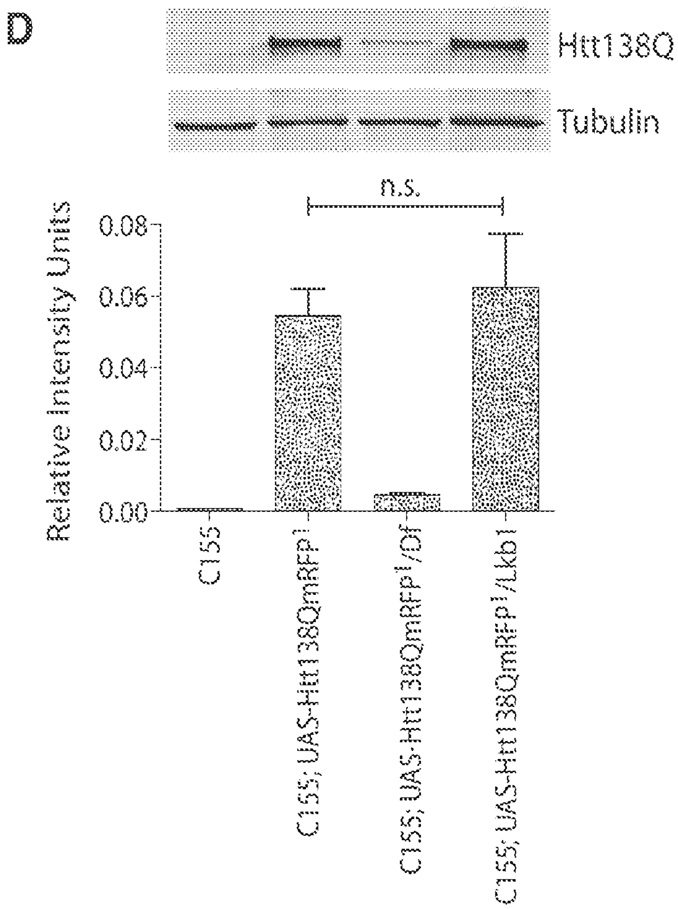
D
Htt138Q
Tubulin
n.s.
Relative Intensity Units
0.08
0.06
0.04
0.02
0.00
C155
C155; UAS-Htt138QmRFP1
C155; UAS-Htt138QmRFP1/Df
C155; UAS-Htt138QmRFP1/Lkb1

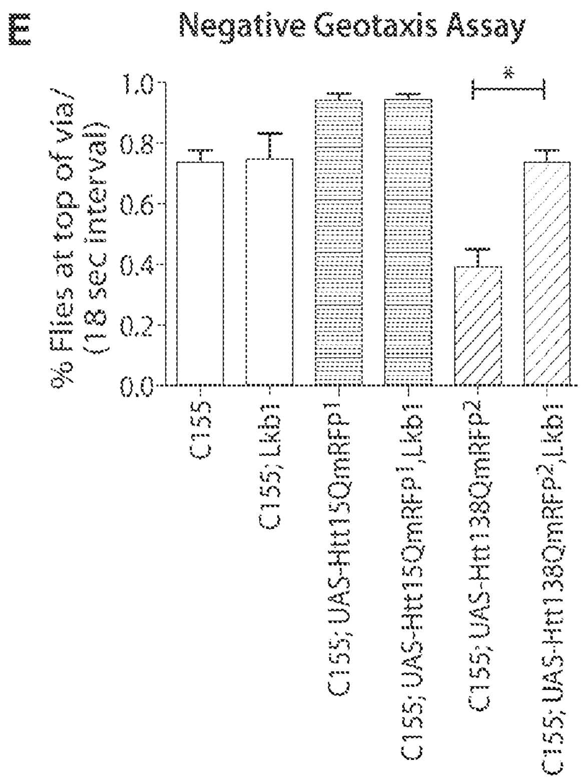
E
Negative Geotaxis Assay
*
% Flies at top of via/ (18 sec interval)
1.0
0.8
0.6
0.4
0.2
0.0
C155
C155; Lkb1
C155; UAS-Htt15QmRFP1
C155; UAS-Htt15QmRFP1,Lkb1
C155; UAS-Htt138QmRFP2
C155; UAS-Htt138QmRFP2,Lkb1

Fig. 5-2

**C2-8**

N-(4-bromophenyl)-3-[[(4-bromophenyl)amino]sulfonyl]benzamide

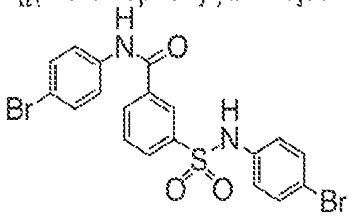

**Juglone**

5-hydroxy-1,4-naphthalenedione

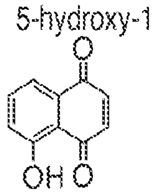

**Camptothecin**

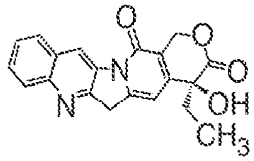

**Metformin**

[1,1-Dimethylbiguanide - hydrochloride]

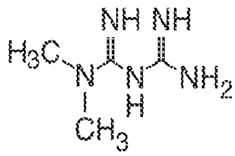

**10-Hydroxycamptothecin**

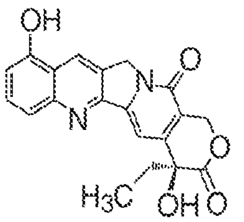

**Radicicol**

[Monorden; 1aS-(1aR*,2Z,4E,14*,15aR*)]-8-Chloro-1a,14,15,15a-tetrahydro-9,11-dihydroxy-14-methyl-6Hoxireno[e][2] benzoxacyclotetradecin-6, 12(7H)-dione]

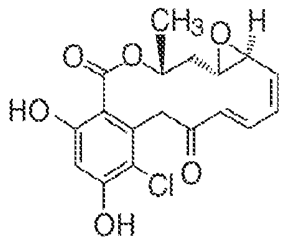

**18β-Glycyrrhetinic acid**

3beta-Hydroxy-11-oxo-18beta,20beta-olean-12-en-29-oic acid

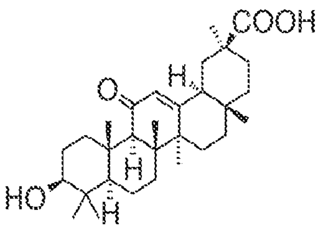

**Rapamycin**

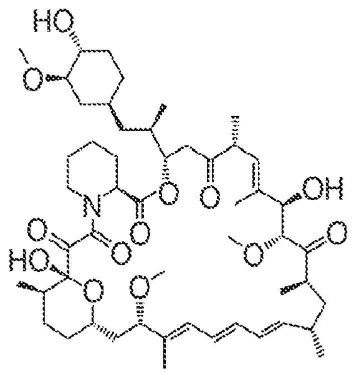

**GW 5074**

3-(3,5-Dibromo-4-hydroxy-benzylidene)-5-iodo-1,3-dihydr o-indol-2-one

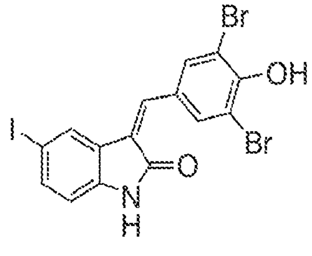

Fig. 6A

**Ethacrynic acid**

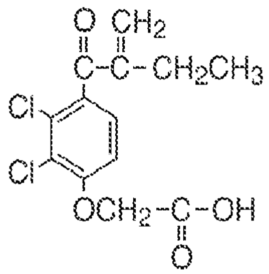

**Proscillaridin A**

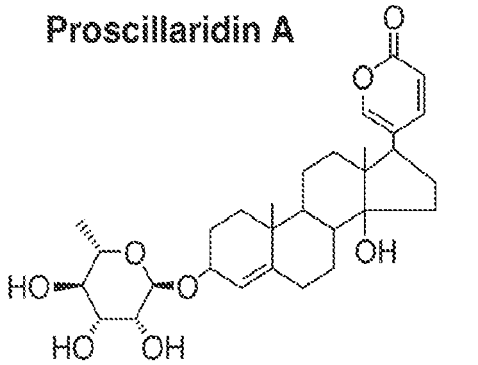

**Ouabain**

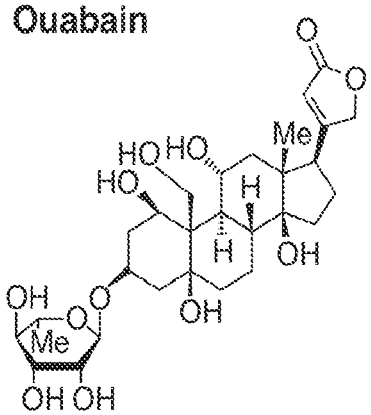

**Etoposide**

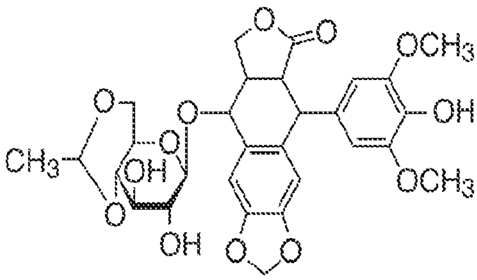

Fig. 6B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61419157 B
- US 61476040 B
- EP 11845326 A


### Non-patent literature cited in the description


- **JAMES CHAMPOUX.** DNA Topoisomerases: Structure, Function, and Mechanism. *Annu. Rev. Biochem.,* 2001, vol. 70, 369-413
- **KIEBURTZ K.** Unified Huntington's Disease Rating Scale. *UHDRS, Huntington Study Group*
- Unified Huntington's Disease Rating Scale: Reliability and Consistency. Mov Dis. 1996, vol. 11, 136-142
- Huntington's disease. *Brain,* vol. 119, 2085-2095
- **AZIZ NA ; PIJL H ; FRÖLICH M ; VAN DER GRAAF AW ; ROELFSEMA F ; ROOS RA.** Increased hypothalamic -pituitary-adrenal axis activity in Huntington's disease. *J Clin Endocrinol Metab,* 2009, vol. 94, 1223-1228
- **HAYDEN MR ; MARTIN WR ; STOESSL AJ ; CLARK C ; HOLLENBERG S ; ADAM MJ ; AMMANN W ; HARROP R ; ROGERS J ; RUTH T et al.** Positron emission tomography in the early diagnosis of Huntington's disease. *Neurology,* 1986, vol. 36, 888-894
- **HEUSER IJ ; CHASE TN ; MOURADIAN MM.** The limbic-hypothalamic-pituitary-adrenal axis in Huntington's disease. *Biol Psychiatry,* 1991, vol. 30, 943-952
- **HULT S ; SOYLU R ; BJÖRKLUND T ; BELGARDT BF ; MAUER J ; BRÜNING JC ; KIRIK D ; PETERSÉN Å.** Mutant huntingtin causes metabolic imbalance by disruption of hypothalamic neurocircuits. *Cell Metab,* 2011, vol. 13, 428-39
- **KUHL DE ; PHELPS ME ; MARKHAM CH ; METTER EJ ; RIEGE WH ; WINTER J.** Cerebral metabolism and atrophy in Huntington's disease determined by 18FDG and computed tomographic scan. *Ann Neurol,* 1982, vol. 12, 425-434
- **KUWERT T ; LANGE HW ; LANGEN KJ ; HERZOG H ; AULICH A ; FEINENDEGEN LE.** Cortical and subcortical glucose consumption measured by PET in patients with Huntington's disease. *Brain,* October 1990, vol. 113, 1405-1423
- **LUPIEN SJ ; DE LEON M ; DE SANTI S ; CONVIT A ; TARSHISH C ; NAIR NP ; THAKUR M ; MCEWEN BS ; HAUGER RL ; MEANEY MJ.** Cortisol levels during human aging predict hippocampal atrophy and memory deficits. *Nat Neurosci,* 1998, vol. 1, 69-73
- **MCEWEN BS.** Stress, sex, and neural adaptation to a changing environment: mechanisms of neuronal remodeling. *Ann NY Acad Sci,* September 2010, vol. 1204
- **SALEH N ; MOUTEREAU S ; DURR A ; KRYSTKOWIAK P ; AZULAY JP ; TRANCHANT C ; BROUSSOLLE E ; MORIN F ; BACHOUD-LÉVI AC ; MAISON P.** Neuroendocrine disturbances in Huntington's disease. *PLoS One,* 2009, vol. 4 (3), e4962
- **SCHULTE J ; SEPP KJ ; WU C ; HONG P ; LITTLETON JT.** High-content chemical and RNAi screens for suppressors of neurotoxicity in a Huntington's disease model. *PLoS One,* 2011, vol. 6 (8), e23841
- **VAN DUIJN E ; SELIS MA ; GILTAY EJ ; ZITMAN FG ; ROOS RA ; VAN PELT H ; VAN DER MAST RC.** Hypothalamic-pituitary-adrenal axis functioning in Huntington's disease mutation carriers compared with mutation-negative first-degree controls. *Brain Res Bull,* 2010, vol. 83, 232-237
- **YAU JL ; MCNAIR KM ; NOBLE J ; BROWNSTEIN D ; HIBBERND C ; MORTON N ; MULLINS JJ ; MORRIS RG ; COBB S ; SECKL JR.** Enhanced hippocampal long-term potentiation and spatial learning in aged 11beta-hydroxysteroid dehydrogenase type 1 knock-out mice. *J Neurosci,* 2007, vol. 27, 10487-10496
- **YOUNG AB ; PENNEY JB ; STAROSTA-RUBINSTEIN S ; MARKEL DS ; BERENT S ; GIORDANI B ; EHRENKAUFER R ; JEWETT D ; HICHWA R.** PET scan investigations of Huntington's disease: cerebral metabolic correlates of neurological features and functional decline. *Ann Neurol,* 1986, vol. 20, 296-303

- **GROTE HE ; BULL ND ; HOWARD ML ; VAN DELLEN A ; BLAKEMORE C ; BARTLETT PF ; HANNAN AJ.** Cognitive disorders and neurogenesis deficits in Huntington's disease mice are rescued by fluoxetine. *Eur J Neurosci,* 2005, vol. 22, 2081-2088
- **SPARGO E ; EVERALL IP ; LANTOS PL.** Neuronal loss in the hippocampus in Huntington's disease: a comparison with HIV infection. *J Neurol Neurosurg Psychiatry,* 1993, vol. 56, 487-491
- **SCHUBERT MI ; KALISCH R ; SOTIROPOULOS I ; CATANIA C ; SOUSA N ; ALMEIDA OF ; AUER DP.** Effects of altered corticosteroid milieu on rat hippocampal neurochemistry and structure - an in vivo magnetic resonance spectroscopy and imaging study. *J Psychiatr Res,* 2008, vol. 42, 902-912
- **BUTTERS N ; SAX D ; MONTGOMERY K ; TARLOW S.** Comparison of the neuropsychological deficits associated with early and advanced Huntington's disease. *Arch Neurol,* 1978, vol. 35, 585-589
- **DUBROVSKY B.** Effects of adrenal cortex hormones on limbic structures: some experimental and clinical correlations related to depression. *J Psychiatry Neurosci,* 1993, vol. 18, 4-16
- **VAN DUIJN E ; KINGMA EM ; VAN DER MAST RC.** Psychopathology in verified Huntington's disease gene carriers. *J Neuropsychiatry Clin Neurosci,* 2007, vol. 19, 441-448
- **AMULF I ; NIELSEN J ; LOHMANN E ; SCHIEFER J ; WILD E ; JENNUM P ; KONOFAL E ; WALKER M ; OUDIETTE D ; TABRIZI S.** Rapid eye movement sleep disturbances in Huntington's disease. *Arch Neurol,* 2008, vol. 65, 482-488
- **CARROLL BJ ; CASSIDY F ; NAFTOLOWITZ D ; TATHAM NE ; WILSON WH ; IRANMANESH A ; LIU PY ; VELDHUIS JD.** Pathophysiology of hypercortisolism in depression. *Acta Psychiatr Scand Suppl,* 2007
- Remington's Pharmaceutical Sciences. 1990
- Remington's Pharmaceutical Sciences. Mack Publishing Co,
- **LEE W-CM ; YOSHIHARA M.** Littleton JT (2004) Cytoplasmic aggregates trap polyglutamine-containing proteins and block axonal transport in a Drosophila model of Huntington's disease. *Proc Natl Acad Sci USA.,* 3224-3229
- **ROOS RA.** Huntington's disease: a clinical review. *Orphanet J Rare Dis,* 2010, vol. 5, 40
- **DIFIGLIA M ; SAPP E ; CHASE K ; DAVIES S ; BATES G et al.** Aggregation of Huntingtin in neuronal intranuclear inclusions and dystrophic neurites in brain. *Science,* 1997, 1990
- **WU C ; SCHULTE J ; SEPP KJ ; LITTLETON JT ; HONG P.** Automatic robust neurite detection and morphological analysis of neuronal cell cultures in high-content screening. *Neuroinformatics,* 2010, vol. 8, 83-100
- **JENNE DE ; REIMANN H ; NEZU J ; FRIEDEL W ; LOFF S et al.** Peutz-Jeghers syndrome is caused by mutations in a novel serine threonine kinase. *Nat Genet,* 1998, vol. 18, 38-43
- **VAN VEELEN W ; KORSSE SE ; VAN DE LAAR L ; PEPPELENBOSCH MP.** *The long and winding road to rational treatment of cancer associated with LKB1/AMPK/TSC/mTORC1 signaling,* 2011
- **LEE JH ; KOH H ; KIM M ; PARK J ; LEE SY et al.** JNK pathway mediates apoptotic cell death induced by tumor suppressor LKB1 in Drosophila. *Cell Death Differ,* 2006, vol. 13, 1110-1122
- **RAVIKUMAR B ; VACHER C ; BERGER Z ; DAVIES J ; LUO S et al.** Inhibition of mTOR induces autophagy and reduces toxicity of polyglutamine expansions in fly and mouse models of Huntington disease. *Nat Genet.,* 2004, 585-595
- **SARKAR S ; PERLSTEIN EO ; IMARISIO S ; PINEAU S ; CORDENIER A et al.** Small molecules enhance autophagy and reduce toxicity in Huntington's disease models. *Nat Chem Biol,* 2007, vol. 3, 331-338
- **FLEMING A ; NODA T ; YOSHIMORI T ; RUBINSZTEIN DC.** Chemical modulators of autophagy as biological probes and potential therapeutics. *Nat,* 2011, vol. 7, 9-17
- **YAMAMOTO A ; CREMONA M ; ROTHMAN J.** Autophagy-mediated clearance of Huntingtin aggregates triggered by the insulin-signaling pathway. *Journal of Cell Biology,* 2006, 719
- **DAVID DC ; OLLIKAINEN N ; TRINIDAD JC ; CARY MP ; BURLINGAME AL et al.** Widespread protein aggregation as an inherent part of aging in C. elegans. *PLoS,* 2010, vol. 8, e1000450
- **JU TC ; CHEN HM ; LIN JT ; CHANG CP ; CHANG WC et al.** Nuclear translocation of AMPK-{alpha} 1 potentiates striatal neurodegeneration in Huntington's disease. *J Cell Biol,* 2011, vol. 194, 209-227
- **CHIN PC ; LIU L ; MORRISON BE ; SIDDIQ A ; RATAN RR et al.** The c-Raf inhibitor GW5074 provides neuroprotection in vitro and in an animal model of neurodegeneration through a MEK-ERK and Akt-independent mechanism. *J Neurochem,* 2004, vol. 90, 595-608
- **KAO TC ; SHYU MH ; YEN GC.** Neuroprotective effects of glycyrrhizic acid and 18beta-glycyrrhetinic acid in PC 12 cells via modulation of the PI3K/Akt pathway. *J Agric Food Chem,* 2009, vol. 57, 754-761
- **JUSZCZAK GR ; SWIERGIEL AH.** Properties of gap junction blockers and their behavioural, cognitive and electrophysiological effects: animal and human studies. *Prog Neuropsychopharmacol Biol Psychiatry,* 2009, vol. 33, 181-198

- **SHIN JY ; FANG ZH ; YU ZX ; WANG CE ; LI SH et al.** Expression of mutant Huntingtin in glial cells contributes to neuronal excitotoxicity. *J Cell Biol,* 2005, vol. 171, 1001-1012
- **TAMURA T ; SONE M ; YAMASHITA M ; WANKER EE ; OKAZAWA H.** Glial cell lineage expression of mutant ataxin-1 and Huntingtin induces developmental and late-onset neuronal pathologies in Drosophila models. *PLoS One,* 2009, vol. 4, e4262
- **BRADFORD J ; SHIN JY ; ROBERTS M ; WANG CE ; SHENG G et al.** *Mutant Huntingtin in glial cells exacerbates neurological symptoms of Huntington disease mice. J,* 2010, vol. 285, 10653-10661
- **KRETZSCHMAR D ; TSCHAPE J ; BETTENCOURT DA CRUZ A ; ASAN E ; POECK B et al.** Glial and neuronal expression of polyglutamine proteins induce behavioral changes and aggregate formation in Drosophila. *Glia,* 2005, vol. 49, 59-72
- **SAPP E ; KEGEL KB ; ARONIN N ; HASHIKAWA T ; UCHIYAMA Y et al.** Early and progressive accumulation of reactive microglia in the Huntington disease brain. *J Neuropathol Exp Neurol,* 2001, vol. 60, 161-172
- **LIN JH ; WEIGEL H ; COTRINA ML ; LIU S ; BUENO E et al.** Gap-junction-mediated propagation and amplification of cell injury. *Nat Neurosci,* 1998, vol. 1, 494-500
- **TAKEUCHI H ; MIZOGUCHI H ; DOI Y ; JIN S ; NODA M et al.** Blockade of gap junction hemichannel suppresses disease progression in mouse models of amyotrophic lateral sclerosis and Alzheimer's disease. *PLoS One,* 2011, vol. 6, e21108
- **DIFIGLIA M ; SENA-ESTEVES M ; CHASE K ; SAPP E ; PFISTER E et al.** Therapeutic silencing of mutant Huntingtin with siRNA attenuates striatal and cortical neuropathology and behavioral deficits. *Proc Natl Acad Sci U S A,* 2007, vol. 104, 17204-17209
- **LANG-ROLLIN IC ; RIDEOUT HJ ; NOTICEWALA M ; STEFANIS L.** Mechanisms of caspase-independent neuronal death: energy depletion and free radical generation. *J Neurosci,* 2003, vol. 23, 11015-11025
- **KLUZA J ; MARCHETTI P ; GALLEGO MA ; LANCEL S ; FOURNIER C et al.** Mitochondrial proliferation during apoptosis induced by anticancer agents: effects of doxorubicin and mitoxantrone on cancer and cardiac cells. *Oncogene,* 2004, vol. 23, 7018-7030
- **REIPERT S ; BERRY J ; HUGHES MF ; HICKMAN JA ; ALLEN TD.** Changes of mitochondrial mass in the hemopoietic stem cell line FDCP-mix after treatment with etoposide: a correlative study by multiparameter flow cytometry and confocal and electron microscopy. *Exp Cell Res,* 1995, vol. 221, 281-288
- **FU X ; WAN S ; LYU YL ; LIU LF ; QI H.** Etoposide induces ATM-dependent mitochondrial biogenesis through AMPK activation. *PLoS One,* 2008, vol. 3, e2009
- **THOMAS CJ ; RAHIER NJ ; HECHT SM.** Camptothecin: current perspectives. *Bioorg Med Chem,* 2004, vol. 12, 1585-1604
- **SAPP E ; PENNEY J ; YOUNG A ; ARONIN N ; VONSATTEL JP et al.** Axonal transport of N-terminal Huntingtin suggests early pathology of corticostriatal projections in Huntington disease. *J Neuropathol Exp Neurol,* 1999, vol. 58, 165-173
- **GUNAWARDENA S ; HER LS ; BRUSCH RG ; LAYMON RA ; NIESMAN IR et al.** Disruption of axonal transport by loss of Huntingtin or expression of pathogenic polyQ proteins in Drosophila. *Neuron,* 2003, vol. 40, 25-40
- **TRUSHINA E ; DYER RB ; BADGER JD, 2ND ; URE D ; EIDE L et al.** Mutant Huntingtin impairs axonal trafficking in mammalian neurons in vivo and in vitro. *Mol Cell Biol,* 2004, vol. 24, 8195-8209
- **ARRASATE M ; MITRA S ; SCHWEITZER ES ; SEGAL MR ; FINKBEINER S.** Inclusion body formation reduces levels of mutant Huntingtin and the risk of neuronal death. *Nature,* 2004, vol. 431, 805-810
- **AMBEGAOKAR SS ; ROY B ; JACKSON GR.** Neurodegenerative models in Drosophila: polyglutamine disorders, Parkinson disease, and amyotrophic lateral sclerosis. *Neurobiol,* 2010, vol. 40, 29-39
- **LIM KL.** Non-mammalian animal models of Parkinson's disease for drug discovery. *Expert Opin Drug Discov,* 2010, vol. 5, 165-176
- **O'KANE CJ.** *Drosophila as a Model Organism for the Study of Neuropsychiatric Disorders,* 2011
- **BILEN J ; BONINI NM.** Drosophila as a model for human neurodegenerative disease. *Annu Rev Genet,* 2005, vol. 39, 153-171
- **AGRAWAL N ; PALLOS J ; SLEPKO N ; APOSTOL BL ; BODAI L et al.** Identification of combinatorial drug regimens for treatment of Huntington's disease using Drosophila. *Proc Natl Acad Sci U S A,* 2005, vol. 102, 3777-3781
- **STEFFAN JS ; BODAI L ; PALLOS J ; POELMAN M ; MCCAMPBELL A et al.** Histone deacetylase inhibitors arrest polyglutamine-dependent neurodegeneration in Drosophila. *Nature,* 2001, vol. 413, 739-743
- **AULUCK PK ; BONINI NM.** Pharmacological prevention of Parkinson disease in Drosophila. *Nat Med.,* 2002, 1185-1186
- **MIN KT ; BENZER S.** Preventing neurodegeneration in the Drosophila mutant bubblegum. *Science,* 1999, vol. 284, 1985-1988

- **BANOVIC D ; KHORRAMSHAHI O ; OWALD D ; WICHMANN C ; RIEDT T et al.** Drosophila neuroligin 1 promotes growth and postsynaptic differentiation at glutamatergic neuromuscular junctions. *Neuron,* 2010, vol. 66, 724-738
- **WILLIAMS R.** Development: Neuroligin knockouts: form but no function. *Nature Reviews Neuroscience,* 2006, vol. 7, 831
- **CROOK ZR ; HOUSMAN D.** Huntington's disease: can mice lead the way to treatment?. *Neuron,* 2011, vol. 69, 423-35
- **LIONE LA ; CARTER RJ ; HUNT MJ ; BATES GP ; MORTON AJ ; DUNNETT SB.** Selective Discrimination Learning Impairments in Mice Expressing the Human Huntington's Disease Mutation. *J. Neuroscience.,* 1999, vol. 19, 10428-10437
- The Unified Huntington's Disease Rating Scale: Reliability and Consistency. *Mov Dis,* 1996, vol. 11, 136-142
- **BRAND AH ; PERRIMON N.** Targeted gene expression as a means of altering cell fates and generating dominant phenotypes. *Development,* 1993, vol. 118, 401-415
- **CHIN PC ; LIU L ; MORRISON BE ; SIDDIQ A ; RATAN RR ; BOTTIGLIERI T ; D'MELLO SR.** The c-Raf inhibitor GW5074 provides neuroprotection in vitro and in an animal model of neurodegeneration through a MEK-ERK and Akt-independent mechanism. *J Neurochem,* 2004, vol. 90, 595-608
- **DIFIGLIA M ; SAPP E ; CHASE K ; DAVIES S ; BATES G ; VONSATTEL J ; ARONIN N.** Aggregation of Huntingtin in neuronal intranuclear inclusions and dystrophic neurites in brain. *Science,* 26 September 1997, vol. 277 (5334), 1990-3
- **FISHER, R.A.** Statistical Methods for Research Workers. Oliver and Boyd, 1932
- **GRAHAM R ; DENG Y ; SLOW E ; HAIGH B ; BISSADA N ; LU G ; PEARSON J ; SHEHADEH J ; BERTRAM L ; MURPHY Z.** Cleavage at the caspase-6 site is required for neuronal dysfunction and degeneration due to mutant Huntingtin. *Cell,* 16 June 2006, vol. 125 (6), 1179-91
- **HARDIE D.** Neither LKB 1 nor AMPK are the direct targets of metformin. *Gastroenterology,* September 2006, vol. 131 (3), 973
- **HAY DG ; SATHASIVAM K ; TOBABEN S ; STAHL B ; MARBER M ; MESTRIL R ; MAHAL A ; SMITH DL ; WOODMAN B ; BATES GP.** Progressive decrease in chaperone protein levels in a mouse model of Huntington's disease and induction of stress proteins as a therapeutic approach. *Hum Mol Genet,* 2004, vol. 13, 1389-1405
- **HERTZBERG R ; BUSBY R ; CARANFA M ; HOLDEN K ; JOHNSON R ; HECHT S ; KINGSBURY W.** Irreversible trapping of the DNA-topoisomerase I covalent complex. Affinity labeling of the camptothecin binding site. *Journal of Biological Chemistry,* 05 November 1990, vol. 265 (31), 19287-95
- **INOKI K ; CORRADETTI MN ; GUAN KL.** Dysregulation of the TSC-mTOR pathway in human disease. *Nat Genet,* 2005, vol. 37, 19-24
- **LEE W-CM ; YOSHIHARA M ; LITTLETON JT.** Cytoplasmic aggregates trap polyglutamine-containing proteins and block axonal transport in a Drosophila model of Huntington's disease. *Proc Natl Acad Sci USA,* 2004, 3224-3229
- **MARTIN S ; ST JOHNSTON D.** A role for Drosophila LKB1 in anterior-posterior axis formation and epithelial polarity. *Nature,* 2003, 379-384
- **RAVIKUMAR B ; VACHER C ; BERGER Z ; DAVIES J ; LUO S ; OROZ L ; SCARAVILLI F ; EASTON D ; DUDEN R ; O'KANE C.** Inhibition of mTOR induces autophagy and reduces toxicity of polyglutamine expansions in fly and mouse models of Huntington disease. *Nat Genet,* 2004, 585-595
- **ROZE E ; SAUDOU F ; CABOCHE J.** Pathophysiology of Huntington's disease: from Huntingtin functions to potential treatments. *Curr Opin Neurol,* 2008, vol. 21, 497-503
- **SCHULTE J ; SEPP KJ ; JORQUERA RA ; WU C ; SONG Y ; HONG P ; LITTLETON JT.** Mob4/Phocein regulates synapse formation, axonal transport, and microtubule organization. *J Neurosci.,* 14 April 2010, vol. 30 (15), 5189-203
- **SEPP KJ ; HONG P ; LIZARRAGA SB ; LIU JS ; MEJIA LA ; WALSH CA ; PERRIMON N.** Identification of neural outgrowth genes using genome-wide RNAi. *PLoS Genet,* 2008, vol. 4 (7), e1000111
- **SHAW R ; LAMIA K ; VASQUEZ D ; KOO S ; BARDEESY N ; DEPINHO R ; MONTMINY M ; CANTLEY L.** The kinase LKB1 mediates glucose homeostasis in liver and therapeutic effects of metformin. *Science,* 2005, vol. 310 (5754), 1642-6
- **SHAW RJ ; BARDEESY N ; MANNING BD ; LOPEZ L ; KOSMATKA M ; DEPINHO RA ; CANTLEY LC.** The LKB 1 tumor suppressor negatively regulates mTOR signaling. *Cancer Cell,* 2004, vol. 6, 91-99
- **TSUCHIYA K ; KOHDA Y ; YOSHIDA M ; ZHAO L ; UENO T ; YAMASHITA J ; YOSHIOKA T ; KOMINAMI E ; YAMASHIMA T.** Postictal blockade of ischemic hippocampal neuronal death in primates using selective cathepsin inhibitors. *Exp Neurol,* 1999, vol. 155, 187-194
- **WANG J ; GINES S ; MACDONALD ME ; GUSELLA JF.** Reversal of a full-length mutant Huntingtin neuronal cell phenotype by chemical inhibitors of polyglutamine-mediated aggregation. *BMC Neurosci,* 2005, vol. 6, 1
- **WU C ; SCHULTE J ; SEPP KJ ; LITTLETON JT ; HONG P.** Automatic robust neurite detection and morphological analysis of neuronal cell cultures in high-content screening. *Neuroinformatics,* vol. 8, 83-100

- **ZHANG X ; SMITH DL ; MERIIN AB ; ENGEMANN S ; RUSSEL DE ; ROARK M ; WASHINGTON SL ; MAXWELL MM ; MARSH JL ; THOMPSON LM.** A potent small molecule inhibits polyglutamine aggregation in Huntington's disease neurons and suppresses neurodegeneration in vivo. *Proc Natl Acad Sci U S A,* 2005, vol. 102, 892-897

SEQUENCE LISTING

<110> MASSACHUSETTS INSTITUTE OF TECHNOLOGY

<120> CHEMICAL AND RNAi SUPPRESSORS OF NEUROTOXICITY IN HUNTINGTON’S DISEASE

<130> M0656.70218WO00

<140> EP 11845326.5
<141> 2011-12-02

<150> US 61/419,157
<151> 2010-12-02

<150> US 61/476,040

<151> 2011-04-15

<160> 20

<170> PatentIn version 3.5

<210> 1
<211> 19
<212> DNA
<213> Artificial Sequence

<220>
<223> oligonucleotide

<400> 1
gccgtcaaga tcctgacta 19

<210> 2
<211> 17
<212> DNA
<213> Artificial Sequence

<220>
<223> oligonucleotide

<400> 2
ctccgctgga ccagatg 17

<210> 3
<211> 20
<212> DNA
<213> Artificial Sequence

<220>
<223> oligonucleotide

<400> 3
gcaactccac ggtgatacct 20

<210> 4
<211> 20
<212> DNA
<213> Artificial Sequence

<220>
<223> oligonucleotide

<400> 4
atgcaggacg tcagcttctt 20

<210> 5
<211> 20
<212> DNA
<213> Artificial Sequence

<220>
<223> oligonucleotide

<400> 5
attgcggcga acttactttg 20

<210> 6
<211> 20
<212> DNA
<213> Artificial Sequence

<220>
<223> oligonucleotide

<400> 6
taatcctcac caggcacaca 20

<210> 7
<211> 20
<212> DNA
<213> Artificial Sequence

<220>
<223> oligonucleotide

<400> 7
gagaatgtgc agggacaggt 20

<210> 8
<211> 20
<212> DNA
<213> Artificial Sequence

<220>
<223> oligonucleotide

<400> 8
gtcgatgaag taaagggcca 20

<210> 9
<211> 17
<212> DNA
<213> Artificial Sequence

<220>
<223> oligonucleotide

<400> 9
ggaggaggag aagcgtg 17

<210> 10
<211> 16
<212> DNA
<213> Artificial Sequence

<220>
<223> oligonucleotide

<400> 10
gcgccgcttg atcatg 16

<210> 11
<211> 20
<212> DNA
<213> Artificial Sequence

<220>
<223> oligonucleotide

<400> 11
cacagcgaca gaagcatcat 20

<210> 12
<211> 20
<212> DNA
<213> Artificial Sequence

<220>
<223> oligonucleotide

<400> 12
ttcttgtatt ccctcgtggc 20

<210> 13
<211> 19
<212> DNA
<213> Artificial Sequence

<220>
<223> oligonucleotide

<400> 13
tttgccagag cgatatctc 19

<210> 14
<211> 20
<212> DNA
<213> Artificial Sequence

<220>
<223> oligonucleotide

<400> 14
ccatagtggc tcgatctttt 20

<210> 15
<211> 20
<212> DNA
<213> Artificial Sequence

<220>
<223> oligonucleotide

<400> 15
ttaaacgtgg ctgagaagaa 20

<210> 16
<211> 17
<212> DNA

<213> Artificial Sequence

<220>
<223> oligonucleotide

<400> 16
gcccacgccc tttttca 17

<210> 17
<211> 20
<212> DNA
<213> Artificial Sequence

<220>
<223> oligonucleotide

<400> 17
gtggtcctga ccgaacagat 20

<210> 18
<211> 20
<212> DNA
<213> Artificial Sequence

<220>
<223> oligonucleotide

<400> 18
aggttttgta ccaaccgctg 20

<210> 19
<211> 17
<212> DNA
<213> Artificial Sequence

<220>
<223> oligonucleotide

<400> 19
gcggacttcg gtgagga 17

<210> 20
<211> 18
<212> DNA
<213> Artificial Sequence

<220>
<223> oligonucleotide

<400> 20
cgctggcaga tgttgttg 18